(19) **Europäisches Patentamt / European Patent Office / Office européen des brevets**

(11) **EP 2 772 531 A1**

(12) # EUROPEAN PATENT APPLICATION
published in accordance with Art. 153(4) EPC

(43) Date of publication:
**03.09.2014 Bulletin 2014/36**

(21) Application number: **12843742.3**

(22) Date of filing: **17.10.2012**

(51) Int Cl.:
*C12M 1/34* (2006.01)    *C12M 3/00* (2006.01)
*C12Q 1/02* (2006.01)    *G01N 27/30* (2006.01)
*G01N 27/416* (2006.01)

(86) International application number:
**PCT/JP2012/076860**

(87) International publication number:
**WO 2013/061849 (02.05.2013 Gazette 2013/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.10.2011 JP 2011237608**

(71) Applicant: **National University Corporation
Tokyo Medical and Dental University
Bunkyo-ku
Tokyo 113-8510 (JP)**

(72) Inventors:
• **YASUDA Kenji**
  **Tokyo 113-8510 (JP)**
• **KANEKO Tomoyuki**
  **Tokyo 113-8510 (JP)**
• **NOMURA Fumimasa**
  **Tokyo 113-8510 (JP)**
• **HATTORI Akihiro**
  **Tokyo 100-0005 (JP)**

(74) Representative: **Prinz & Partner
Esplanade 31
20354 Hamburg (DE)**

(54) **METHOD AND DEVICE FOR EXAMINING MYOCARDIAL TOXICITY AND EVALUATING CARDIOMYOCYTE**

(57)    A method wherein a mass of cardiomyocytes is disposed on a transparent substrate and the quality of the cardiomyocytes is evaluated from the response of the cardiomyocytes to a forced pulsation stimulus that is applied to the pulsating cardiomyocytes. The mass of cardiomyocytes, which is disposed on the transparent substrate, is exposed to the flow of a drug-containing liquid in such a manner as to allow the drug to act on cells configuring a network. The level of cardiotoxicity caused by the drug is evaluated by measuring the fluctuations obtained from a comparison of adjacent pulsating cardiomyocytes of the network.

EP 2 772 531 A1

**Description**

TECHNICAL FIELD

The present invention relates to a method and an apparatus for testing myocardial toxicity and evaluating myocardial cells.

BACKGROUND ART

Bio-assays have been widely used to observe changes in the state of cells, the responsiveness of the cells to agents, and the like. In general, cultured cells have been often used in conventional bioassays. In such systems, assays are performed using a plurality of cells, and an average of the values of a cell population has been measured as if it represented the characteristics of a single cell.

However, in fact, it is rare that there are cells whose cell cycle is synchronized in the cell population, and each cell synthesizes proteins in a different manner. Therefore, fluctuation is always the problem when analyzing the results of the response of the cells to a stimulus.

In other words, since the fluctuations of responses of the reaction mechanism of a cell itself are universally present, one can always only obtain an average of the responses. To solve this problem, there have been developed methodologies, such as synchronized culturing. However, to use a group of cells which are in the same stage, one must always continue to supply such cells, and therefore this feature has become an obstacle to broad-based application of the bioassay.

In addition, in reality it has been difficult to decide on the fluctuation because there are two types of stimulation (signals) to cells: one is given by the amount of a signal substance, nutrition, dissolved gas contained in the solution surrounding the cell, and the other is given by the physical contact and cell-to-cell interaction with other cells.

Difficulties in the physical contact and the cell-to-cell interaction problems of the cells can be resolved to some extent by performing bioassays on a cell mass such as tissue fragments. However, in such cases, unlike cultured cells, it is not always possible to obtain a cell mass with a homogeneous feature. Therefore, there is a problem that the resulting data can vary, and that the information is buried in the population.

To enable measurement using an information processing model in which each cell in the cell population is a minimum structural unit, the inventors of the present application have proposed a microarray for aggregated cells (bioassay chip) comprising a plurality of cell culture compartments for confining a cell inside a specific spatial arrangement; a groove or a tunnel linking between adjacent compartments, wherein a cell cannot pass through the groove or the tunnel; and a plurality of electrode patterns for measuring a change in electric potential of the cell arranged in the groove or the tunnel or the cell culture compartment as shown in JP 2006-94703 (Patent Document 1).

In addition, a method for electrocardiogram analysis has been proposed for the evaluation of the electrocardiogram obtained by reflecting complex cardiac functions by utilizing a method typically used for measuring non-linear dynamics. For example, a Poincare plotting method has been the most commonly used for the analysis of electrocardiogram (Non-Patent Document 1). A point in the plot refers to information of two adjacent pulsation data, in which, for example, a rate of pulsation at one time point is indicated on the X axis and a rate of pulsation at a previous time point is indicated on the Y axis. Thus, the fluctuation in the cardiac pulsation is estimated by quantifying the distribution of the points on the graph. Other methods for measuring the fluctuation of the cardiac pulsation include a correlation dimension method, a nonlinear predictability method (Non-Patent Document 2), an approximate entropy method (Non-Patent Document 3), and the like.

In addition, as for evaluation of cardiac toxicity, there are issues relating to an evaluation of side effects of a drug in terms of the contractile force of heart muscle cells, i.e., how a stroke volume of blood can be changed in response to administration of the drug. However, for this issue, *in vivo* measurements are currently a major approach, and a cell-based *in vitro* screening system has not been established so far.

[Background Art Documents]

[Patent Document]

[Patent Document 1] Japanese Laid-open Patent Publication No. 2006-94703

[Non-Patent Document]

[Non-Patent Document 1] Brennan M, Palaniswami M, Kamen P. Do existing measures of Poincare plot geometry reflect non-linear features of heart rate variability? Biomedical Engineering, IEEE Transactions on, Proc. IEEE Transactions on Biomedical Engineering, 2001, 48 , 1342-1347

[Non-Patent Document 2] Kanters JK, Holstein-Rathlou NH, Agner E (1994) "Lack of evidence for low-dimensional chaos in heart rate variability" Journal of Cardiovascular Electrophysiology 5 (7): 591-601.PMID 7987529.

[Non-Patent Document 3] Storella RJ, Wood HW, Mills KM et al (1994) "Approximate entropy and point correlation dimension of heart rate variability in healthy subjects" Integrative Physiological & Behavioral Science 33 (4): 315-20. PMID 10333974.

SUMMARY OF THE INVENTION

PROBLEM TO BE SOLVED BY THE INVENTION

In conventional bioassays, cells were treated as a tissue fragment or as a single cell as in cultured cells. As mentioned in the above background art section, when the number of cells is excessive, information collected is averaged, and there is a problem that responses to agents cannot be obtained accurately. When the cells are used as a single cell, the cell is used in a separated independent state instead of cells in natural multi-cellular tissues. Consequently, the effect of the interaction between cells is not exhibited. Therefore, there is still a problem in obtaining an accurate agent response, that is, a bioassay data.

There is a need for the development of an apparatus or a system that enables accurate measurement of the membrane potential or the cell morphology in a unit of a single cell as a measure of propagation of pulsation from mutually adjacent fibroblasts or cardiomyocytes, and the accurate measurement of the membrane potential or the cell morphology in a unit of a single cell as a measure of the toxicity of agents on cardiac muscle cells.

Use in regenerative medicine or agent screening requires that the functional aspects of cardiomyocytes which are differentiated from human stem cells including human iPS cells or human ES cells must be evaluated quantitatively to ascertain whether the qualitative features of the cardiomyocytes are the same as cardiomyocytes in the human heart cells.

MEANS FOR SOLVING THE PROBLEM

In order to simultaneously measure electrophysiological and mechanical properties of cardiomyocytes so as to quantitatively evaluate their relationship, the present invention provides an apparatus and method as described below.

(1) A cardiotoxicity evaluation apparatus, comprising:

a substrate;
a plurality of stably pulsating subject cardiomyocytes or a cell population comprising the subject cardiomyocytes and non-cardiomyocytes including fibroblasts placed on the substrate;
a wall formed on the substrate to surround the periphery of the cell population and to fill a cell culture medium;
at least one microelectrode on which a single cell of the cell population or a local portion of the cell population is placed;
a reference electrode provided in the area which is to be filled with the cell culture medium and is surrounded by the wall;

a potential-measuring means for measuring a cellular potential of the cell that is placed on the microelectrode using lead wires which are respectively connected to each of the microelectrodes and a lead wire which is connected to the reference electrode;
a control/recording means for controlling an electrical stimulation delivered to the microelectrode and for recording data of the potential measured by the potential measuring means, microparticles (e.g., polystyrene microparticles, glass microparticles, gold microparticles) of a diameter of from about 1 $\mu$m to about 50 $\mu$m having different optical properties to the cell population comprising the cardiomyocytes, wherein said microparticles are disposed in the cell population or in one or more places of the cell population;
an optical measurement system comprising an irradiation light source, an optical microscope and an image-capturing camera for optically measuring the microparticles, wherein said optical measurement system continuously measures a position and a change in the positions of the microparticles as displacement data which includes temporal displacement data and data of change in angle of the orientation of the displacement; and
a recording means for recording the data of the potential and the displacement data which are correlated with each other.

(2) A cardiotoxicity evaluation apparatus, comprising:

a substrate;
a plurality of stably pulsating subject cardiomyocytes or a cell population comprising the subject cardiomyocytes and non-cardiomyocytes including fibroblasts placed on the substrate;
a wall formed on the substrate to surround the periphery of the cell population and to fill a cell culture medium;
at least one microelectrode on which a single cell of the cell population or a local portion of the cell population is placed;
a reference electrode provided in the area which is to be filled with the cell culture medium and is surrounded by the wall;
a potential-measuring means for measuring a cellular potential of the cell that is placed on the microelectrode using lead wires which are respectively connected to each of the microelectrodes and a lead wire which is connected to the reference electrode;
a control/recording means for controlling an electrical stimulation delivered to the microelectrode and for recording data of the potential measured by the potential measuring means; microparticles of a diameter of from about 1 μm to about 50 μm having different optical properties to the cell population comprising the cardiomyocytes, wherein said microparticles are disposed in the cell population or in one or more places of the cell population;
an optical measurement system comprising an object lens having a numerical aperture of about 3 μm or less and a zoom lens system downstream of the object lens, wherein said optical measurement system continuously measures a position and a change in the positions of the microparticles as displacement data which includes temporal displacement data and data of change in angle of the orientation of the displacement; and
a recording means for recording data of the potential and the displacement data which are correlated with each other.

(3) The cardiotoxicity evaluation apparatus according to (1) or (2) above, further comprising a culture medium supply/discharge channel for supplying the cell culture medium to and/or discharging the cell culture medium from the region surrounded by the wall.

(4) The cardiotoxicity evaluation apparatus according to any one of (1) to (3) above, wherein said microelectrode comprises a stimulation electrode for stimulating a cell and a measurement electrode for measuring a cellular potential of the cell.

Further, the present invention provides the following apparatus and method in order to evaluate cardiotoxicity using measurements of an extracellular potential of cardiomyocytes.

(5) A cardiotoxicity evaluation method, comprising:

selecting a cardiomyocyte which is characterized by a sharp generation of inward current of Na ions within about 20ms or less after a start of depolarization with clear rapid depolarization start, a subsequent slow generation of inward current of Ca ions within about 100ms after the start of the depolarization, and a prominent generation of outward current of K ions observed in about 100ms or after the start of the depolarization with no addition of a drug as a cardiomyocyte for measurements, and
measuring an extracellular potential of the cardiomyocyte using the cardiotoxicity evaluation apparatus according to (1) above.

(6) A cardiotoxicity evaluation method, comprising:

selecting a cardiomyocyte which is characterized by a sharp generation of inward current of Na ions within about 20ms or less after a start of depolarization with clear rapid depolarization start, a subsequent slow generation of inward current of Ca ions within about 100ms after the start of the depolarization, and a prominent generation of outward current of K ions observed in about 100ms or after the start of the depolarization with no addition of a drug as a cardiomyocyte for measurements, and

measuring an extracellular potential of the cardiomyocyte using the cardiotoxicity evaluation apparatus according to (2) above.

(7) The cardiotoxicity evaluation method according to (5) or (6) above, wherein said cardiotoxicity evaluation apparatus further comprises a culture medium supply/discharge channel for supplying the cell culture medium to and/or discharging the cell culture medium from the region surrounded by the wall.

(8) The cardiotoxicity evaluation method according to any one of (5) to (7) above, wherein the microelectrodes comprise a stimulation electrode for stimulating the cells and a potential measurement electrode for measuring a cellular potential of the cells.

Further, in order to assess cardiotoxicity by an extracellular potential measurements of cardiomyocytes, the present invention provides a method and apparatus described below as a technique for providing a stimulus.

(9) A cardiotoxicity evaluation apparatus, comprising:

a substrate;
a plurality of stably pulsating subject cardiomyocytes or a cell population comprising the subject cardiomyocytes and non-cardiomyocytes including fibroblasts placed on the substrate;
a wall formed on the substrate to surround the periphery of the cell population and to fill a cell culture medium;
at least one microelectrode on which a single cell of the cell population or a local portion of the cell population is placed;
an array of stimulation electrodes for stimulating the cells comprising a plurality of microelectrodes disposed two-dimensionally on the substrate, wherein a signal strength and a phase of the microelectrodes are mutually controllable;
a reference electrode disposed in the area which is to be filled with the cell culture medium and is surrounded by the wall;
a potential-measuring means for measuring a cellular potential of the cell that is placed on the microelectrode using lead wires which are respectively connected to each of the microelectrodes and a lead wire which is connected to the reference electrode; and

a control/recording means for controlling an electrical stimulation delivered to the microelectrodes for stimulating the cells, and for recording data of the potential measured by the potential-measuring means.

(10) The cardiotoxicity evaluation apparatus of (9) above, further comprising a culture medium supply/discharge channel for supplying the cell culture medium to and/or discharging the cell culture medium from the region surrounded by the wall.

(11) A cardiotoxicity evaluation method, comprising:

selecting a cardiomyocyte which is characterized by a sharp generation of inward current of Na ions within about 20ms or less after a start of depolarization with clear rapid depolarization start, a subsequent slow generation of inward current of Ca ions within about 100ms after the start of the depolarization, and a prominent generation of outward current of K ions observed in about 100ms or after the start of the depolarization with no addition of a drug as a cardiomyocyte for measurements, and

measuring an extracellular potential of the cardiomyocyte using a cardiotoxicity evaluation apparatus which comprises:

a substrate;
a plurality of stably pulsating subject cardiomyocytes or a cell population comprising the subject cardiomyocytes and non-cardiomyocytes such as fibroblasts placed on the substrate;
a wall formed on the substrate to surround the periphery of the cell population and to fill a cell culture medium;
at least one microelectrode on which a single cell of the cell population or a local portion of the cell population is placed;
an array of stimulation electrodes for stimulating the cells comprising a plurality of microelectrodes disposed two-dimensionally on the substrate, wherein a signal strength and a phase of the microelectrodes are mutually controllable;
a reference electrode disposed in the area which is to be filled with the cell culture medium and is surrounded by the wall;
a potential-measuring means for measuring cellular potential of the cell that is placed on the microelectrode using lead wires which are respectively connected to each of the microelectrodes and a lead wire which is connected to the reference electrode; and

a control/recording means for controlling an electrical stimulation delivered to the microelectrodes for stimulating the cells, and for recording data of the potential measured by the potential-measuring means.

(12) The cardiotoxicity evaluation method according to (11) above, wherein the cardiotoxicity evaluation apparatus further comprises a culture medium supply/discharge channel for supplying the cell culture medium to and/or discharging the cell culture medium from the region surrounded by the wall.

(13) A cardiotoxicity evaluation method, comprising:

comparatively analyzing fluctuation (dispersion) of movement distance (displacement) and movement direction (angle) of myocardial cells between contraction intervals in association with contraction of cardiac muscle and fluctuation (dispersion) of FPD (time to maximum inward current position from the first spike of sodium) by electrophysiological measurement using an apparatus system capable of performing measurements and/or analysis of optical measurements of cardiomyocytes in synchronization with measurements of membrane potential of the cardiomyocytes,
wherein said movement distance and movement direction are obtained by optical measurements of the myocardial cells when they brought into contact with a target drug.

(14) A replaceable well system, comprising:

one or more unit wells each comprising a single well, wherein said well comprises an electrode array including a cellular potential measuring electrode, a cell stimulation electrode and a reference electrode for myocardial cells on a bottom surface of the well; and
a well plate comprising one or more compartments to which the one or more unit wells can be mounted interchangeably;
wherein the wells are replaceable in a one-by-one basis and are usable for forming a cardiomyocyte-network array, and wherein each of the compartments of the well plate comprises a contact connected correspondingly to one of the lead wires of the electrode array, and each of the unit wells is arranged interchangeably with respect to the compartment.

(15) A cardiomyocyte measuring apparatus system, comprising:

two or more cardiomyocyte-network chips each comprising a well for accommodating a cardiomyocyte, and an electrode array including a cell stimulation electrode, a cellular potential measuring electrode and a reference electrode on a bottom surface of the well;
a stage for mounting the chips;
a potential measuring system comprising a power supply for providing electric stimulation to the cardiomyocyte through the electrode array and measuring an extracellular potential of the cardiomyocyte;
an optical observation system comprising an optical microscope, a camera for recording images and an illumination light source for optically observing the cardiomyocytes; and
a control and/or analysis device for recording and/or analyzing the potential measuring data and the optical observation data in a synchronous manner.

(16) The cardiomyocyte measuring apparatus system according to (15) above which is used for evaluation of cardiotoxicity.

(17) A multi-electrode substrate for the potential measuring of cardiomyocyte network arranged in a circle, comprising:

either

(i) a ring-shaped potential measuring electrode corresponding to the cardiomyocyte network, wherein a portion of the electrode is cut out;

a stimulation electrode for applying a local force stimuli located in the cut-out site of the potential measuring electrode; and
a reference electrode for eliminating noise, which is disposed near the outside of the ring, or

(ii) a plurality of potential measuring electrodes in which the ring-shaped potential measuring electrode of (i) above is further cut out into two or more parts;

a stimulation electrode for applying a local forced stimuli, which is located in the cut-out site of the potential measuring electrodes; and
a reference electrode for eliminating noise, which is disposed near the outside of the ring.

(18) A multi-electrode substrate for measuring the potential of cardiomyocyte network using a two-dimensional cardiomyocytes sheet, comprising:

a ring-shaped potential measuring electrode, wherein a portion of the electrode is cut out;
a stimulation electrode for applying a local force stimuli, which is located in the center of the ring; and
a reference electrode for eliminating noise, which is disposed near the outside of the cut-out site of the potential measuring electrode.

(19) A sample loader for placing a cardiomyocyte sample in a block-type unit well comprised of one well comprising an electrode array including a cellular potential measuring electrode, a stimulating electrode and a reference electrode arranged on the bottom surface of the well,
wherein the sample loader has an external shape corresponding to the shape of the unit well such that the sample loader can be inserted into a top surface of the unit well; a funnel-shaped inner structure; and a slit (opening) corresponding to the shape of the electrode of the unit well on a bottom surface, and wherein the cardiomyocytes can be placed on the electrode by dropping an appropriate amount of the cardiomyocyte sample onto the inner surface of the sample loader.

(20) A cardiomyocyte culture measurement substrate on which microprojections are disposed regularly to prevent contraction of the cardiomyocyte during culturing and measuring using a cardiomyocyte network or a myocardial cell sheet.

(21) A cardiotoxicity evaluation apparatus characterized in that a microelectrode wire is used as an electrode.

Effect of the Invention

According to the present invention, changes in the response of cardiomyocytes and fibroblasts to an agent can be accurately evaluated by measuring fluctuations of cells.
Conventionally, proposals have been made to test cardiotoxicity independently using field potential duration (FPD) (see description below) and the magnitude of the fluctuation of adjacent pulsations of adjacent cardiomyocytes (for example, short-term variability: STV). However, no proposals have been made in relation to testing of cardiotoxicity using a combination of both those features. The method for testing cardiotoxicity of the present invention enhances the accurate evaluation of cardiotoxicity by using not only the prolongation of the FPD waveform, but also an increase in the magnitude of the fluctuation of the adjacent pulsations of the cardiomyocytes (STV).
Further, the present invention provides an *in vitro* system capable of evaluating both an electrophysiological response and a mechanical response of a population of cardiomyocytes. Measurements at an *in vitro* cardiomyocyte level, which make the evaluation at a close to an individual level, are available.

BRIEF DESCRIPTION OF THE DRAWINGS

[Figure 1] Figure 1 is a perspective view schematically showing an exemplary structure of a cardiotoxicity testing apparatus according to an example of the present invention.

[Figure 2] Figure 2 is a perspective view schematically showing an exemplary structure of a cell holding unit CH of the cardiotoxicity testing apparatus shown in Figure 1.

[Figure 3] Figure 3 is a diagram for illustrating an optical system for optically detecting a cell on the cell holding unit CH of the cardiotoxicity testing apparatus shown in Figure 1.

[Figure 4] Figures 4(a), 4(b) and 4(c) are diagrams showing signals associated with measurement of membrane potentials. Each diagram shows time along the horizontal axis and the membrane potential between the microelectrode 2 and the comparison electrode $2_C$ along the vertical axis.

[Figure 5] Figures 5(a), 5(b) and 5(c) are diagrams showing signals associated with the changes in the volume due to cell pulsation, which is measured with the optical system.

[Figure 6] Figure 6(a) shows changes in the potentials according to the amounts of $Na^+$, $Ca^{2+}$ and $K^+$ ion in- and out-flow into/from the target cells under a normal state where the culture solution is free of agent. Figure 6(b) shows changes in the potentials according to the amounts of $Na^+$, $Ca^{2+}$ and $K^+$ ion in- and out-flow into/from the target cells under a state where the culture solution contains an agent.

[Figure 7] Figure 7 illustrates an exemplary arrangement of an optical system and a movable electrode of the cardiotoxicity testing apparatus for optically detecting the cells.

[Figure 8] Figure 8 is a schematic view for illustrating generation of an electric signal of a cell.

[Figure 9] Figure 9(a) shows an exemplary change in the membrane potentials upon addition of an agent; and Figure 9(b) shows one example of a Poincare plot for evaluating homology between two successive pulses with respect to the change in the membrane potentials upon each pulsation.

[Figure 10] Figure 10(a) is a schematic view showing an exemplary re-entry circuit prepared with an annular network of cardiomyocytes by means of a cell arrangement technique at single-cell level; and Figure 10(b) is a micrograph showing an actual exemplary arrangement of the cells on the microelectrodes.

[Figure 11] Figure 11 (a) is a schematic view showing an exemplary re-entry circuit prepared with an annular network of cardiomyocytes using a cell population having a certain width; Figure 11(b) is a microscopic picture showing an actual exemplary arrangement of the cells on the microelectrodes; and Figure 11(c) is a microscopic picture showing an actual exemplary annular arrangement of the cell population on the microelectrode array.

[Figure 12] Figure 12(a) is a schematic view showing an exemplary re-entry circuit measurement apparatus using an annular electrode; and Figure 12(b) is a graph showing normal pulse data and abnormal pulse data actually measured with the electrode.

[Figure 13] Figure 13(a) is a schematic view showing an exemplary arrangement of an electrode for measuring potentials of a single cell and the cell; Figure 13(b) shows a picture of the isolated single cell on the electrode actually measured with the electrode and electric pulse data thereof; and Figure 13(c) shows a picture of a cell population measured on the electrode and a graph showing electric pulse data of one of the cells of the cell population.

[Figure 14] Figure 14 is a schematic view illustrating an example of the present invention in which a photosensitive element of the camera is used for measuring a potential of a single cell.

[Figure 15] Figure 15 is a schematic view illustrating an exemplary mechanism for measuring a plurality of samples with a cell measurement system of the present invention.

[Figure 16] Figure 16 is a schematic view illustrating cardiac information that can be measured with a cell measurement system of the present invention.

[Figure 17] Figure 17 is an example of a graph illustrating the addition of the agent changes to the field potential signal waveform of the cells measurable by the measurement system of the present invention cells.

[Figure 18] Figure 18 is an example of a graph illustrating an example of the average value of the changes in response to the addition of a potassium ion channel inhibitor E4031 in connection with elapsed time (FPD: field potential duration) of the peak position of the release of potassium ions from the release time of sodium ions in the signal waveform of the field potential of the cells that can be measured by the cell measurement system of the present invention.

[Figure 19] Figure 19 is an example of a graph and a formula illustrating one of the methods for evaluating quantitatively the size of fluctuation of short-term variability of adjacent pulsations on the basis of Poincare plotting in connection with elapsed time (FPD: field potential duration) of the peak position of the release of potassium ions from the release time of sodium ions in the signal waveform of the field potential of the cells that can be measured by the cell measurement system of the present invention.

[Figure 20] Figure 20 is an example of a graph and a formula illustrating one of the methods for evaluating quantitatively, based on Poincare plotting, the size of the fluctuation of elapsed time (FPD: field potential duration) of the peak position of the release of potassium ions from the release time of sodium ions in the signal waveform of the field potential of the cells that can be measured by the cell measurement system of the present invention.

[Figure 21] Figure 21 is an example of a representation of the size of the fluctuation produced by the addition of EE4031 in (a) Poincare plotting and (b) STVs in connection with elapsed time (FPD: field potential duration) of the peak position of the release of potassium ions from the release time of sodium ions in the signal waveform of the field potential of the cardiomyocytes that can be measured by the cell measurement system of the present invention.

[Figure 22] Figure 22 shows FPD and STV in the case of adding agents known to have a variety of cardiotoxicities on cardiomyocytes and a reference agent measurable by the measurement system cells of the present invention.

[Figure 23] Figure 23 shows an example of Poincare plotting of FPD against addition of an agent in terms of the difference in the shape of the cardiomyocyte network and the difference in position thereof which can be measured by the measurement system of the present invention. (a) A micrograph showing an example of an actual cellular network (a); (b) A graph showing measured changes at points A, B, C and D of (a).

[Figure 24] Figure 24 shows an example of Poincare plotting of the transmission time to a local point from the pacemaker area in response to an addition of an agent in terms of the difference in the shape of the cardiomyocyte network and the difference in the position thereof which can be measured by the measurement system of the present invention. (a) A micrograph showing an example of an actual cellular network (a); (b) A graph showing measured changes at points A, B, C and D of (a).

[Figure 25] Figure 25 is a diagram schematically showing a relationship between a conventional *in vitro* measurement method and a conventional *in vivo* measurement method, and a relationship between an FP waveform of a single cell and a composite FP waveform of a cellular network during the measurements of a network of cardiomyocytes measurable by the cell measurement system of the present invention.

[Figure 26] Figure 26 is a diagram schematically showing a configuration of an apparatus having a function to estimate membrane potentials of cells from the FP waveform of the cells collected from each electrode and a function to compose a comparison waveform of an electrocardiogram from a composite FP waveform of the cellular network during the cardiomyocyte-network measurements which are measurable by the cell measurement system of the present invention.

[Figure 27] Figure 27 shows examples of: (A) an annularly arranged cardiomyocyte network; (B) FP waveforms of cells obtained from each electrode of the network of (A); (C) a composite FP waveform composing the waveforms of (B). This example shows an example in which the pulsation signal is transmitted normally from the PM area.

[Figure 28] Figure 28 shows examples of: (A) an annularly arranged cardiomyocyte network; (B) FP waveforms of cells obtained from each electrode of the network of (A); (C) a composite FP waveform composing the waveforms of (B). This example shows an example in which the pulsation signal is transmitted abnormally from the PM area.

[Figure 29] Figure 29 is a graph showing an exemplary relationship between the beating frequency (beating frequency) of cardiomyocytes and the FPD during the cardiomyocyte-network measurements which are measurable by the cell measurement system of the present invention.

[Figure 30] Figure 30 is a graph showing an example of chronological change of FPD when forced pulsation is imparted to the cardiomyocytes during the cardiomyocyte-network measurements which are measurable by the measurement system of the present invention.

[Figure 31] Figure 31 is a photomicrograph showing an example of the cellular network arrangement when the FPD is measured when forced pulsation is imparted to cardiomyocytes during the cardiomyocyte-network measurements which are measurable by the measurement system of the present invention.

[Figure 32] Figure 32 is a schematic diagram showing an example of using a mechanism to maintain a constant potential at the microelectrodes using a feedback control of a trace electrode potential to measure the FP of cardiomyocytes during the cardiomyocyte-network measurements which are measurable by the measurement system of the present invention.

[Figure 33] Figure 33 is a graph showing an example of the relationship with the response of the beating frequency of the cardiomyocyte population when forced pulsation is given to a partial area of the cardiomyocyte population during the cardiomyocyte-network measurements which are measurable by the measurement system of the present invention.

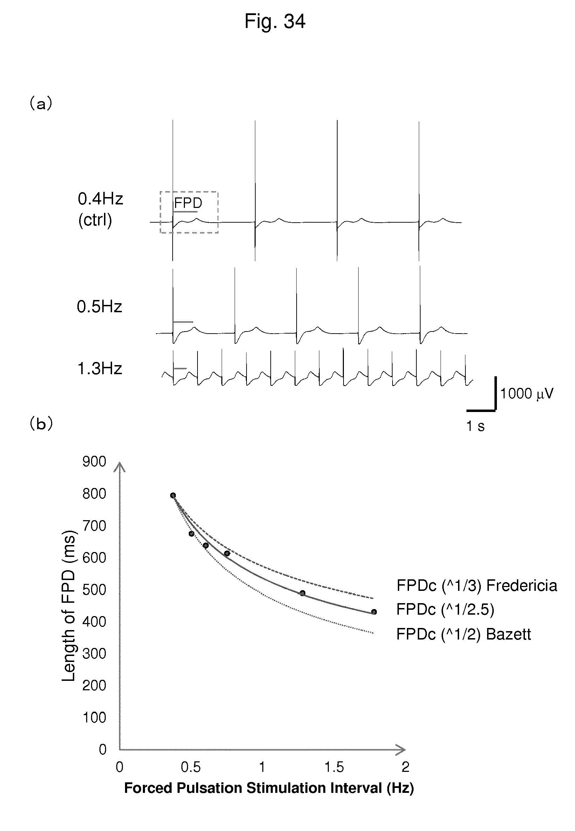

[Figure 34] Figure 34 is a graph showing an example of the change in the length of the FPD when forced pulsation is given to a partial area of the cardiomyocyte population during the cardiomyocyte-network measurements which are measurable by the measurement system of the present invention. A graph showing (a) an example of the relationship of the change in the length of the FPD and the change in the FP waveform caused by forced pulsatile stimulation; and (b) an example of the change in the length of the FPD in response to the change in the stimulation interval of the forced pulsatile stimulation

[Figure 35] Figure 35 is a table summarizing the results shown in Figure 33 and Figure 34 regarding an example of the response of the cell population when forced pulsation is given to a partial area of the cardiomyocyte population during the cardiomyocyte-network measurements which are measurable by the measurement system of the present invention.

[Figure 36a] Figure 36 schematically shows a difference circuit between a reference electrode and microelectrodes for noise removal during a measurement of an electrode potential in accordance with the present invention. (a) A schematic diagram of an example of a circuit illustrating the principles.

[Figure 36b] Figure 36b schematically shows a difference circuit between a reference electrode and microelectrodes for noise removal during a measurement of an electrode potential in accordance with the present invention. (b) A circuit diagram of an example of an amplifier circuit incorporating the difference circuit.

[Figure 36c] Figure 36c schematically shows a difference circuit between a reference electrode and microelectrodes for noise removal during a measurement of an electrode potential in accordance with the present invention. (c) A diagram showing an example in which noise is reduced by the circuit.

[Figure 37] Figure 37 is a diagram schematically showing an example of a comprehensive evaluation method for cardiotoxicity evaluation in accordance with the present invention. (a) The degree of a prolongation of the FPD from the FPD data of the cells is plotted in the X-axis, and the magnitude of temporal fluctuations of the FPD is plotted in the Y axis. (b) An example of a plot for the average data from the above results, plotted in the X-Y diagram.

[Figure 38] Figure 38 is a schematic diagram showing an example of the configuration of a system for measuring the cardiac toxicity of the present invention.

[Figure 39] Figure 39 is a schematic diagram and a photography showing an example of the configuration of the measurement chamber of the cell culture system to measure the cardiac toxicity of the present invention.

[Figure 40] Figure 40 is a diagram schematically showing a cross-section of the measuring cell culture plate.

[Figure 41] Figure 41 is a diagram schematically illustrating a configuration of the electrode wire electrode arrangement being disposed in a multi-electrode substrate.

[Figure 42] Figure 42 is a schematic diagram showing an example of a multi-electrode arrangement of electrodes on the substrate.

[Figure 43] Figure 43 is a schematic diagram showing an example of a system configuration of the present invention to simultaneously measure mechanical properties and electrical properties of cardiomyocytes.

[Figure 44] Figure 44 is an example of a data acquisition monitor screen showing an example of data obtained from an example of a system configuration of the present invention to simultaneously measure mechanical properties and electrical properties of cardiomyocytes.

[Figure 45] Figure 45 is an example of data obtained from an example of a system configuration of the present invention to simultaneously measure mechanical properties and electrical properties of cardiomyocytes.

[Figure 46] Figure 46 is a diagram illustrating an example of acquisition of direction data of cell displacements obtained from an example of a system configuration of the present invention to simultaneously measure mechanical properties and electrical properties of cardiomyocytes.

[Figure 47] Figure 47 is a diagram illustrating an example of a spatial arrangement of a myocardial cell system in the network system of the present invention to simultaneously measure mechanical properties and electrical properties of cardiomyocytes.

[Figure 48] Figure 48 is a diagram illustrating an example of waveform patterns of the extracellular potential in cells for measuring the electrical characteristics of cardiomyocytes.

[Figure 49] Figure 49 is a diagram showing an example of cellular potential and fluctuation changes in drug response of hERG ion channel of cardiomyocytes.

[Figure 50] Figure 50 is a diagram illustrating the principle of cell stimulation at any position by superposition of stimulation potentials from a stimulation electrode array.

[Figure 51] Figure 51 is a diagram illustrating effects of combining a zoom lens system and an objective lens having a numerical aperture less than 0.3 for the optical measurement of microparticles.

[Figure 52] Figure 52 is a diagram showing an example of results obtained with a cardiomyocyte network when electrophysiological extracellular potential data and changes in contractile force of cells are measured at the same time after administration of verapamil.

[Figure 53] Figure 53 is a diagram showing an example of analysis results obtained with a cardiomyocyte network when electrophysiological extracellular potential data and changes in contractile force of cells are simultaneously measured after administration of verapamil.

[Figure 54] Figure 54 is a diagram showing an example of analysis results obtained with a cardiac muscle cell network when an electrophysiological extracellular potential, its fluctuation data, the fluctuation amounts of changes in the contractile force of cells in the direction of displacement and angular direction are simultaneously measured after administration of verapamil and their use in the analysis.

[Figure 55] Figure 55 is an example of a configuration that combines extracellular potential measurement and a contractile-function-change measuring optical system.

[Figure 56] Figure 56 illustrates an example of a configuration of a high-throughput myocardial-cell network array chip comprised of a cell culture module array.

[Figure 57] Figure 57 illustrates an example of a configuration of a cellular network deployment technique using a sample loader for placing cardiomyocytes in each well effectively.

[Figure 58] Figure 58 illustrates another example of a system for measuring an extracellular potential by a multi-electrode system in which block-type wells are actually arranged.

[Figure 59] Figure 59 illustrates an example of a configuration incorporating an optical measurement module to the system of Figure 58.

[Figure 60] Figure 60 is a diagram illustrating the structure of a substrate on which microprojections are regularly disposed to prevent contraction of cardiomyocytes in a culture medium during measurements using a myocardial-cell network and a myocardial cell sheet.

[Figure 61] Figure 61 is a schematic view showing an example of an electrode arrangement on a multi-electrode substrate.

[Figure 62] Figure 62 illustrates schematically an example of an embodiment in which metal micro wires are used as electrodes.

[MODE FOR CARRYING OUT THE INVENTION]

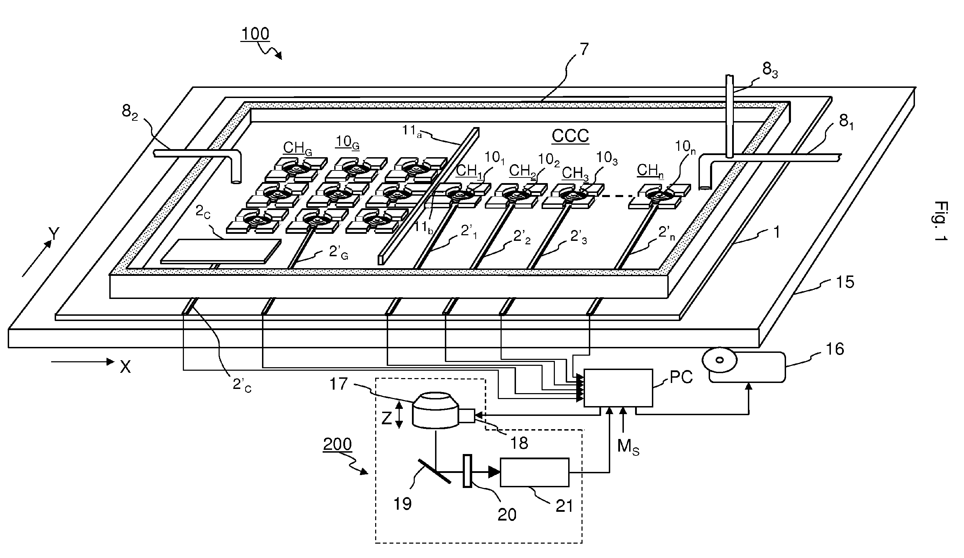

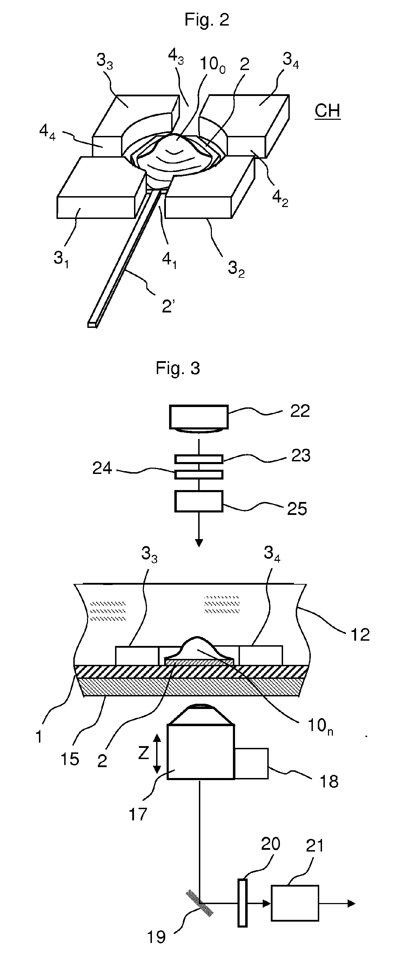

Figure 1 is a perspective view schematically showing an exemplary structure of an apparatus for testing cardiotoxicity according to an example of the present invention. Figure 2 is a perspective view schematically showing an exemplary structure of a cell holding unit CH of the cardiotoxicity testing apparatus shown in Figure 1. Figure 3 is a view for illustrating an optical system for optically detecting the cell retained in the cell holding unit CH of the cardiotoxicity testing apparatus shown in Figure 1.

Referring to Figure 1 and Figure 2, the cardiotoxicity testing apparatus 100 mainly consists of parts built on a transparent substrate 1. The transparent substrate 1 is an optically transparent material, for example, a glass substrate or a silicon substrate. The microelectrodes 2 are transparent ITO electrodes, for example, arranged on the transparent substrate 1. Reference numeral 2' denotes readout lines from the microelectrodes 2. Reference numerals $3_1$, $3_2$, $3_3$ and $3_4$ denote agarose

gel walls, which are arranged around each of the microelectrodes 2 with gaps $4_1$, $4_2$, $4_3$ and $4_4$. The agarose gel walls $3_1$, $3_2$, $3_3$ and $3_4$ are cutout in the middle to form a space as cell housing. The microelectrode 2 is placed on the transparent substrate 1, as necessary, within the space as the cell housing formed with the agarose gel walls $3_1$, $3_2$, $3_3$ and $3_4$. Regardless of the presence of the microelectrode 2, a single cell 10 can be retained in the cell housing. In Figure 2, the microelectrode 2 is arranged on the transparent substrate 1 within the space as the cell housing formed with the agarose gel walls $3_1$, $3_2$, $3_3$ and $3_4$, where a cardiomyocyte 10 is additionally retained on the microelectrode 2. The microelectrode 2 is shown to be connected to the readout line 2'. A material, e.g., collagen, which enhances cellular adherence to the electrode surface or the transparent substrate, is preferably applied onto the cell-bearing surface of the microelectrode 2 or, directly onto the transparent substrate 1 when the cell is disposed in the absence of the microelectrode 2. Since the cell within the cell housing formed with the agarose gel walls $3_1$, $3_2$, $3_3$ and $3_4$ is non-adherent to the agarose gel, the cell 10 will not transfer beyond the walls even if its height is equivalent to the heights of these walls $3_1$, $3_2$, $3_3$ and $3_4$. Furthermore, since the gaps $4_1$, $4_2$, $4_3$ and $4_4$ surrounding the cell housing formed by cutting out in the middle of the agarose gel walls $3_1$, $3_2$, $3_3$ and $3_4$ are smaller than the size of the cell, the cell 10 will not move across these gaps $4_1$, $4_2$, $4_3$ and $4_4$.

With reference to Figure 1, the cell holding units $CH_1$, $CH_2$, $CH_3$ and $CH_n$ each retains a cardiomyocyte or a fibroblast $10_1$, $10_2$, $10_3$ or $10_n$ in the cell housing. Each holding unit is provided, although not evident from the figure, with the microelectrode 2 from which extends the readout line $2'_1$, $2'_2$, $2'_3$ or $2'_n$. These cardiomyocytes or fibroblasts form a tandemly arranged cell communication channel CCC. Here, "n" is, for example, 20. Although these twenty tandemly-arranged cardiomyocytes and fibroblasts may be allocated randomly, the cells in the cell holding units $CH_1$ and $CH_{20}$ are preferably cardiomyocytes. On the left side of this cell communication channel CCC are provided 3 x 3 cell holding units $CH_G$ to form a region that retains a cardiomyocyte population $10_G$ where each cell holding unit CH retains a cardiomyocyte 10. This cell population $10_G$ serves as a stably-pulsating pacemaker. Among the cell population $10_G$, only one of the cell holding units CH is provided with the microelectrode 2 from which extends the readout line $2'_G$. In addition, the right middle cell holding unit CH of the cell population $10_G$ is arranged to face the cell holding unit $CH_1$ of the cell communication channel CCC. A barrier $11_a$ is provided on the right of the cell population $10_G$ and the left of the cell communication channel CCC. A small opening $11_b$ is formed in the lower middle part of this barrier $11_a$. On both sides of this opening $11_b$, the right middle cell holding unit CH of the cell population $10_G$ is facing the cell holding unit $CH_1$ of the cell communication channel CCC to allow physical contact/intercellular interaction between the cells retained in the cell housings via the gaps 4 at the periphery of the housings. A comparison electrode $2_C$ is provided below the cell population $10_G$, from which the readout line $2'_C$ extends.

Reference numeral 7 denotes a surrounding wall that surrounds the cell population $10_G$, the cell communication channel CCC and the comparison electrode $2_C$. Reference numerals $8_1$ and $8_2$ denote pipes for supplying a cell culture solution into the region surrounded by the wall 7 and for draining the cell culture solution from the region surrounded by the wall 7. In the case of this figure, a culture solution is supplied from the pipe $8_1$ extending toward the bottom surface of the substrate 1 and drained from the pipe $8_2$ extending from the bottom surface of the substrate 1. A pipe $8_3$ is connected to the culture solution-supplying pipe $8_1$ near the culture solution outlet so that an agent that acts on the cells is supplied via this pipe $8_3$. Accordingly, the cells 10 are exposed to the cell culture solution supplied from the pipe $8_1$ into the region surrounded by the wall 7, while being stably retained on the microelectrodes 2. Once the cells no longer need to be exposed to the culture solution, the culture solution can be drained from the region surrounded by the wall 7 with the pipe $8_2$. Moreover, when the culture solution needs to be exchanged with a fresh culture solution, the culture solution may be supplied after or while draining the cell culture solution. On the other hand, if one wants to affect the cells with an agent, the agent for affecting the cells may be added to the culture solution via the pipe $8_3$ for supply together with the culture solution via the pipe $8_1$ while draining the cell culture solution from the pipe $8_2$. In this case, due to the barrier $11_a$ provided between the cell population $10_G$ and the cell communication channel CCC, when the culture solution containing the agent is supplied into the region surrounded by the wall 7 from the pipe $8_1$, the cells of the cell population $10_G$ are less influenced by the agent than the cells of the cell communication channel CCC. Specifically, when an agent-containing culture solution is supplied via the pipe $8_1$, this culture solution flows through the spacing between the wall 7 and the both edges of the barrier $11_a$ as well as over the top of the barrier $11_a$ toward the cell population $10_G$. Thus, the cells of the cell population $10_G$ are also affected by the agent. This influence, however, is indirect compared to the influence on the cells of the cell communication channel CCC, and thus it does not affect the function as a pacemaker. The structures and arrangements of the pipes $8_1$, $8_2$ and $8_3$ may arbitrarily be changed depending on the measurement configuration. For example, the pipes $8_1$ and $8_3$ may be separated, or the pipe $8_2$ may be omitted by using the pipe $8_1$ for both supply and drainage.

PC refers to a personal computer (potential measurement means, control/recording means), which measures and records the membrane potentials between the readout lines 2' from the microelectrodes 2 of the cell holding units CH and the readout line 2' from the comparison electrode $2_C$. Furthermore, operation signals Ms from an operator are input into the personal computer 9.

The cardiotoxicity testing apparatus 100 may be mounted on an XY stage 15 of the optical observation device 200 where the pulsation of a certain cell 10 of the cell communication channel CCC can be observed with an optical system. The XY stage 15 is optically transparent and may be moved to a given position with an X-Y drive unit 16 according to the signal given by the personal computer PC reflecting the operation signal Ms from the operator. Figure 3 shows an exemplary configuration for observing the pulsating state of a cell $10_n$ of the cell communication channel CCC. Reference numeral 12 denotes a culture solution.

Reference numeral 22 denotes a light source of a phase-contrast microscope or a differential interference microscope. Generally, a halogen lamp is used. Reference numeral 23 denotes a bandpass filter that only allows transmission of light with a specific wavelength from the light source for observation with a stereoscopic microscope such as a phase-contrast microscope. For example, in the case of observing the cell $10_n$, narrow-band light having a wavelength in the vicinity of 700 nm is used to prevent damage to the cell $10_n$. Reference numeral 24 denotes a shutter that has a function of blocking irradiation light when image measurement is not executed, for example, while moving the XY stage 15. Reference numeral 25 denotes a condenser lens, where a phase ring is installed for phase-contrast observation or a polarizer for differential interference observation. The cardiotoxicity testing apparatus 100 formed on the substrate 1 is mounted on the XY stage 15 which can be moved with the X-Y drive unit 16 to observe and measure a certain location of the cardiotoxicity testing apparatus 100. The pulsating state of the cell $10_n$ in the cardiotoxicity testing apparatus 100 is observed with an objective lens 17. The focal position of the objective lens 17 can be transferred in the Z-axis direction with a drive unit 18 according to the signal from the PC. The magnification of the objective lens 17 may be 40 or higher. The objective lens 17 allows observation of a phase-contrast image or a differential interference image of the cell $10_n$ obtained with light transmitted from the light source 22. A diachronic mirror 19 and a bandpass filter 20 that reflect light having the same wavelength as the light that passes through the bandpass filter 23 allow observation of only a phase-contrast microscope image or a differential interference microscope image with a camera 21. The image signal observed with the camera 21 is input into the personal computer PC. In addition, although it is not illustrated in a diagram, images are displayed on a monitor or a display connected to the PC.

Exemplary dimensions of the structures of the cardiotoxicity testing apparatus 100 shown in Figure 1 are as follows. In this example, the size of a cell is 10 $\mu m\varphi$. The transparent substrate 1 has dimensions of 100 mm x 150 mm, the microelectrode 2 has dimensions of 8 $\mu m$ x 8 $\mu m$ and each of the agarose gel walls $3_1$, $3_2$,

$3_3$ and $3_4$ has dimensions of 20 μm x 20 μm x 10 μm (height). Each of the gaps $4_1$, $4_2$, $4_3$ and $4_4$ has a width of 2 μm, the cell housing formed with the agarose gel walls $3_1$, $3_2$, $3_3$ and $3_4$ has a 12 μmφ cylindrical space, and the wall 7 has external dimensions of 5 mm x 5 mm with a height of 5 mm. The height of the barrier $11_a$ is 1 mm. Although the microelectrode 2 has a square shape of 8 μm x 8 μm in this example, it may be an annular electrode of 10 μmφ that corresponds to the shape of the cell housing made with the agarose gel walls $3_1$, $3_2$, $3_3$ and $3_4$ and the widths of the gaps $4_1$, $4_2$, $4_3$ and $4_4$.

Hereinafter, an exemplary structure of the cell response measurement apparatus 100 of the present invention and a specific example of measurement using the same will be described.

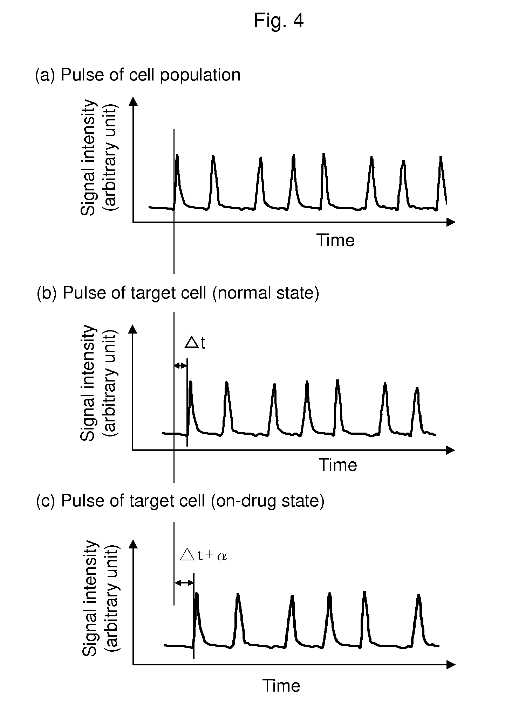

Figures 4(a), 4(b) and 4(c) are diagrams showing signals associated with measurement of membrane potentials. Each diagram shows time along the horizontal axis and the membrane potential between the microelectrode 2 and the comparison electrode $2_C$ along the vertical axis. Figure 4(a) shows membrane potentials resulting from the pulses of the cell population $10_G$. Here, a potential refers to an electric difference between the readout line $2'_G$ extending from one of the cell population $10_G$ and the readout line $2'_C$ extending from the comparison electrode 2c shown in Figure 1. The diagram shows stable pulses indicating that the cells are capable of serving as a pacemaker. Figure 4(b) shows membrane potentials resulting from the pulses of a target cell in a normal state where the culture solution does not contain an agent. Here, a cell targeted for measurement is the cell $10_n$ of the cell communication channel CCC, where the potential between the readout line $2'_n$ extending from the cell $10_n$ and the readout line $2'_C$ extending from the comparison electrode $2_C$ are measured. As can be appreciated from comparison with the waveform of Figure 4(a), the time required for conducting the pulse of the cell 10 of the cell communication channel CCC is delayed by Δt. Meanwhile, Figure 4(c) shows membrane potentials resulting from the pulse of the target cell in a state where the culture solution contains an agent. Again, the cell targeted for measurement is the cell $10_n$ of the cell communication channel CCC for the sake of facilitating comparison with Figure 4(b). As can be appreciated from comparison with the waveforms of Figures 4(a) and 4(b), the time required for conducting pulse of the cell 10 of the cell communication channel CCC is found to be delayed not just by Δt but by Δt +α. This means that the level of the Na-ion inhibition due to the agent acting on the cell of the cell communication channel CCC appears as the increase in the delayed time, i.e., +α. Specifically, toxicity of an agent on a cardiomyocyte can be assessed as sodium-ion inhibition. It should be noted that microelectrodes that are used for observation may be referred to as observation electrodes herein.

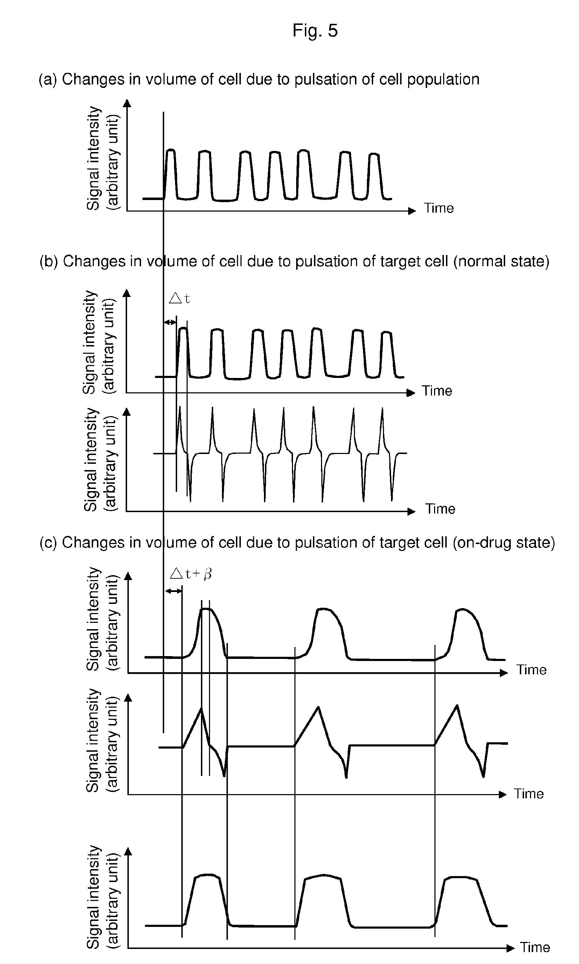

Figures 5(a), 5(b) and 5(c) are diagrams showing signals associated with the changes in the volume due to pulse of cells, which is measured with the optical system. Figure 5(a) shows the change in the volume associated with pulse of a cell of cell population $10_G$, where the pulse of one of the cells of the cell population $10_G$ is optically detected with the configuration shown in Figure 3. The contraction and dilatation associated with the pulsation of the cell can be observed as pulse-shaped changes. The cycle of this waveform is the same as the cycle of the changes in the membrane potential associated with the pulsation shown in Figure 4(a). Figure 5(b) shows, in the upper diagram, the change in the volume associated with the pulsation of the target cell under the normal state where the culture solution is free of the agent, and shows, in the lower diagram, a waveform of the same in time-differential values for evaluation as electric signals. Again, the cell targeted for measurement is the cell $10_n$ of the cell communication channel CCC, where the pulse of the cell $10_n$ is optically detected with the configuration shown in Figure 3. As can be appreciated from comparison with the waveform shown in Figure 5(a), the time required for conducting pulse of the cell 10 of the cell communication channel CCC is delayed by Δt. Meanwhile, Figure 5(c) shows diagrams for evaluating changes in the volume associated with the pulsation of the target cell under the state where the culture solution contains an agent. In Figure 5(c), the time axes are extended when compared to those in Figures 5(a) and 5(b). The upper diagram represents a waveform corresponding to the waveform of the upper diagram of Figure 5(b), where the time required for conducting pulse of the cell 10 of the cell communication channel CCC is further delayed by β in addition to Δt as can be appreciated by comparison with the waveform shown in Figure 5(a). The influence on the change in the volume associated with the pulsation of the target cell is more prominent in a smaller inclination of the change in the volume rather than the increase in the delay. This is apparent from comparison with the change in the volume with an agent-free culture solution shown as a reference waveform in the lower diagram in Figure 5(c). The middle diagram of Figure 5(c) shows the waveform of the upper diagram processed as time-differential values for evaluation thereof. As can be appreciated from comparing the time-differential values with those shown in the lower diagram of Figure 5(b), smaller peak values are associated with increased gentleness in the inclination. This means that the agent decreased the contraction rate of cardiac muscle and therefore the cardiac output is also decreased. In other words, toxicity of an agent on the cardiomyocyte can be evaluated as a decrease in the contraction rate.

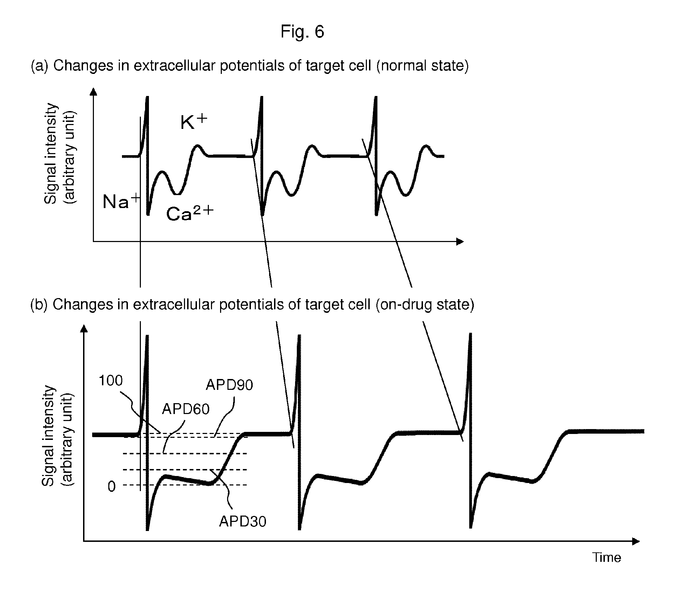

Figure 6(a) shows changes in the potentials according to the amounts of $Na^+$, $Ca^{2+}$ and $K^+$ ion in- and out-flow into/from the target cells under a normal state where the culture solution is free of agent. Figure 6(b) shows changes in the potentials according to the amounts of $Na^+$, $Ca^{2+}$ and $K^+$ ion in- and out-flow into/from the target cells under a state where the culture solution contains an agent. As can be appreciated by a cursory comparison of Figures 6(a) and 6(b), QT prolongation emerges where the waveform is extended along the time axis. Moreover, the waveform is largely deformed due to in- and out-flow of the $K^+$ ions. In order to evaluate this as an electric signal, the durations of the detected 30%, 60% and 90% values are shown as APD30, APD60 and APD90, respectively, with respect to the broken lines indicating the values between "0" and "100" in the diagram. Here, APD stands for action potential duration. Evaluations of the magnitudes and percentages of these values can provide evaluation of influence of the agent on the amounts of the $Na^+$, $Ca^{2+}$ and $K^+$ ion in- and out-flow.

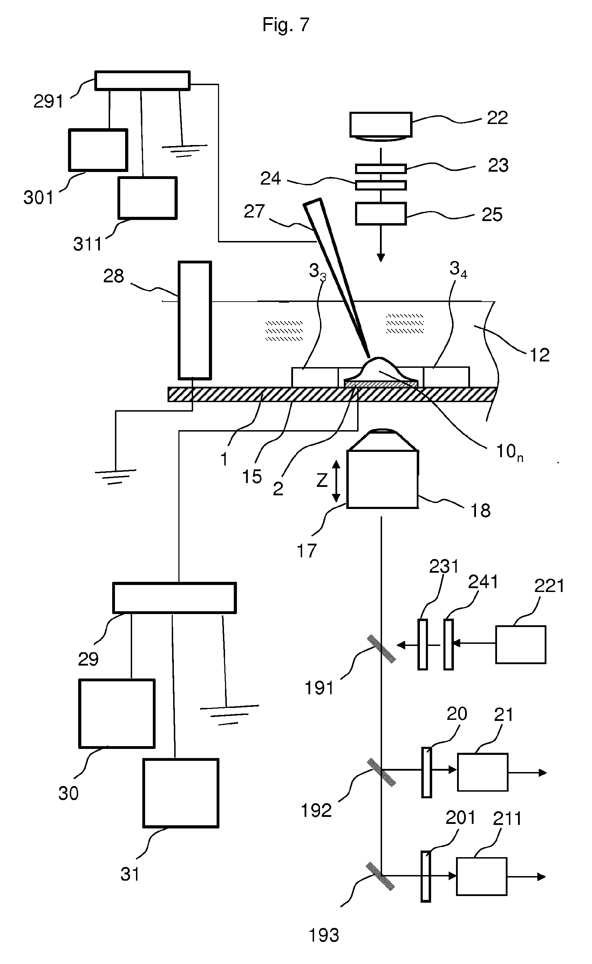

Figure 7 is a view illustrating an exemplary arrangement of an optical system and a movable electrode of the cardiotoxicity testing apparatus for optically detecting the cells, in which observation of the pulsating state, for example, of the cell $10_n$ to be measured is exemplified. Reference numeral 12 denotes a culture solution. Reference numeral 22 denotes light source for a phase-contrast microscope or a differential interference microscope, which is generally a halogen lamp. Reference numeral 221 denotes a fluorescent light source for fluorescent measurement of the cells, which is generally a mercury lamp, a monochromatic laser, an LED light source, or the like. Reference numeral 23 denotes a bandpass filter that allows transmission of only light with a particular wavelength from the light source for observation with a stereoscopic microscope such as a phase-contrast microscope, while reference numeral 231 denotes a bandpass filter that allows transmission of only light with an excitation wavelength that excites particular fluorescence from the fluorescent light source 221. For example, when observing the change in the shape such as information of change in the volume of the pulse of the cell $10_n$, an image that passed the bandpass filter 20 that allows only light with a wavelength for measuring the cell shape is measured with the camera 21 on a real-time basis, where narrowband light having the wavelength in the vicinity of 700 nm is used for measurement to prevent damage of the cell $10_n$. Reference numerals 24 and 241 denote shutters that have a function of blocking irradiation light when image measurement is not executed, for example, while moving the XY stage 15. Reference numeral 25 denotes a condenser lens, where a phase ring is installed for phase-contrast observation or a polarizer for differential interference observation. In the case of fluorescent measurement, for example, in the case of intracellular calcium release measurement, a combination of a bandpass filter that selectively passes light with the excitation wavelength of approximately 500 nm and a bandpass filter that selectively passes light with the fluorescent measurement wavelength of approximately 600 nm is used, to measure, with the camera 201, the fluorescent image that passed through the bandpass filter 201 that only selectively passes light with the fluorescent wavelength. In this case, if calcium release per cell unit in the cell network is to be measured in terms of time to determine the pathway of the signal conduction in the cell network, continuous high-speed images can be acquired with the time resolution of the camera being 0.1 ms or less. The cardiotoxicity testing apparatus 100 formed on the substrate 1 is mounted on the XY stage 15 which can be moved with the X-Y drive unit 16 to observe and measure certain location of the cardiotoxicity testing apparatus 100. The pulsating state of the cell $10_n$ in the cardiotoxicity testing apparatus 100 is observed with an objective lens 17. The focal position of the objective lens 17 can be transferred in the Z-axis direction with a drive unit 18 according to the signal from the personal computer PC. The magnification of the objective lens 17 may be 40 or higher. The objective lens 17 allows observation of a phase-contrast image or a differential interference image of the cell $10_n$ obtained with light transmitted from the light source 22. A diachronic mirror 192 and a bandpass filter 20 that reflect light with the same wavelength as the light that passes through the bandpass filter 23 allow observation with a camera 21 of only a phase-contrast microscope image or a differential interference microscope image. The image signal observed with the camera 21 is input into the personal computer PC. Moreover, according to this example, a movable electrode 27 for stimulating a cell is arranged with a position controlling mechanism for adjusting the coordinates of the movable electrode with respect to not only within the plane parallel to the plane of the XY stage but also with respect to its height. Using this position controlling mechanism, the tip of the movable electrode is transferred to stimulate one or more particular cells in the cell network. The movable electrode may be a metal electrode provided with an insulating coating except for the tip, a glass electrode having the opening size of the tip of about 5 micrometers or less, or the like, where any electrode that can apply electrical stimulation only to a particular cell or cells in the vicinity of the tip of the movable electrode can be used. When a metal electrode is used, platinum black or the like may be applied to the tip surface for effectively transmitting electrical stimulation to the cell(s). The positioning of the tip of the movable electrode can be adjusted according to the level of the response of the cell(s) to the electrical stimulation, and may make a contact with the cell(s) or placed near the cell(s). In addition, in order to accurately apply stimulation from the stimulation electrode to the target cell(s), the electrode 2 for measuring the membrane potentials may be used as a ground electrode by switching the electrode at the moment of applying electrical stimulation, or a separate ground electrode 28 may be provided. Moreover, in order to stimulate a particular cell, the existing microelectrode 2 may be used as a stimulation electrode. In this case, the switching circuit 29 connected to the microelectrode is switched upon stimulation so that the microelectrode that is usually connected to an electric signal measurement circuit 30 is connected to an electrical stimulation circuit 31 for applying square-wave stimulation signals to the microelectrode 2. Furthermore, when the movable electrode 27 is used to provide stimulation, the switching circuit 29 may be switched to a grounding state. On the other hand, the movable electrode may also be used not only as a stimulation electrode, but also as an electrode for measuring the electric signal of the cell(s) or as a ground electrode. In this case, the movable electrode is connected to a switching circuit 291, and switched, according to its use, i.e., for membrane potential measurement, for cell stimulation or as a ground electrode, is connected to an electric signal measurement circuit 301 to measure the membrane potential, is connected to an electrical stimulation circuit 311 for applying a square-wave stimulation signal to the cell(s), or is grounded for use as a ground electrode, respectively. The timing of the electrical stimulation applied to the cells with the electrical stimulation circuits 31 and 311 can be employed primarily for the following two applications. One is to apply irregular stimulations between the pulse intervals of the normal cardiomyocyte network in an autonomous pulsation configuration. The other is to provide pulse interval to the cardiomyocyte network without an autonomous pulsation configuration. In both cases, changes in the response of the cell network can be traced through measurement by gradually shortening the cycle of the pulse interval (time interval between two pulses) by 5 ms. In order to do so, the electrical stimulation circuits 31 and 311 can analyze the pulsation cycle information acquired with the electric signal measurement circuits 30 and 301 and conduct feedback regulation based on the acquired results to determine the timing of the stimulation. Moreover, when the movable electrode 27 is used for the electric signal measurement, measurement can equivalently be carried out in the present system without the microelectrode 2. Since the pulsation cycle of each cell in the cell network can be measured by the optical measurement installed in the system, a change from a stable state to an unstable state such as abnormal cardiac rhythm in this pulsation cycle can be measured only with the optical measurement device arranged in the system. Then, if necessary, the movable electrode is used to acquire the data of the electric property of the particular cell from these results. In this case, the number of the microelectrodes arranged on the system in the first place is not limited, and a larger cell network can be configured freely as long as optical measurement is possible.

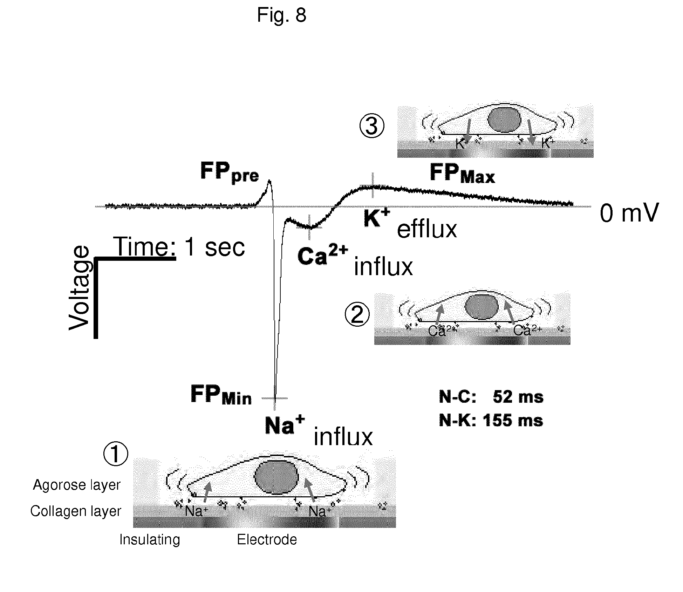

Figure 8 shows a schematic view of an example of generation of an electric signal of a cell. First, inflow of sodium ions into a cell occurs via sodium-ion channels on the cellular membrane, where the membrane potential is rapidly decreased. Then, the membrane potential is decreased after a slight delay due to inflow of calcium ions, and then as the subsequent step, outflow of potassium ions from the cell occurs where the membrane potential is increased. The changes in the membrane potentials occur due to the different response imparted by the properties of various ion channels present in the cardiomyocyteular membrane. By analyzing the positions of the peaks of change in the potentials caused by the respective ion channels as time characteristic of the ion channels, the changes in the waveforms of the electric signals can be measured for each type of the ion channels that are blocked due to the effect of the agent. As a result, an inhibition effect of the agent on the ion channels can be estimated. There are four particularly important ion channels for evaluation of an agent, i.e., FastNa, SlowNa, Ca, IKr and IKs. Blocking of these four types of ion channels can be measured.

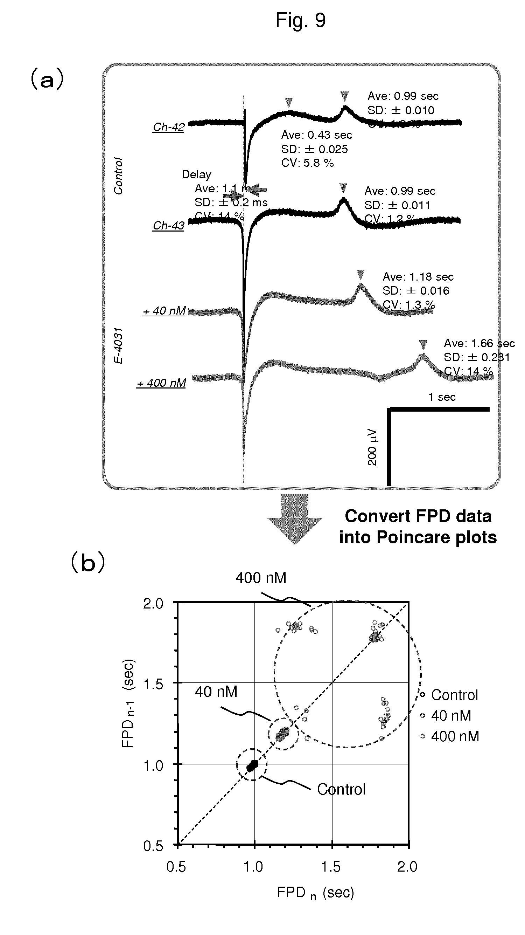

Figure 9(a) shows the influence on the electric signals of the cell shown in Figure 8 upon actual addition of reagent E-4031 at various concentrations that selectively inhibits the potassium-ion channels. Since the IKr-ion channel that is responsible for outflow of K-ion from the cells and that increases the membrane potential is inhibited, a change in the membrane potentials can be observed to be gradually delayed in the positive direction as the concentration of the agent increases. Figure 9(a) shows data of a particular single pulsation of a cellular response. In practice, the magnitude of the fluctuation width of the responses between the successive pulses is an important index for estimating the influence of the agent. Figure 9(b) shows one example of an analysis technique where successive pulse data called Poincare plots are compared correlatively. Here, the X-axis represents plots of response time of a particular ion channel upon the n-th pulse while the Y-axis represents plots of the response time of the same ion channel upon the (n + 1)-th pulse. Accordingly, if the properties of the successive pulses are the same, the plots will be drawn along the Y = X line represented by the broken line in the graph. If there is a significant fluctuation in the responses between the successive pulses, the plots observed will be placed distant from the Y = X line. In fact, in this example, although addition of 40 nM results in the delay of the response time as compared to the control without addition of the agent, homology between the successive pulses remains the same. At the same time, these plots reveal that addition of the agent up to 400 nM further delays the response time, and homology is no longer retained between the successive pulses, resulting in generation of an unstable pulsation cycle. This result agrees with the results of prolongation in the QT interval measurement representing cardiac toxicity. Generation of a prolongation of the QT interval can be estimated by using the Poincare plots as an index of increase in the fluctuations of the successive pulses at a cellular level. This phenomenon can be described as follows: when a particular ion channel is blocked with an agent, only a phenomenon of decrease in the ion outflow ability is observed where the degree of the blocking is small and the cell response is not yet unstable. In contrast, when the degree of blocking increases as the number of functioning ion channels becomes extremely decreased, the reproducibility of the ion outflow ability deteriorates and fluctuation for the same cell increases. Hence, the magnitude of this fluctuation can be used as an index of likelihood of generating a prolongation of QT interval.

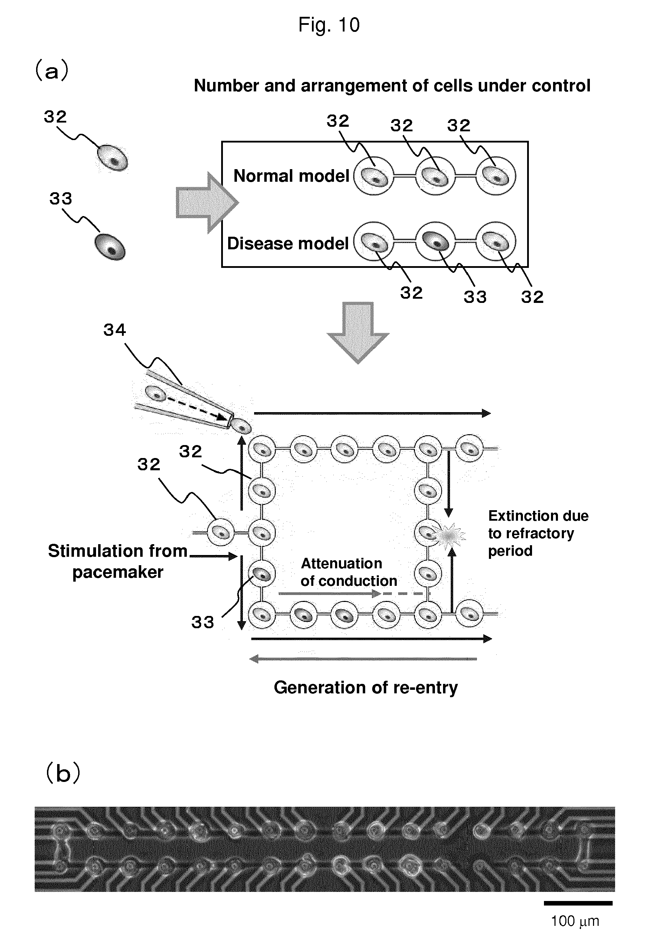

Figure 10(a) is a schematic view showing an example of an agent for a re-entry circuit with an annular network of cardiomyocytes using a cell arrangement technique at a single-cell level. An annular network produced with only cardiomyocytes is used as a normal network model. A pathologic model such as cardiac hypertrophy is realized by incorporating fibroblast cells into the cell network. The fibroblast cells present in the network will cause delay of the conduction velocity or attenuation of the conduction of the cardiomyocyte network, as a result of which, generation of premature contraction can be estimated. Figure 10(b) is a microscopic picture showing an example of actual arrangement of cardiomyocytes on the microelectrodes. In fact, when the cells are arranged on the microelectrodes in cell units as shown in this picture, delay in the signal conduction between the adjacent cardiomyocytes can be measured. Since this conduction velocity depends on the magnitude of the first electric signal generated upon pulsation, data for delay in this signal conduction can be interpreted as the inhibitory effect on the sodium-ion channel.

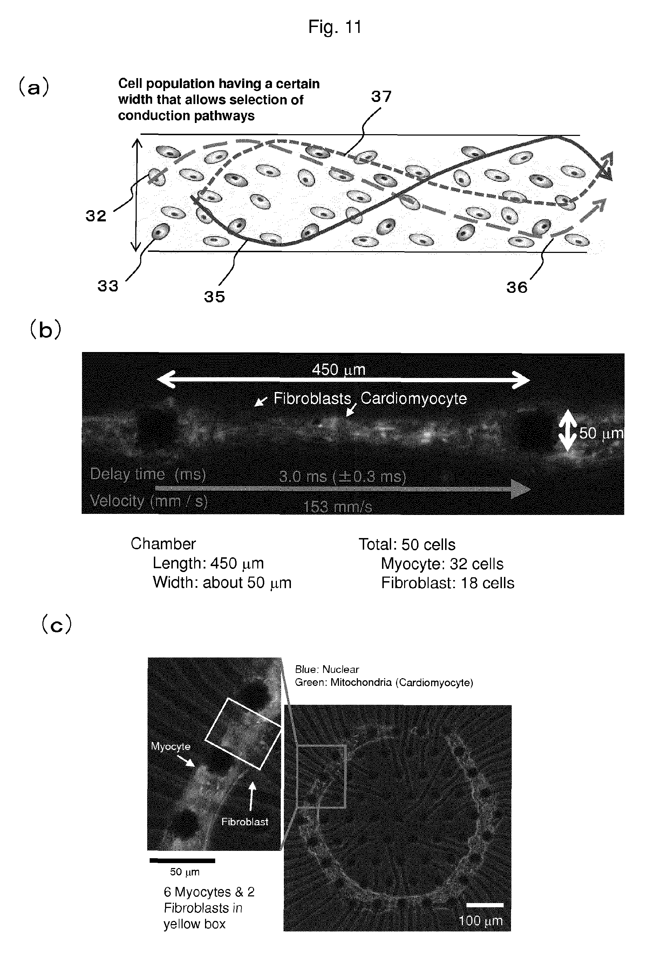

Figure 11(a) is a schematic view showing an exemplary re-entry circuit by an annular network of cardiomyocytes using a cell population having a certain width. In the annular cell network in cell units shown in Figure 10, pulsation signals of the cardiomyocytes are uniquely transmitted, and the cells will transmit pulsation signals between the adjacent cells while maintaining the same property unless there are fluctuations in the pulses of the cells themselves as shown in Figure 9. On the other hand, when the cells were arranged with a certain width to form an annular network as shown in Figure 11, the cell population will be imparted with the flexibility to have different conduction pathways for different pulses as represented by solid line 35, broken line 36 and dotted line 37. In particular, when a large fluctuation occurs in the response property of each cardiomyocyte due to the addition of an agent as described with reference to Figure 9, the cells that are likely to respond differ in response to the travel of the stimulation signals through the annular network, thereby rendering the difference in the pathways significant. Since this is the same mechanism as the mechanism of premature contraction, i.e., a fatal cardiac status called spiral/re-entry, measurement of spiral/re-entry becomes possible by particularly using an annular network based on cell population having such a width. Figure 11(b) is a microscopic picture showing an actual exemplary arrangement of the cell population on the microelectrodes, in which the cell population has about 60% cardiomyocytes and about 40% fibroblasts. In fact, such an arrangement increases fluctuations between successive pulses in the conduction velocity between adjacent electrodes. Since the increase in the fluctuation becomes significant particularly by the addition of the agent, generation of spiral/re-entry can be estimated according to the change in the fluctuation width of the conduction velocity between successive pulses. Figure 11(c) is a microscopic picture showing another example of the actual annular arrangement of the cell population on the microelectrode array. For actual measurement of spiral/re-entry, calcium spike firing in each cell of the cell population network can be estimated at the single-cell level by using the high-speed fluorescent measurement camera shown in Figure 7. As a result, actual analysis of the pathway taken by the signal conduction of the cells and actual analysis of the change in the pathways at each round can be realized.

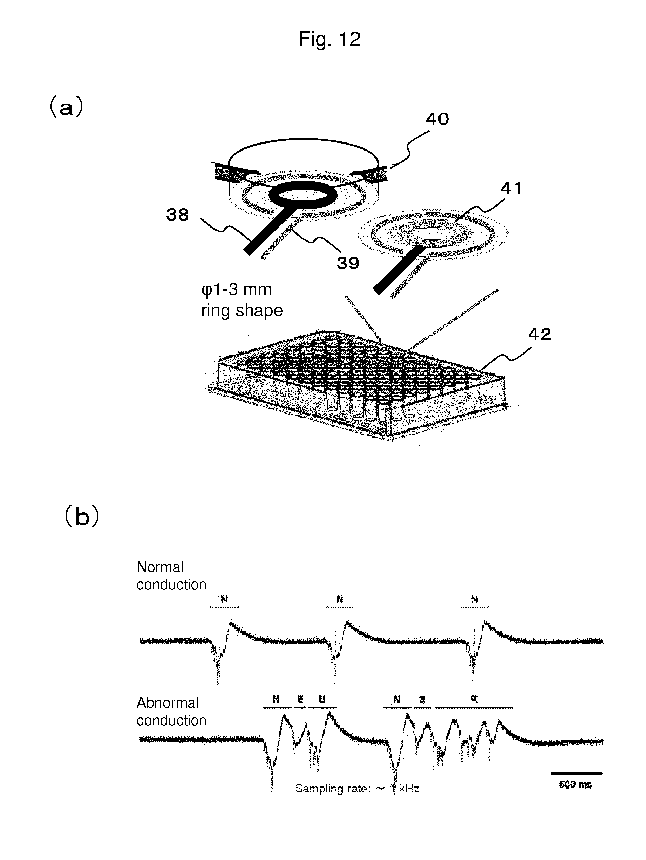

Figure 12(a) is a schematic view showing an exemplary re-entry circuit measurement device using an annular electrode. In this example, an annular electrode 38 with an electrode width of 50-100 micrometers is formed into a ring shape to have a diameter of 1-3mm and arranged on each of the bottom surfaces of a 96-well plate 42. The bottom surface of the plate other than the electrode is coated with a non-cell-adhesive material such as agarose so that the cell population 41 is annularly placed only on the electrode surface. A reference electrode ring 39 is placed concentrically on this non-cell-adhesive coated region, and a flow passage 40 is provided for entrance and exit of a reagent. By using such an electrode, abnormal pulsation of a cardiomyocyte can be simply and conveniently measured. Figure 12(b) is a graph showing normal pulse data and abnormal pulse data actually measured with the electrode. Although an annular electrode is used in this example, a system for optically measuring abnormal pulsation which is equivalently effective as this annular electrode can be constructed by using the optical measurement system shown in Figure 7. In this case, an electric signal to be measured can be acquired by allowing the moving electrode shown in Figure 7 to make contact with the annular cell network.

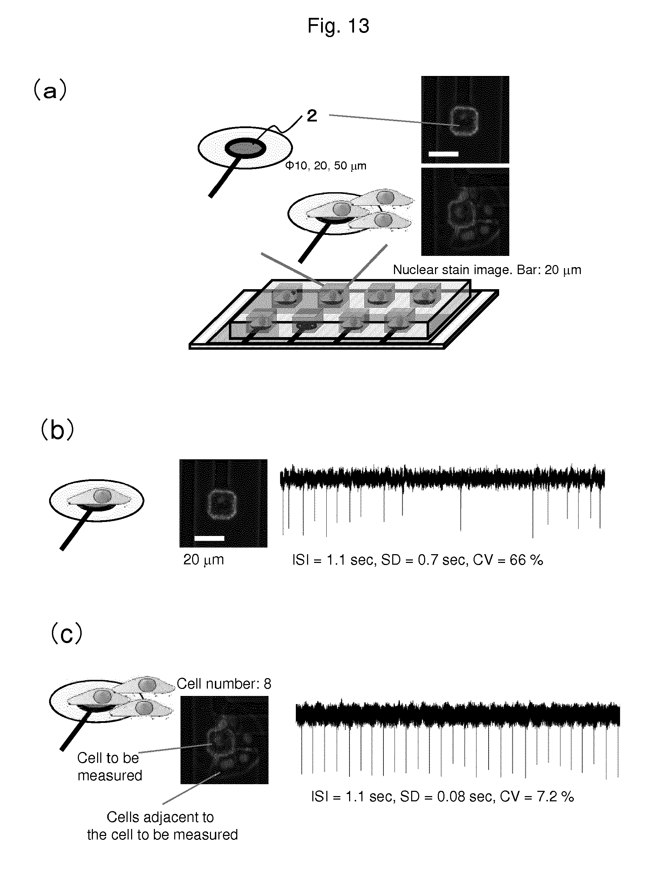

Figure 13(a) is a schematic view showing an exemplary arrangement of a cell and a microelectrode 2 for measuring a potential of a single cell, which illustrates a measurement technique in which a single cell targeted for measurement is arranged on the microelectrode 2 with a diameter of 10 to 50 micrometers. Again in this example, likewise in other examples, the area of the bottom surface other than the electrode is coated with a non-cell-adhesive material such as agarose such that the cell is retained on that place on the electrode. Figure 13(b) is a view of an isolated single cell on the electrode which was actually measured with the microelectrode 2, and electric pulse data thereof. Signals from the isolated single cell are unstable and pulses undergo a large fluctuation as shown in the graph. On the other hand, in Figure 13(c), a single cell is placed on the microelectrode 2 as in Figure 13(b) but to thereby form a cell population with other cells and realize stability of the pulsation cycle as can be appreciated from the pulsation signal graph. In an actual pulse measurement at the single-cell level, the magnitude of the fluctuation between successive pulses serves as an index as shown in Figure 9. Therefore, as described in the present example, a measurement system is useful in that only a specific cell to be measured is placed on the microelectrode while other cardiomyocytes are not provided on the electrode to thereby maintain stability of the specific cell. Accordingly, pulse data of a single cell can be acquired while realizing stability by providing a cell population.

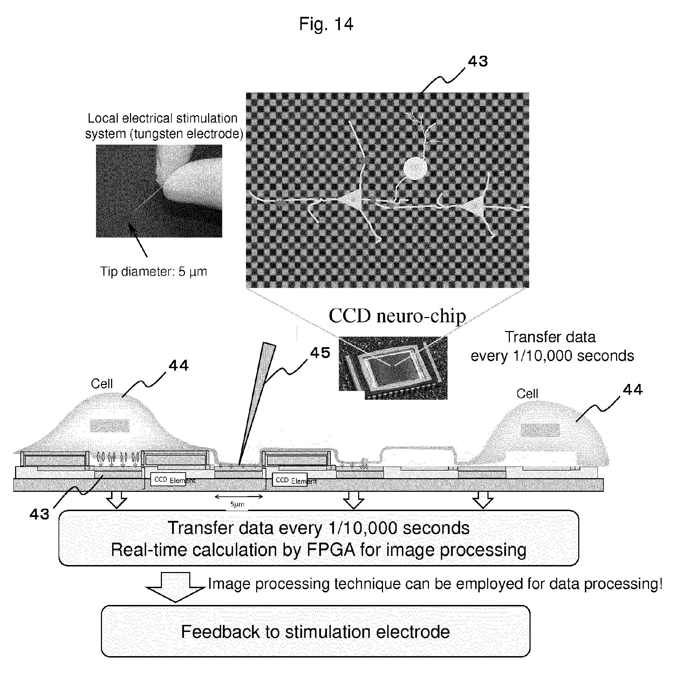

Figure 14 is a schematic view for illustrating an example using a photo-sensitive element of the camera for measuring a potential of a single cell according to the present invention. In general, a photo-sensitive element of the camera converts a light signal into an electric signal on a photoelectric conversion surface to use this electric signal for measurement. This photoelectric conversion surface can be removed and an electric signal array can be used to obtain an electric signal in two dimensions. Therefore, since an electrode array at the single-cell level can be used, for example, a change in the signal conduction pathway in the cell population network with certain spaced intervals as shown in Figure 11, i.e., generation of spiral/re-entry, can be measured, which requires simultaneous measurement of electric signals of respective cells in the cell population. The required interval for pixel measurement in an actual measurement is about 1/10,000 seconds, and thus a photo-sensitive element of a high-speed camera with a shutter speed of 1/10,000 seconds is required. In this case, an image processing technique employed in conventional cameras can directly be applied to the acquired signal data of the cells, which allows real-time processing using FPGA for image processing. In addition, feedback stimulation can be applied to the stimulation electrode based on the data obtained by this real-time processing.

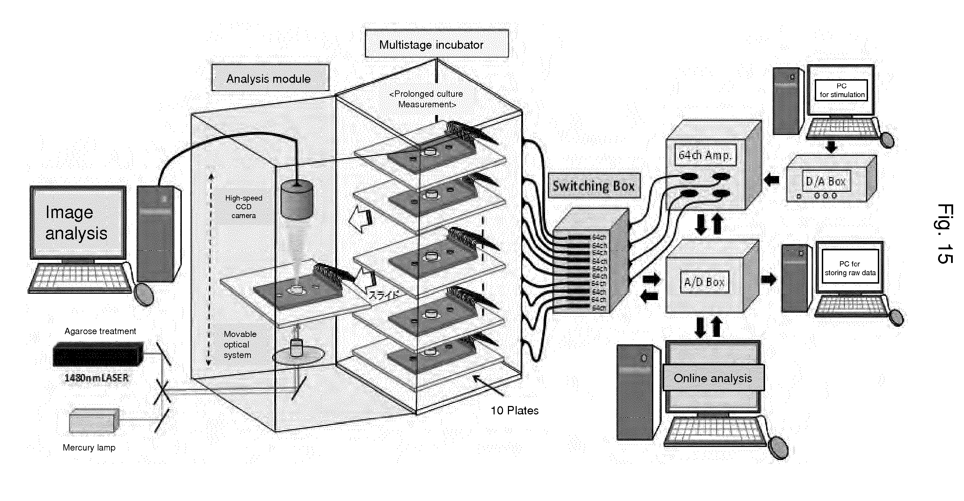

Figure 15 is a schematic view for illustrating an exemplary mechanism for measuring a plurality of samples with a cell measurement system of the present invention. The system of this example comprises an analysis module, a multistage incubator, an electroanalysis module and an online analysis module connected thereto via an online network. Here, the analysis module comprises a phase-contrast microscope or a differential interference microscope for measuring changes in the cellular shape, optical measurement means associated with a fluorescent microscope and a camera photography analysis, and an agarose processing technique that can locally dissolve agarose at a micrometer scale with a microscopic system. Multiple cell culture baths are arranged in the multistage incubator, where microelectrode chips are arranged in the cell culture bath such that measurement of electric signals of each cell and electrical stimulation can be sequentially processed in parallel in the incubator. The obtained electric signals are subjected to real-time measurement in the electroanalysis module, and resulting data are recorded in a storage that is accessible online such that the results of optical measurement data and electric measurement data are recorded with the same time stamp. The analysis module can appropriately access to these record data online for analysis.

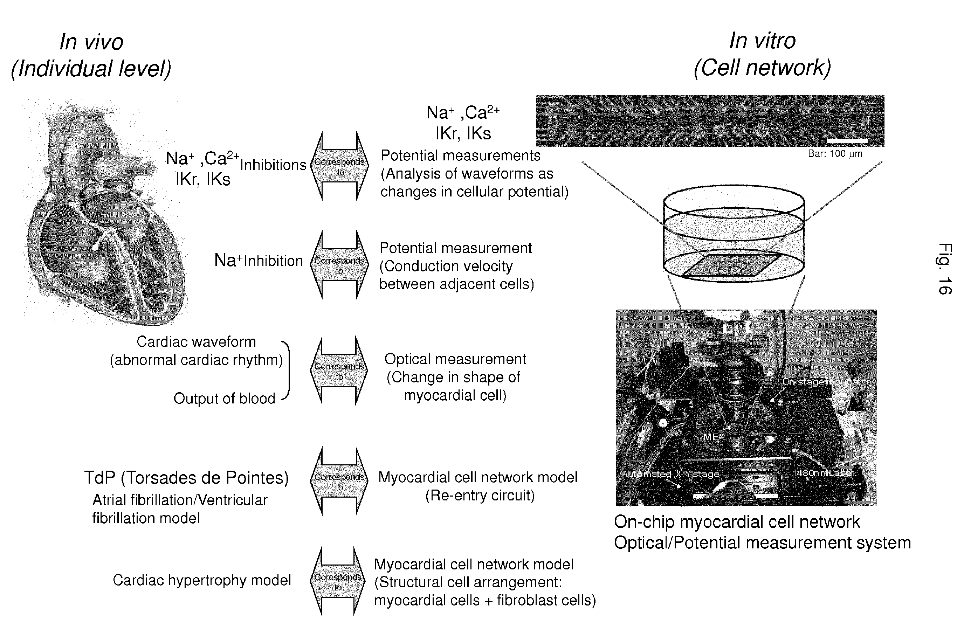

Figure 16 is a schematic view for illustrating information of heart measured with a cell measurement system of the present invention. Electric signal measurement for a single cell on a microelectrode enables measurement of signal data of ion channels such as Na-, Ca-, IKr- and IKs-ion channels and sodium-ion channel inhibition can be measured by measuring the changes in the signal conduction velocities between adjacent cardiomyocytes. In addition, optical measurement of the change in the shape of a single cell allows measurement of the generation of abnormal cardiac rhythm as well as estimation of cardiac output. Furthermore, generation of re-entry can be measured by annularly arranging the cell network. Moreover, measurement as a cardiac pathologic model such as cardiac hypertrophy can be realized by adding fibroblast cells to the cell arrangement.

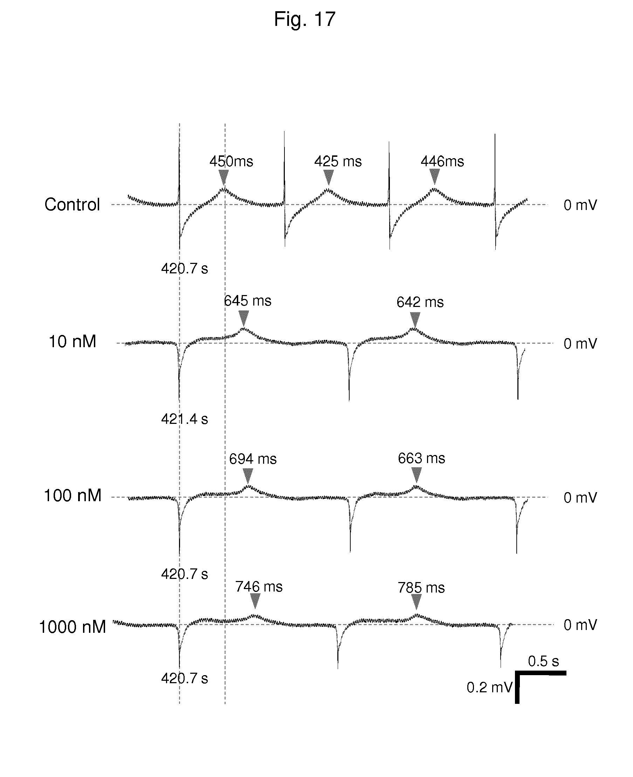

Figure 17 is a graph illustrating an example of changes in the field potential (FP) signal waveform of cells obtained from autonomously pulsating cardiomyocytes in response to the addition of agents in accordance with the cell measurement system of the present invention. The signal waveform of the field potential of the cells shows a change in a membrane potential generated by ions flowing into the cells and ions flowing out of the cells as shown in Figure 8. The signal waveform represents a differential value of the membrane potential, i.e. the sum of an ion current flow per unit time. In this case, the inward ion current, such as sodium or calcium ions or the like in the process leading to depolarization, tends towards negative, and the outward ion current, such as potassium ions in the subsequent process of repolarization, tends towards positive. As shown in Figure 17, information for the FP signal waveform of the cells is usually extracted as one FP waveform as a mean value of a plurality of adjacent waveforms to eliminate the effects of noise components or differences in adjacent waveforms, rather than focusing on the differences between each other for each adjacent pulsation, and detailed analysis of one waveform reflecting the average value is used to estimate the state of each of the ion channels. However, in the present invention, rather than acquiring the average value of the adjacent FP signal waveforms, the adjacent FP signal waveforms are compared and any difference due to the fluctuation of the response of the ion channel is extracted. Based on the size of the fluctuation, the amount of the blocked ion channel is estimated quantitatively. The magnitude of the fluctuations is in general represented by $[1/(n)^{1/2}]$, the reciprocal of the square root of n elements, to facilitate comprehension thereof. That is, given that when $10^4$ channels of the number of ion channels in the cell surface are working, for example, the magnitude of the fluctuation of the function as a sum of the ion channels will be 1% $[1/(10^4)^{1/2}]$, while if the number of the working ion channels decreases to $10^2$ due to blockage by an agent, then the magnitude of the fluctuation increases sharply to 10% $[1/(10^2)^{1/2}]$, resulting in a big change in its feature of the adjacent FP waveform. In other words, if it is possible to estimate the magnitude of the fluctuation by comparing the change in the adjacent FP waveform, then the total amount of ion channels that are blocked can be estimated from the magnitude of the fluctuation.

For the change of the adjacent waveform, focusing on the location of the peak of the outward ion current generated by the release of potassium ions, in particular, taking the time at which sodium ions flow into the cell as a reference (zero), for example, and defining the time from the reference point to the peak of the emission potassium ions as field potential duration (FPD), then the change in the length of the FPD will be the peak value of the inflow of potassium ions subsequent to in- and out- flows of ions such as sodium ions and calcium ions. It can thus be used as an indicator of the amount of change as the sum of the change in in- and outflows of the ions generated by blocking of various ion channels on cells by an agent. In addition, this fluctuation of the position of the FPD reflects the sum of the fluctuations of the adjacent FP waveforms of all the involved ion channels of the cell. In fact, when the position of the FPD (position of the red arrowhead) in Figure 17 is checked, it is seen that the FPD is between 425-450ms prior to the addition of E4031, which is an inhibitor of potassium ion channels, but then became 642-645ms due to the addition of 10nM, 663-694ms due to the addition of 100nM, and 746-785ms due to the addition of $1\mu$M E4031. Thus, the value of the FPD increased monotonically due to the addition of the inhibitor. Consequently, adjacent FPDs will not take the same value, but will take a different value to reflect the fluctuation.

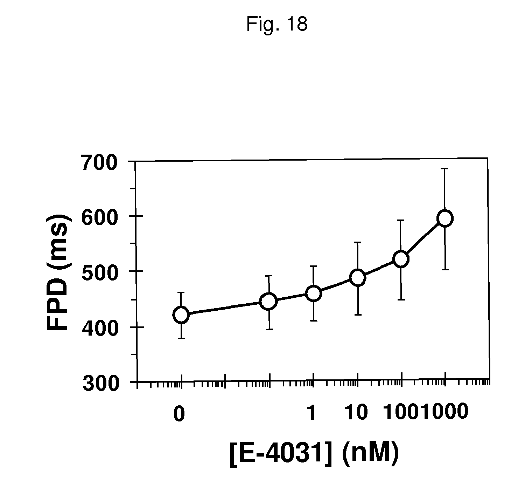

Figure 18 shows an actual example of the experimental results of E4031-concentration-dependency on the prolongation of the FPD when potassium ion channels of the cell were inhibited by an E4031 agent having the ability to specifically inhibit potassium ion channels. Here, it is estimated that the ion outflow is delayed by the inhibition of the potassium ion channels, and the FPD is prolonged in a concentration-dependent manner. Next, measurements of fluctuations in relation to the results of this experiment will be described in the same manner as above.

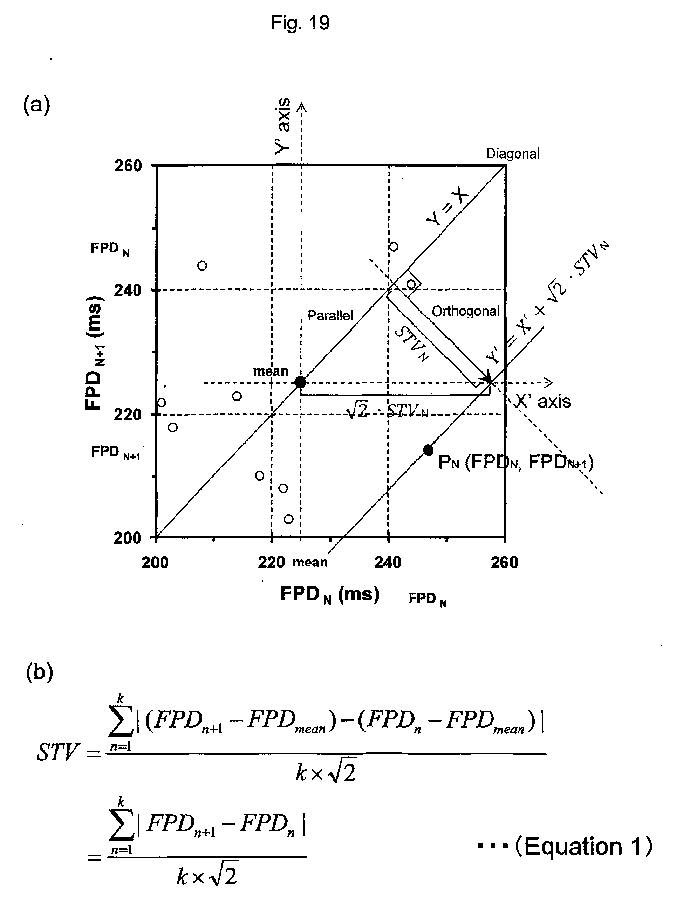

Figure 19 illustrates the estimation of the magnitude of the fluctuation of the adjacent pulsations (short-term variability: STV) among other points of interest in estimating to what extent the FPD of adjacent pulsations shift from a homologous state when the fluctuation of the FPD is observed using the Poincare plotting for measuring the fluctuation of pulsation in the electrocardiogram in general to evaluate the value of the FPD in the FP waveform. In Figure 19 (a), the diagonal, in which X = Y, corresponds to the case where the size of adjacent pulsations $FPD_n$ and $FPD_{n+1}$ have exactly the same FPD size, and the vertical distance of the magnitude of the difference between two FPDs (i.e., $FPD_{n+1} - FPD_n$) from the diagonal is the size of the standardized fluctuation of the adjacent pulsation itself. In particular, for the number of samples "k", it can be evaluated by a formula such as the formula (1) shown in Figure 19 (b).

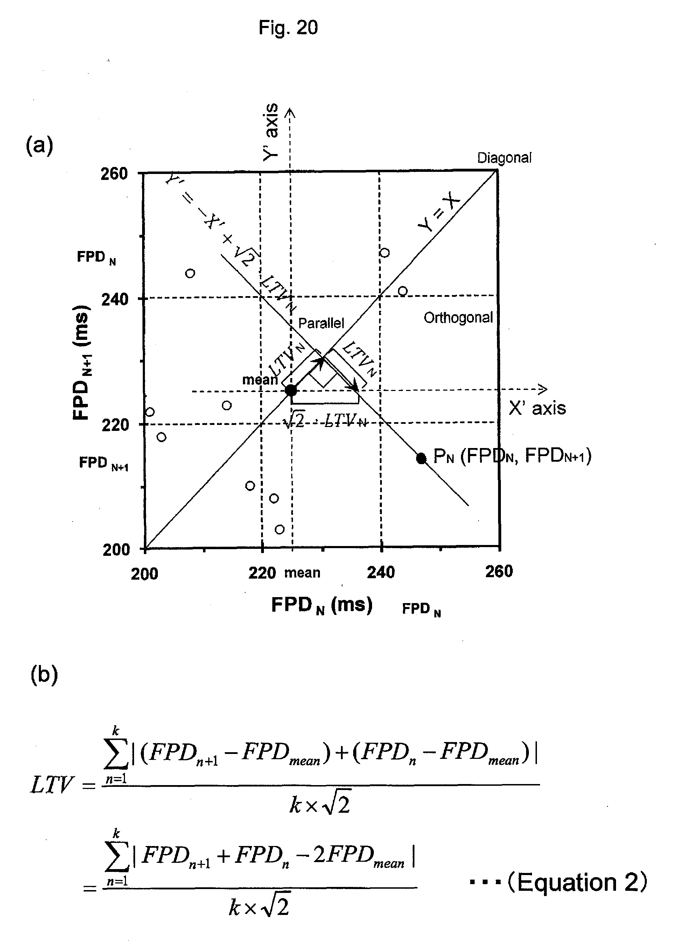

On the other hand, Figure 20 illustrates, among other methods for estimating to what extent the FPD of adjacent pulsations shifted from a homologous state, how to estimate the magnitude of the fluctuation of pulsations (: Long-Term Variability: LTV) in terms of to what extent each adjacent pulsation is shifted from the average value of the pulsations (the sum of all samples and corresponding to the ideal value of the response of the ion channel) when the fluctuation of the FPD is observed using the Poincare plotting. In Figure 20(a), the magnitude of $[(FPD_{n+1} - FPD_{mean}) + (FPD_n - FPD_{mean})]$, which are the two distance values between two FPD values, i.e., adjacent pulsation $FPD_n$ and $FPD_{n+1}$, respectively, and $FPD_{mean}$, the average value of the FPD, which corresponds to the diagonal X = Y, is the magnitude of fluctuation from the mean value of the FPD and the vertical distance from the diagonal which has been normalized. In particular, with respect to the number of samples "k", it can be evaluated by the formula 2 of Figure 20 (b). This shows the deviation from the symmetry of X = -Y, and this size can be used as an index to find out whether or not it is merely a fluctuation of beating near the average value, or whether there is an historical correlation.

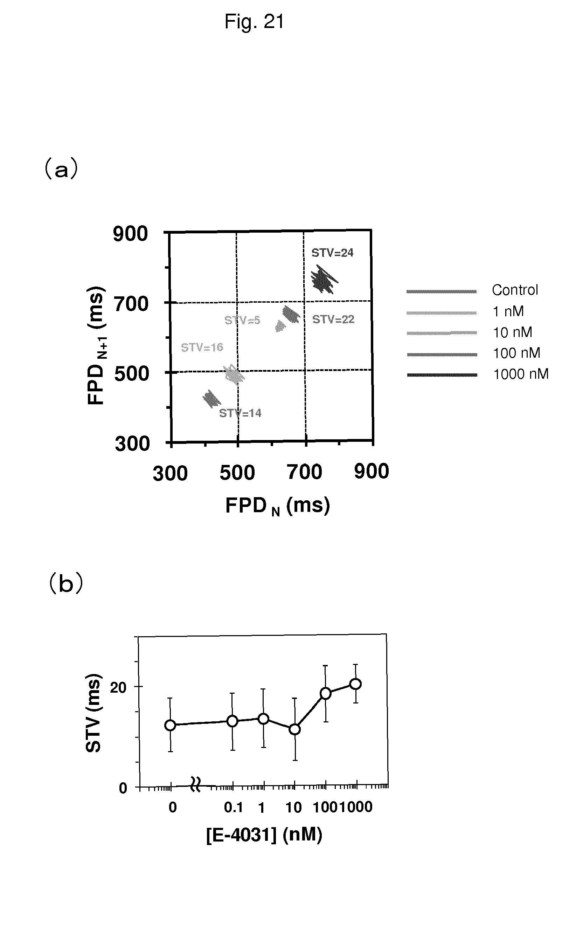

Figure 21 shows, in Poincare plotting, one example of the fluctuation of the FPD of the response of cardiac muscle cells when E4031 was actually added stepwise; and a quantitative summary as STV. It can be seen that it is estimated that ion channels are blocked in response to the addition of E4031 by an prolongation of the length of time of the FPD, while the value of the STV increases rapidly by, in particular, the addition of high concentrations.

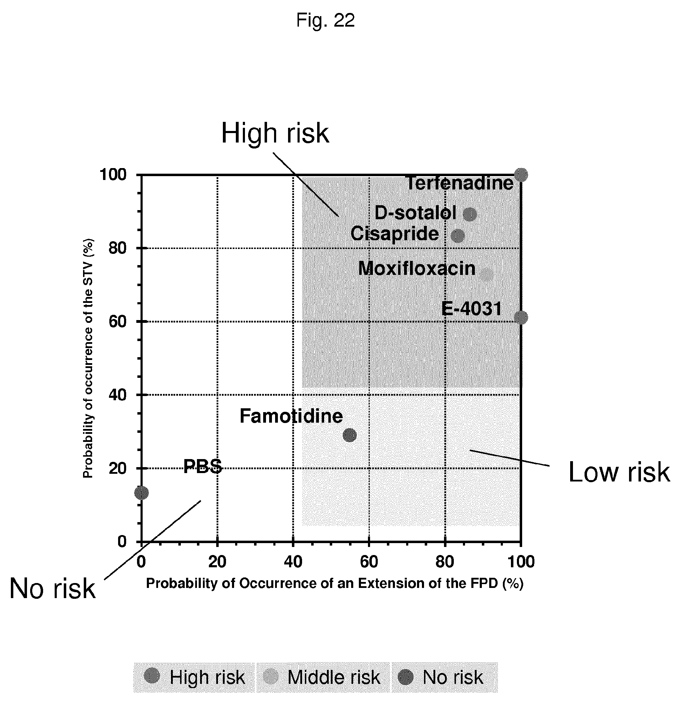

Figure 22 shows an example of an evaluation of chemical agents that are known to have cardiac toxicity and those that are known to have no cardiac toxicity wherein the X-axis is the percentage (%) of observed prolongation of the FPD using the cardiomyocytes, which corresponds to conventional measurement of QT prolongation, and the Y-axis is the percentage (%) of observed increase in the STV. In a conventional agent toxicity test, the evaluation is made only with the results of the data of the FPD on the X-axis. When the evaluation is made with additional results of the STV on the Y-axis, as can be seen from the figure, it is found that there are three areas, i.e., areas for high risk (High risk), low risk (Low risk) and no risk (No risk) of cardiotoxicity in a two-dimensional mapping on a graph, the same distribution as the known result from the relevant literature. From this result, it is found that a more accurate and simplified prediction on the probability of cardiac toxicity of an agent is possible by use of the STV in addition to the conventional FPD.

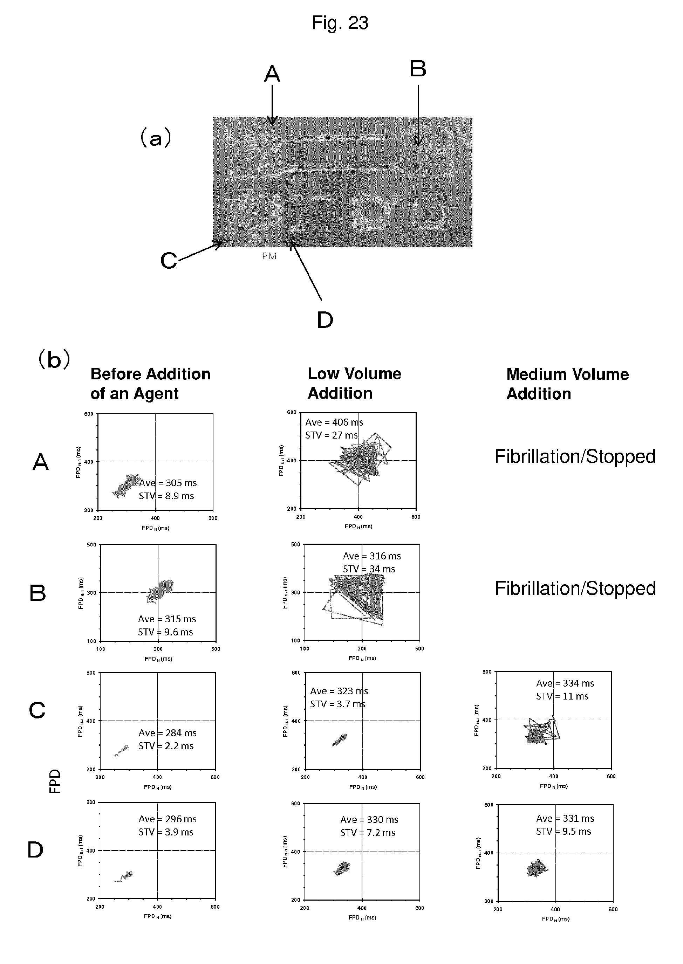

Figure 23 shows the differences in the responses of the STV with regard to the FPD in response to addition of agents. Figure 23 (a) shows an example of a Poincare plotting (A, B) for the FPD for a local portion where a cardiomyocyte-network has been constituted, and (b) a Poincare plotting (C, D) of a local portion of a myocardial sheet having a two-dimensional sheet configuration. In this example, B and D are located near, and A and C are located separated from the pacemaker area PM. In (b), a large fluctuation in FPDs, that were distributed on the diagonal of X = Y of a Poincare plotting prior to the addition of the agent, is observed to occur in both the annular models (A, B) by the addition of low volume of a cardiac toxic agent, and an increase in the STV is observed, while little fluctuation occurs in the two-dimensional sheet model (C, D). In response to addition of an agent in a medium volume, the pulsation changes to a fibrillation state or a stopped state in the annular model (A, B), while an increase in the STV is observed in the area C located away from the PM in the two-dimensional sheet model (C, D). A lower rate of an increase in the STV than the area C is observed in the vicinity of the area D. As can be seen from this example, as for prediction of cardiac toxicity of an agent by measuring the STV of the FPD, it is evident that a population (network) of cells which are arranged linearly from the pacemaker area reflects more accurately the effects of the agent than a sheet-like two-dimensional cell population (network).

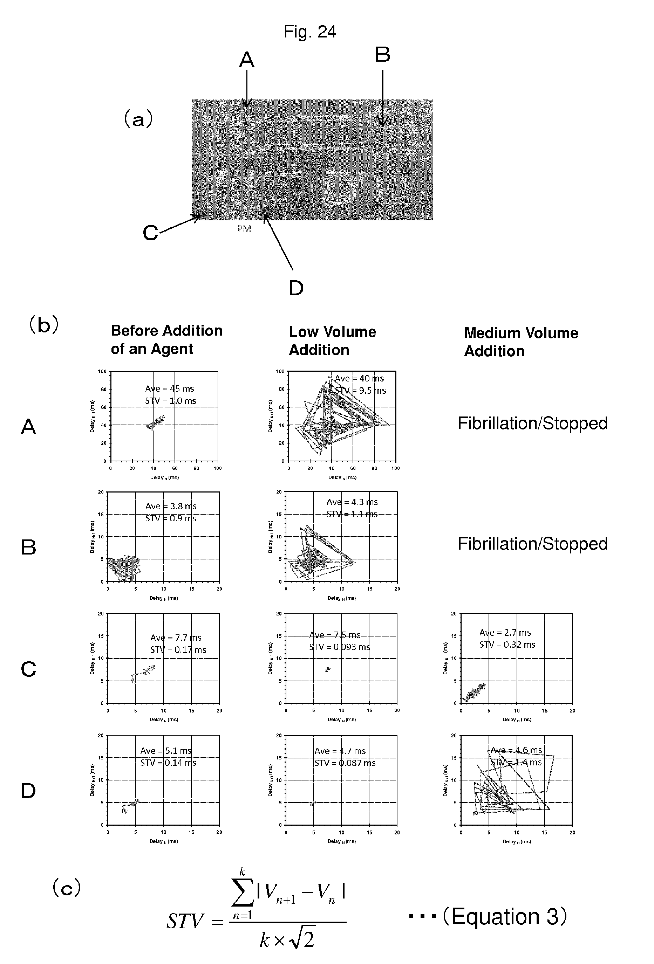

Figure 24 shows the difference in the response of the STV for the transmission speed (V) of pulsatile stimulation from the PM area in response to an addition of an agent. Torsade de Pointes (TdP), which is caused by cardiac toxicity, is a transmission defect in myocardial tissue, and represents a method for estimating the agent toxicity by checking to what extent the fluctuation of the transmission speed from the PM area is actually generated. In this case, as shown in (Equation 3) in Figure 24 (c) in relation to the definition of the STV, the transmission time T from the PM area or (an apparent transmission rate V at the observation point, which is the distance from the PM divided by this transmission time) is used for the measurements instead of the FPD. The definition of LTV is also derived from changing the FPD in terms of T or V in the same way as the STV. As an example of measurement results, a Poincare plotting of transmission time T for a local point in the case of an annularly-constituted cardiomyocyte network is shown in Figure 24 (a) (A, B); and a Poincare plotting for a local point in the case of a two-dimensionally spread myocardial sheet is shown in Figure 24 (b) (C, D). In the same manner as Figure 23, B and D in this example are also located in proximity to, and A and C are located separated from the pacemaker area PM. In Figure 24(b), for the FPDs, which were distributed on the diagonal of X = Y of a Poincare plotting prior to the addition of an agent, a large increase in the fluctuation is observed to occur for both the annular models (A, B) as well as an increase in the STV showing a great fluctuation in transmission time by the addition of a low volume of the cardiac toxic agent, while little fluctuation is observed to occur for the 2-dimensional sheet model (C, D). In response to the addition of a medium volume of the agent, the pulsation changes to a fibrillation state or a stopped state in the annular model (A, B), while an increase in the STV is observed in the area C located away from the PM in the two-dimensional sheet model (C, D). A lower rate of an increase in the STV than the area C is observed in the vicinity of the area D. As can be seen from this example, as for the prediction of cardiac toxicity of an agent by measuring the STV of the transmission time T (or an apparent transmission time at each local point), a population (network) of cells which are arranged linearly from the pacemaker area reflects more accurately the effects of the agent than a sheet-like two-dimensional cell population (network), and at the same time, it can be seen that a generation of the fluctuation, which exhibits a spatial-dependent arrangement, can be measured more effectively.

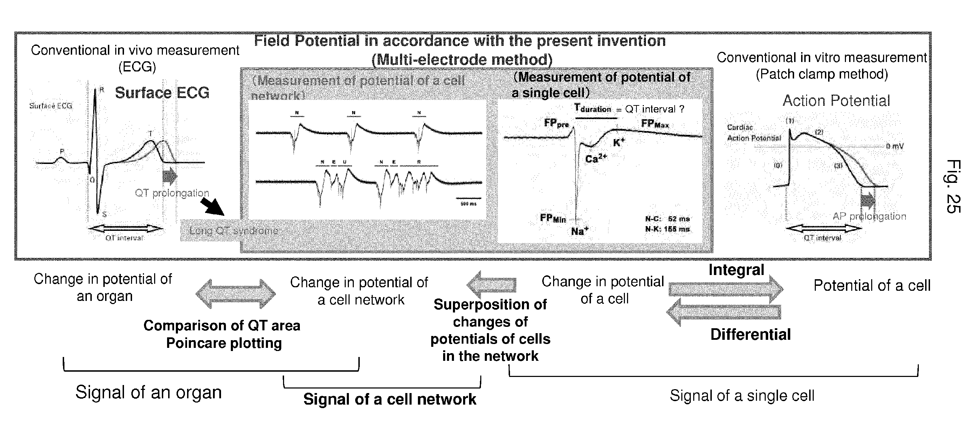

Figure 25 schematically illustrates an electrical FP waveform obtained from a cardiomyocyte in accordance with the cardiomyocyte network of the present invention in relation to a conventional *in vitro* measurement technique (e.g., patch clamp technique) and a conventional *in vivo* measurement technique (e.g., electrocardiogram). The waveform obtained by measuring the FP of the cell in accordance with the present invention indicates the magnitude of ion current per unit time into and out from the cell, which is equivalent to information on changes in the potential of the cell (which is electrically ion current), and which has the differential- and integral-relationships with the electric potential of the cell obtained from conventional *in vitro* measurements on a cell base as depicted in Figure 25. Then, a composite waveform of the FP for the cellular network can be obtained by superposing the FP waveform which is measured for each cell (or a local point of the cell network) and collected from one electrode on each of the FP waveforms collected from a plurality of electrodes that are arranged in a plurality of areas of the cellular network. This data has a homology with the electrocardiogram data of the QT area corresponding to a response of a ventricular tissue portion of the electrocardiogram which is a signal waveform of a potential change obtained from the heart.

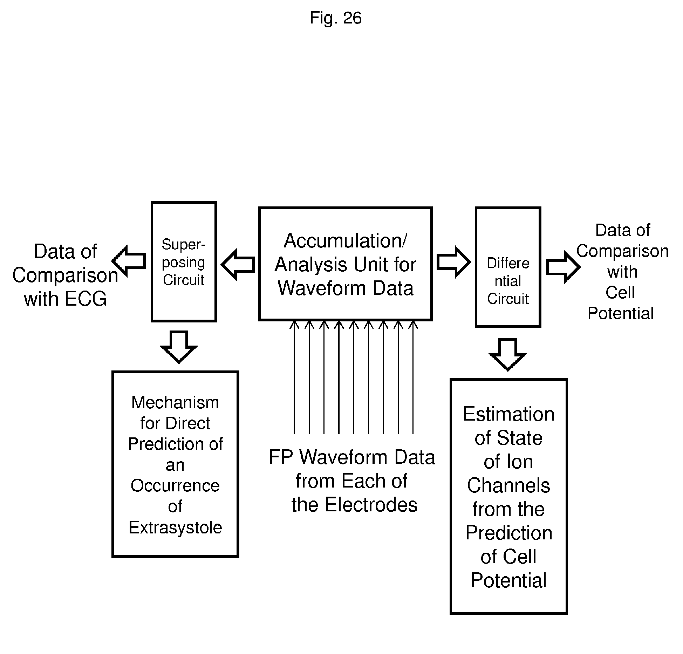

Figure 26 schematically shows a configuration of an apparatus system for estimating a correlation between information measured by the conventional technique as described in Figure 25 above and the FP data obtained with the apparatus of the present invention. The apparatus system is comprised of an arithmetic circuit that has a function to integrate the FP data obtained from each one of the plurality of microelectrodes which are arranged to be able to measure the FP of one cell or a local portion of the cellular network to estimate the membrane potential by differentiating their respective FP data; or an arithmetic circuit that is capable of comparing the data of each electrode with an electrocardiogram waveform of the ventricular portion (Q-T portion) of the electrocardiogram by superposing each electrode data. In particular, in addition to analysis of the FP data of a single electrode that is made possible by using the superposing circuit, it is also possible to makes predictions similar to electrocardiogram analysis using the results obtained by, for example, composing data of an array of a plurality of microelectrodes which are arranged in series and equally spaced on a cellular network so that data reflecting the state of intercellular transmission as well as results of the FP of the cell on each electrode can be displayed; and in particular, by transferring the information of the results of the superposing circuit directly to the prediction mechanism after occurrence of extrasystole for estimating arrhythmia which is an abnormal transmission between cardiomyocytes. This is due to the fact that an abnormality in the transmission is reflected in the waveform of the FP. On the other hand, data of a membrane potential obtained from the differentiation circuit is used to assess the state of ion channels which have different activated states in a membrane potential dependent manner.

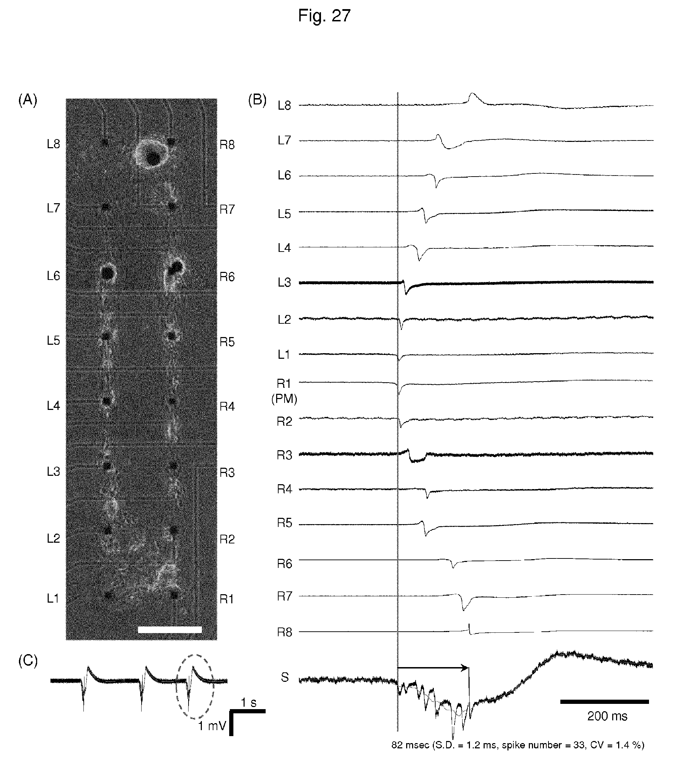

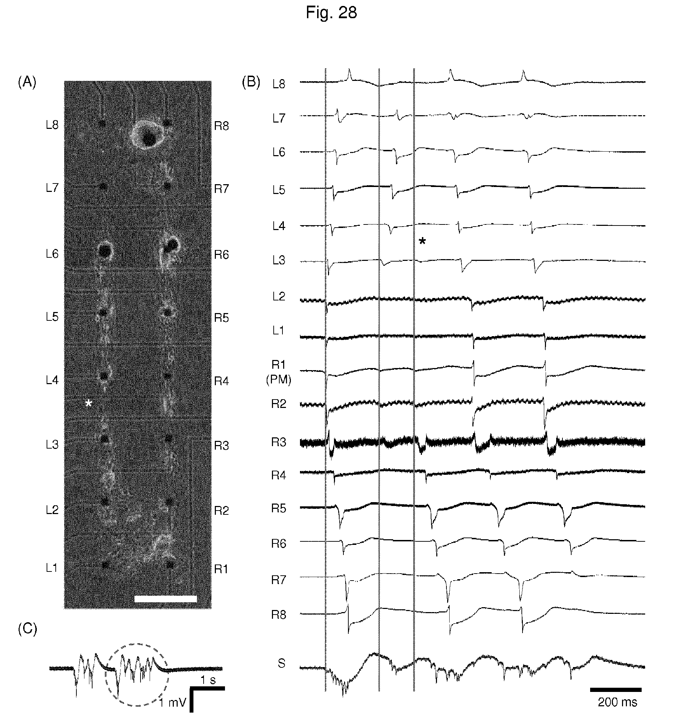

Figure 27 and Figure 28 show an example in which the FP data from each electrode are actually superimposed by an arithmetic circuit as described in Figure 26. In Figure 27, as shown in Figure 27(A), the cardiomyocyte network is annularly arranged; microelectrodes are arranged at regular intervals along the network. In the annular cardiomyocyte network in which the pacemaker (PM) area is located at electrode R1, it can be seen from the FP waveform of each electrode shown in Figure 27(B) that the pulsation signal is transmitted from R2 → R8 or L1 → L8. The S waveform at the bottom is the superimposed waveform. Figure 27 (C) shows the result of a long-term measured composite waveform. This is an actual composite FP waveform which includes information on the FP transmission required for estimating the waveform for the QT area in the electrocardiogram. As can be seen from this figure, when the pulsation signal is transmitted from the area PM in a normal way, the composite waveform is a smooth waveform as can be seen from Figure 27(C). On the other hand, in an arrhythmia state where the pulsation signal from the PM area is no longer transmitted in a normal way, the S, a composite FP, becomes a very disturbed-waveform as can be seen from Figure 28(B). Also in Figure 28(C), which corresponds to the electrocardiogram, the composite FP waveform is a waveform with a similar shape to the waveform for arrhythmia. It should be particularly noted here that when arrhythmia is predicted based only on one electrode data of each microelectrode of Figure 28 (B), it is difficult to predict the occurrence of a significant arrhythmia in some observed electrodes (L5, for example). However, a more accurate prediction is possible when a composite FP waveform is used as can be seen in Figure 28(B) S or Figure 28(C).

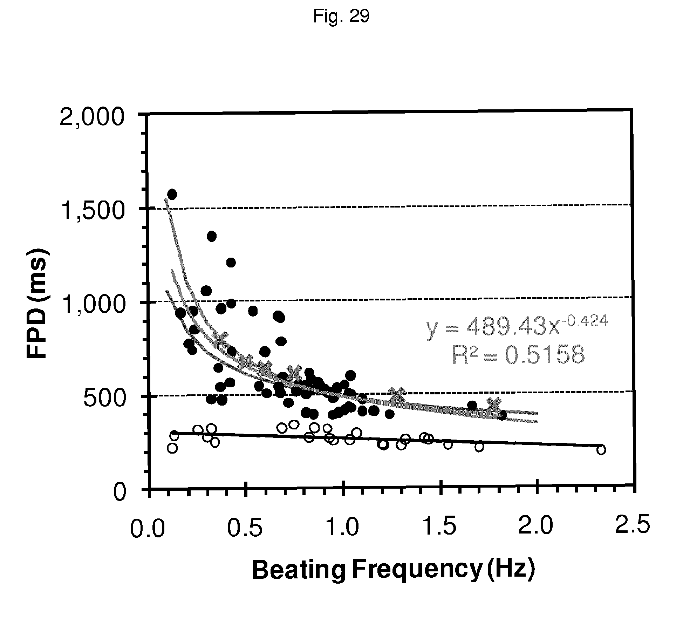

Figure 29 is a graph showing the size of the FPD in relation to the pulsation period of cardiomyocytes. The result of the measurements of the FPD for cardiomyocytes with various autonomous pulsations by the apparatus system of the present invention is indicated in black circles. As can be seen from this result, it can be seen that the cells varied their pulsation period depending on the value of the FPD. This suggests that when the measurement is performed with cardiomyocytes with various autonomous pulsations, side effects such as stopping or destabilization of the pulsation period by an agent raise the possibility that the FPD changes are due to a cause other than natural blocking of ion channels.

In addition, the red x mark denotes the value of the FPD when the pulsation period of the cell was forced to change by forced pulsation. It can be seen that the FPD becomes stable by maintaining a certain pulsation period over a certain period of time by continuous external stimulation.

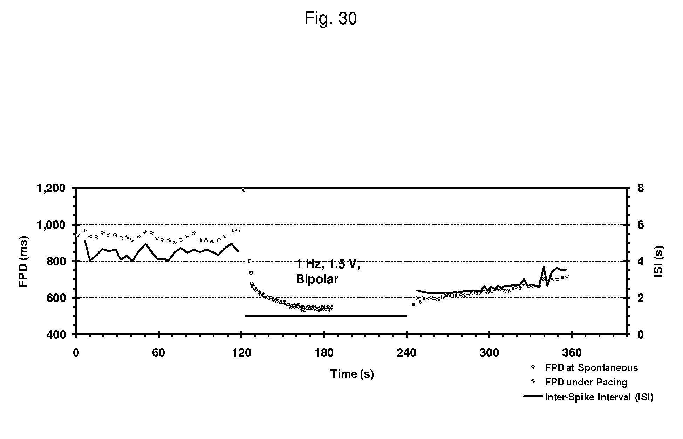

Figure 30 shows an actual example of the change over time of the FPD of cardiomyocytes when external forced pulsatile stimulation is given using the system of the present invention to cardiomyocytes which are autonomously pulsating. It can be seen in this example that initially the autonomous pulsation interval is about 4 seconds, then the value of the FPD significantly changes immediately after the cell was given a forced pulsation stimulus of 1Hz, then the FPD is stabilized at the position of 550ms at approximately 30 seconds after the start of stimulation. It can also be seen that even after the forced pulsatile stimulation, the autonomous pulsation period varies, and the FPD steadily increases. As can be seen from these results, it is desirable to test the agent toxicity after 30 seconds from the start of forced pulsatile stimulation where the FPD is stable.

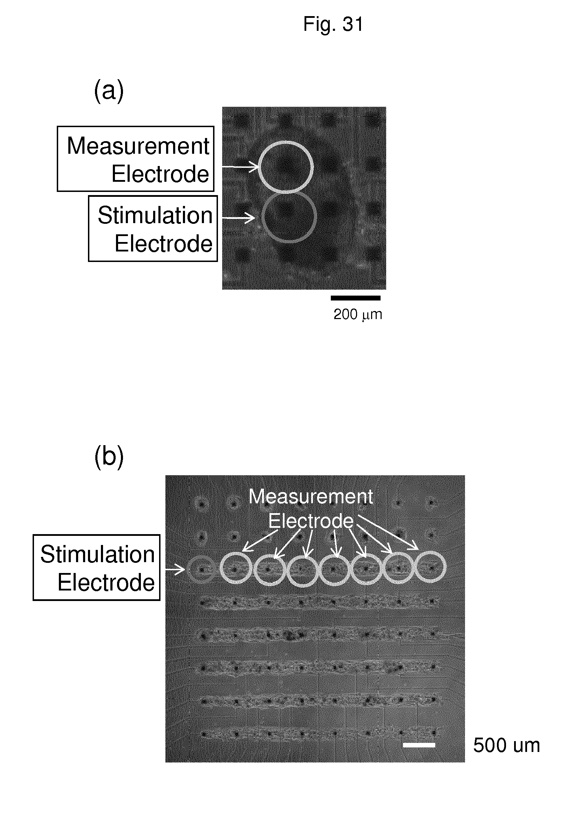

Figure 31 shows an actual example of the arrangement of cells when measuring the FPD or transmission time T or transmission velocity V while giving external forced pulsatile stimulation using the system of the present invention. Figure 31 (a) is an example of measurement of stimulation with cell populations that are disposed to cover at least two microelectrodes. While providing forced stimulation signals at a fixed interval of 60 beats per minute from the stimulating electrode, for example, the FPD of the cells at the adjacent measurement electrode, or the transmission time T or the transmission speed V from the time of stimulation at the stimulating electrode to the cells on the measuring electrode are measured. Figure 31 (b) shows an example in which forced pulsatile stimulation is given by a stimulation microelectrode disposed at the end point of the network of cardiomyocytes which are arranged in a straight line, the FPD, T and V of cardiomyocytes on each electrode are measured for the transmission by a microelectrode array disposed along the network of the cardiomyocytes at regular intervals, and for example, prediction of the occurrence of arrhythmia by a composite FP of the FPs of each recording electrode, the relationship between T and V of each electrode as well as the data of each of the electrodes to the stimulation signals of the stimulating electrode can be estimated. However, what is shown here is only an example of the arrangement of the cells. It is also possible to make similar measurements by providing forced pulsations in the PM area of the annular cellular network shown in Figure 27, or alternatively, it is also possible to make measurements of the FPD on the minimum number of cells using the stimulating electrode, on which the cells are placed, as a measurement electrode.

For all examples so far, the cardiomyocyte network is described only for cardiomyocytes. However, it is intended to include embodiments where fibroblasts are added to have properties similar to biological tissues.

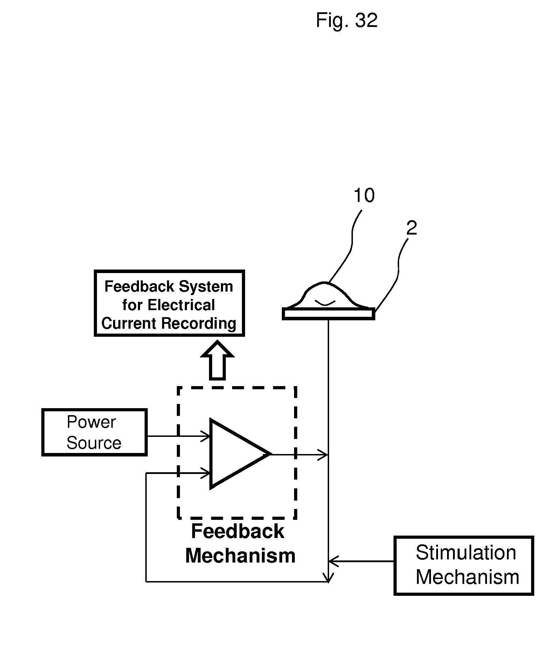

Figure 32 shows a potential clamp-type feedback control mechanism to maintain a constant voltage of microelectrode 2 and make measurements for the FP of cells disposed on the microelectrode 2. Here, the FP of cells is estimated by analyzing the result in real time by monitoring the current supplied from the external power supply to maintain the potential of the electrode 2 instead of measuring the amplified signal from the electrodes in the conventional configuration. It shows an example of the change over time of the FPD of the cardiomyocytes. As the potential is to be kept constant, in this context, it is normally chosen to take a value of zero, however, in the case that the state of cells is changed, for example, by changing the potential of depolarization, it is also possible to adjust to those different potentials.

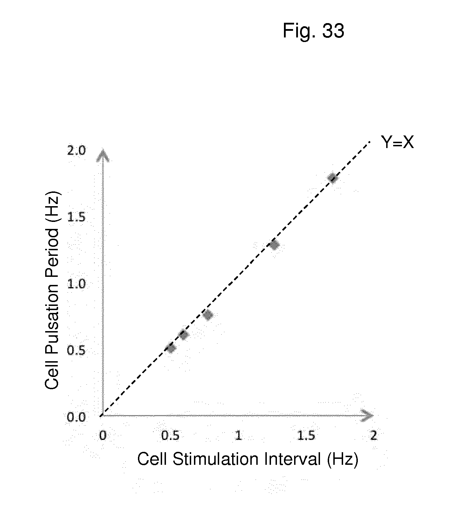

Figure 33 is a graph of an example of the results of actually measuring the change in the period of pulsation of the cell population when forced pulsatile stimulation is provided using the system of the present invention described above in a partial area of the cell population which has differentiated from human ES cells into cardiomyocytes. As can be seen from this graph, it is found that for the normal population of cardiomyocytes, when the forced stimulation of 0.6Hz to 1.8Hz e.g., is given as in this example, the pulsation follows linearly in response to the forced stimulation in all of this range.

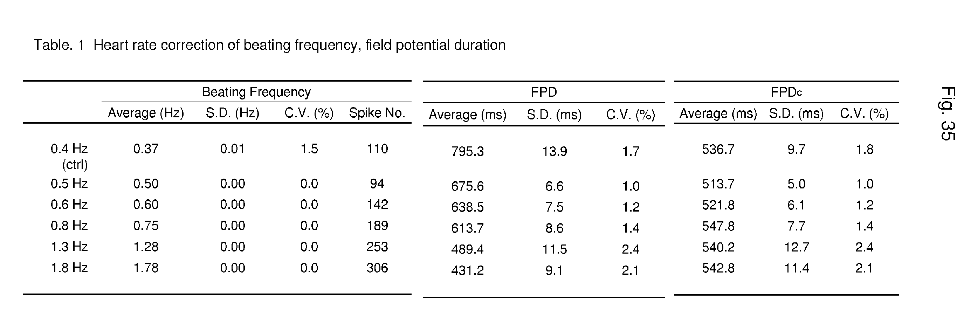

Figure 34 (a) shows changes in the waveform of the FP and in the length of the FPD of the cardiomyocyte population under forced pulsatile stimulation where the pulsation period of the cell population is the same as the interval of the forced pulsatile stimulation when forced pulsatile stimulation is actually provided. As can be seen from the graph, the FP waveform changes and the length of the FPD shortens by shortening the interval of the forced pulsatile stimulation. As shown in Figure 34(b), a graph of the change of the FPD indicates that this shortening depends on the cycle of the forced pulsation interval (RR). According to a known study of the relationship between a heart rate and the length of the QT interval in a human heart [Patrick Davey, How to correct the QT interval for the effects of heart rate in clinical studies. Journal of Pharmacological and Toxicological Methods 48 (2002) 3- 9], a Fredericia correction with respect to this compensation relationship, i.e., in order to make a correction to the length of QT ($QT_c$) during cardiopulsation at a pulsation period of 60 beats per minute, mainly depends on whether or not it conforms with the converted value of $QT_c = QT/(RR)^{1/3}$, or with the converted value of $QT_c = QT/(RR)^{1/2}$ which has been proposed by Bazett due to the fact that it is not possible to make a relative comparison because of change in the length of the QT due to the difference in the pulsation rate. As described above, the length of QT *in vivo* corresponds to the superposition of the length of the FPD measured across the cellular network measured by the present system. That is, it suggests that the FPD itself of each cell should enter into the range of the correction of Fredericia or Bazett. In fact, however, the result of Figure 34(b) indicates that $QT_c = QT/(RR)^{1/2.5}$, showing that it lies between the correction of Fredericia and the correction of Bazett. In addition, Figure 35 is a table summarizing the data shown in the graphs of Figure 33 and Figure 34.

These results indicate that evaluation of the quality of cardiomyocytes to be actually used for screening or in regenerative medicine can be addressed by measuring the response of the cardiomyocytes when forced pulsatile stimulation is given to the cardiomyocytes. In other words, the following procedures are noted:

1) providing forced pulsatile stimulation to a cardiomyocyte or a cardiomyocyte population; evaluating as to whether the cell or the population of the cells respond to the forced pulsatile stimulation and respond at the same interval as the forced pulsatile stimulation; verifying what frequency range of the response of the cells to the forced pulsation signal; and determining that one of the sufficient conditions for a healthy cardiomyocyte is met when it is demonstrated that the pulsation follows the stimulation; more specifically, determining that one of the sufficient conditions for a healthy cardiomyocyte is met when it is demonstrated that the pulsation follows the stimulation up to at least 1.8Hz, for example.
2) determining that one of the sufficient conditions for a healthy cardiomyocyte is met when it is verified that the change in the FPD in response to the forced pulsatile stimulation is between $FPD/(RR)^{1/3}$ and $FPD/(RR)^{1/2}$ within a range of the frequency at which the follow-up of the pulsation of the cells in response to the forced pulsatile stimulation interval (RR) has been confirmed.

By using the above procedures, quality control of cardiomyocytes can be achieved. A healthy cardiomyocyte is a cell that is capable of making a stable pulsation. Here, the cell population that underwent differentiation induction may be used as the cell population to be evaluated, or the cardiomyocytes that underwent a differentiation induction may be dispersed for measurement and evaluation on a single cell basis, or the dispersed cardiomyocytes may be collected and used as a cell population for measurement, or alternatively, the dispersed cardiomyocytes may be mixed with fibroblasts derived from a human heart and used as a new cellular population for the measurement and the evaluation. These cardiomyocytes can be used for the cardiotoxicity test.

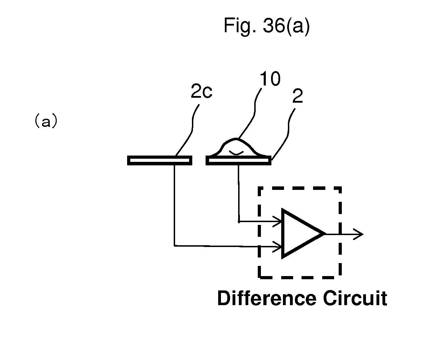

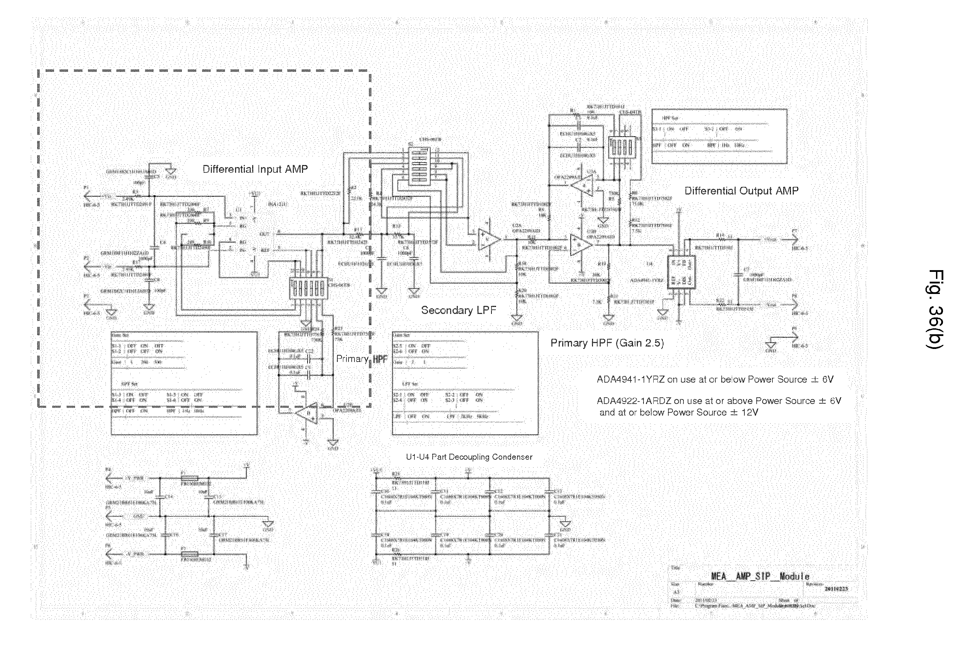

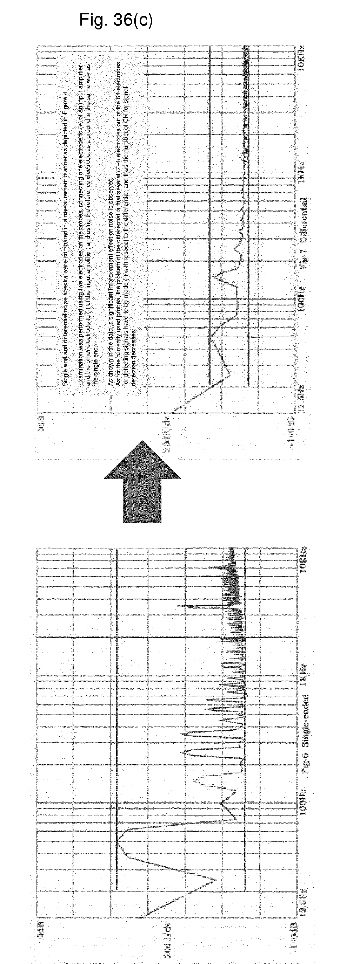

Figure 36 (a) schematically shows a circuit for outputting a value of the difference in electric potential between a microelectrode 2 on which a cell 10 is disposed and a comparison electrode 2c, which is in the vicinity of the microelectrode 2, and on which no cell is disposed, for use in electrically reducing noise in cell signals. In fact, as shown in Figure 36(b), by incorporating this circuit in the first stage of the amplifier circuit, it is found that the noise reduction does not depend on a specific frequency, as shown in Figure 36(c). The position of the reference electrode 2c is preferably in the vicinity of the microelectrode. For example, it is fully functional if it is located at a distance of 50$\mu$m, and it can function to reduce noise if it is within a distance of 1mm.

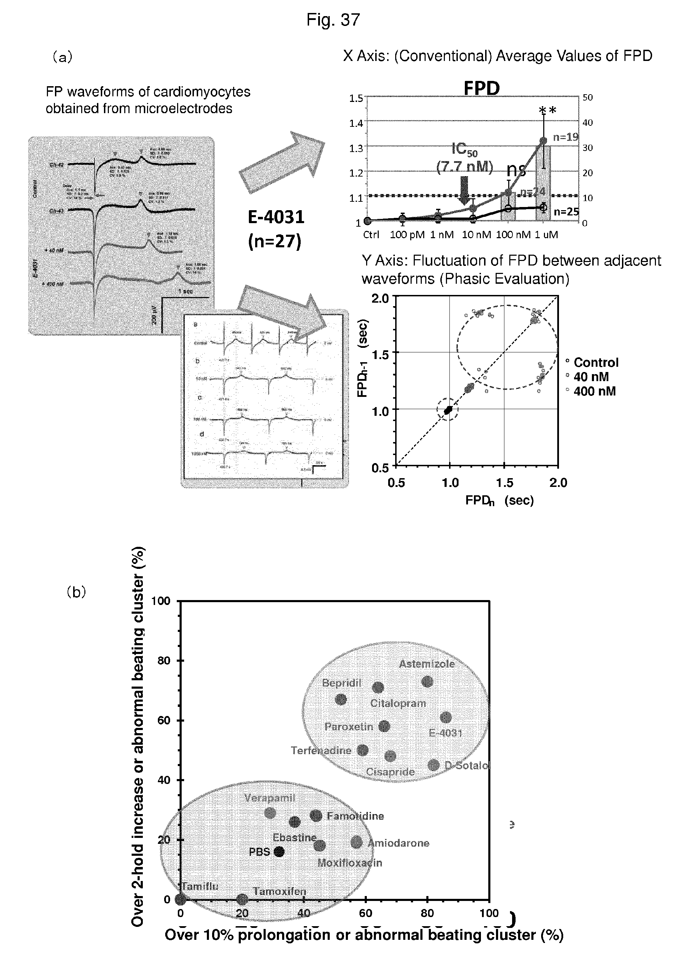

Figure 37 is a diagram schematically showing an example of a comprehensive cardiotoxicity evaluation method of the present invention. Regarding the values for the FPD obtained from the results of measurements of membrane potential of cardiomyocytes after addition of an agent of a particular concentration, the results are plotted taking a level of the FPD prolongation as a value on the X axis and taking STV, which is derived from Poincare plotting of the magnitude of the fluctuations with time of the FPD described above, as a value on the Y axis. Figure 37 (b) is one example of the results plotted in an X-Y diagram for a variety of agents. As can be seen from the figure, an agent in the area where the increase in the prolongation of the FPD and the fluctuation (STV) is decreased can be determined as having a QT prolongation but no cardiac toxicity, while cardiac toxicity such as TdP can be predicted when prolongation of the FPD and the fluctuation of (STV) occur simultaneously (upper right in the X-Y diagram).

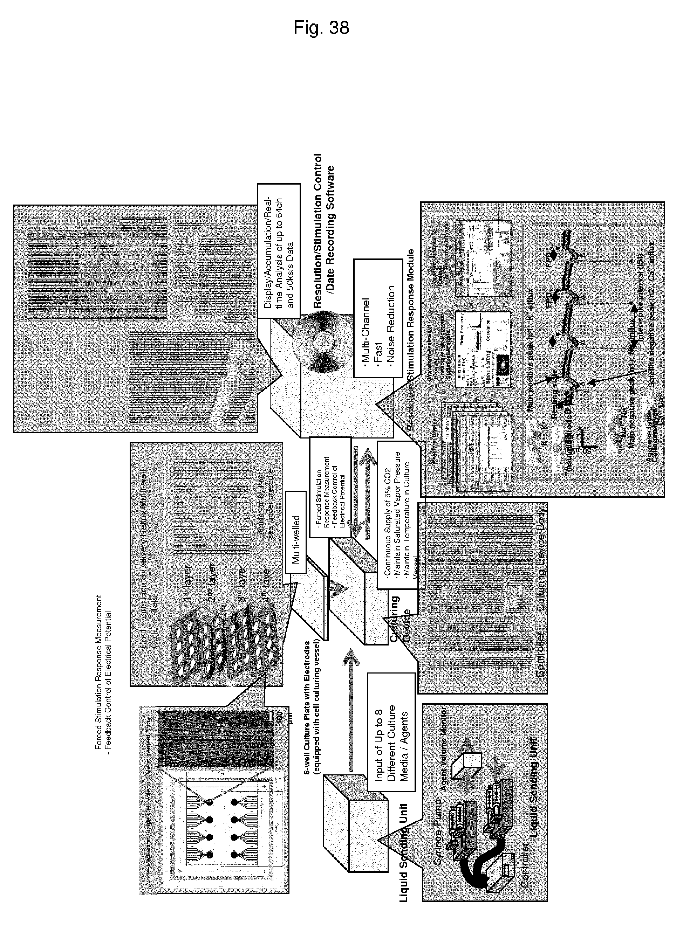

Figure 38 is a schematic diagram showing an example of the configuration of a system for measuring the actual cardiac toxicity. The system of this embodiment includes a liquid sending unit, a cell culture measurement unit, and a cell analysis/stimulation unit.

The liquid sending unit can send liquid by a syringe pump system or a peristaltic pump system or a HPLC pump system by which the culture solution is continuously fed to each of the cell culture chambers in which cells are cultured in the measurement unit. In addition, a resistive heating wire for temperature control is wound around the outer circumference of the pipe of for sending liquid, and a solution is always introduced at a constant temperature by monitoring the temperature of the liquid in the tube continuously with a detecting mechanism of heat such as a micro-thermocouple type K or a thermistor, and adjusting the temperature of the liquid to be introduced in terms of the degree of resistance heating for controlling solution temperature. In addition, the liquid sending unit includes piping in which mechanisms such as junction pipes and switching pipes are arranged for addition of agents to be tested, and through which desired concentrations of agents can be introduced into each of the cell culture chambers. Further, the quantitative determination of the concentration of the agent solution desirably includes addition of a mechanism in which a portion of an inlet pipe of the liquid is optically transparent, and by which quantitative evaluation can be made by spectrophotometric measuring in the range of a wavelength of 280nm - 800nm. Likewise, it is also desirable that a mechanism for waste liquid is added in which a part of the waste tube is optically transparent, and by which quantitative evaluation is possible by measurement of spectroscopy absorption in the range of a wavelength of 280nm to 800nm. The controlled temperature of the agent solution preferably approximates a normal temperature of a human body, and from this point of view, it is desirable to be able to control the temperature in the range of 30 degrees to 45 degrees centigrade.

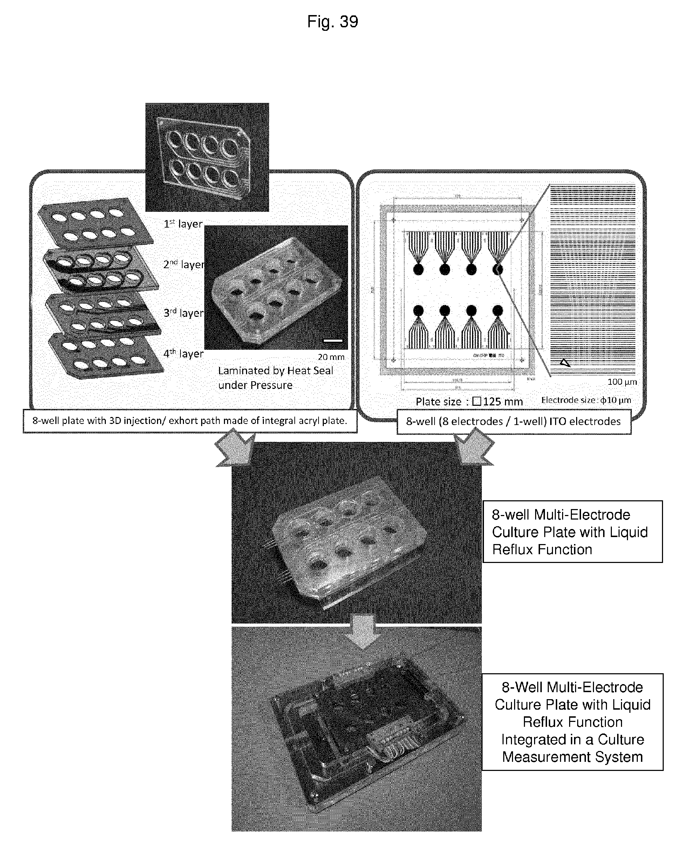

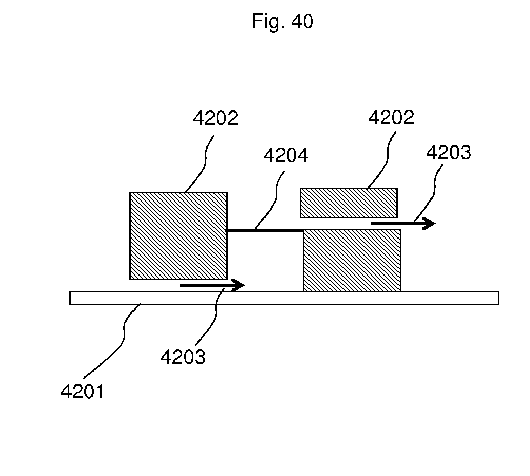

Figure 39 shows schematic diagrams and photographs of an example of a configuration of a measurement chamber of a cell culture system for measuring cardiac toxicity of the present invention. The cell culture vessel 4202 on which an introducing mechanism and a draining mechanism of the culture liquid are arranged has been adhered to the multi-electrode substrate 4201 on which a plurality of membrane potential measurement electrodes is arranged (see Figure 40), forming a cell-culture-measurement plate capable of measuring 8 samples at the same time. As shown in Figure 40 in which a cross-sectional view of a cell culture measurement plate is schematically shown, in the cell culture vessel 4202, the inlet of the solution is arranged in a fan-shaped form spread in the bottom surface closest to the multi-electrode substrate 4201, while the liquid draining mechanism has been deployed in a fan shape in the same direction as the direction of the interface of the liquid surface at a position that determines the height of the liquid surface 4204 at the top.

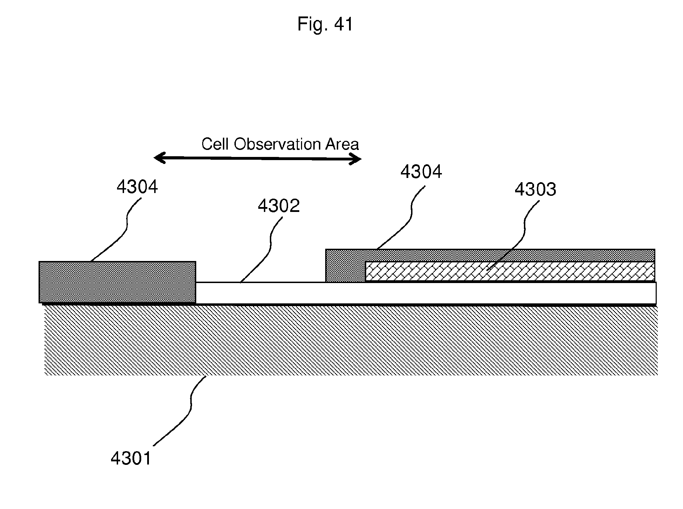

Figure 41 is a diagram schematically illustrating a configuration of electrode wires of the electrode arrangement arranged in a multi-electrode substrate. In the present invention, in order to observe the shape of the cell, transparent electrodes such as ITO electrodes are used. However, an increase in the length of the wiring will result in a high resistance compared to normal metal electrodes due to their characteristics as a transparent electrode, and as a result the impedance becomes very large especially for a large plate such as the multi-electrode substrate. In order to avoid this problem, a metal layer may be disposed in the same arrangement as the transparent electrode to reduce the resistance value owing to the conductivity of the metal electrode. In fact, in the area for culturing the cells, a wiring using a transparent electrode 4302 on a glass substrate 4301 is disposed in order to perform an optical observation, while in an area not used for observing cells, a metal layer 4303 is disposed thereon to overlap the transparent electrode and the upper surface of which is coated with an insulating film. The metal electrode materials as used herein may be, for example, gold, platinum, titanium, copper, aluminum, and the like.

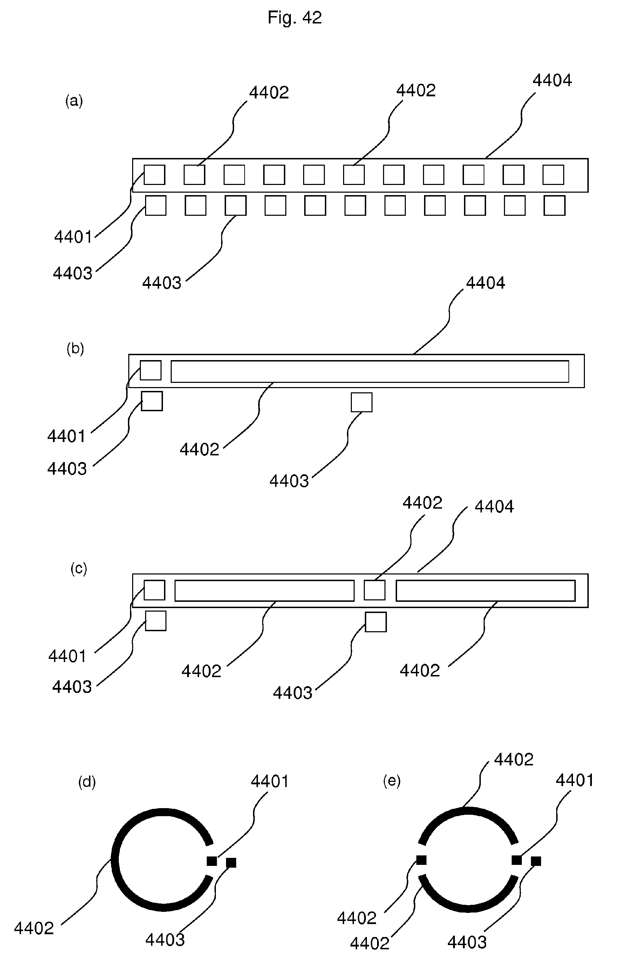

Figure 42 is a schematic diagram showing an example of electrode arrangement disposed on a multi-electrode substrate. First, in Figure 42(a), there are arranged a stimulating electrode 4401 to locally stimulate the end of the myocardial cardiomyocyte network arranged in series in a cell culture area 4404, measuring electrodes 4402 for measuring the excitation conduction of cardiomyocytes stimulated by stimulation electrodes and a reference electrode 4403 for noise reduction. It is possible to measure the results of a plurality of local responses of a cardiomyocyte network obtained from each of the measurement electrodes 4402, and a fluctuation of the transmission rate can be determined by a comparative analysis of the degree of the transmission rate between the measurement electrodes. In Figure 42 (b), there is shown a configuration where the measurement electrodes are connected in a straight line. By this configuration, it is possible to measure the waveform similar to the waveform of an electrocardiogram of the ST area (ventricular area) of the electrocardiogram. In Figure 42 (c), there is shown a configuration that facilitates acquisition of the FP waveform of a local point of cardiomyocytes by separating a part of Figure 42(b). Figure 42 (d) is an example of an electrode arrangement for measuring cells arranged in a ring form on a ring-shaped electrode. Although these are intended to measure the cardiomyocyte network arranged in a ring shape as shown in Figure 11 and Figure 12, they differ in that a part of the ring-shaped measurement electrode is cut out, and a stimulating electrode for providing local forced stimulations is arranged therein, and a reference electrode is arranged for noise reduction. Further, in Figure 42 (e), there is shown a configuration in which the measurement electrodes are split and it is possible to measure responses of a local portion of the cardiomyocytes.

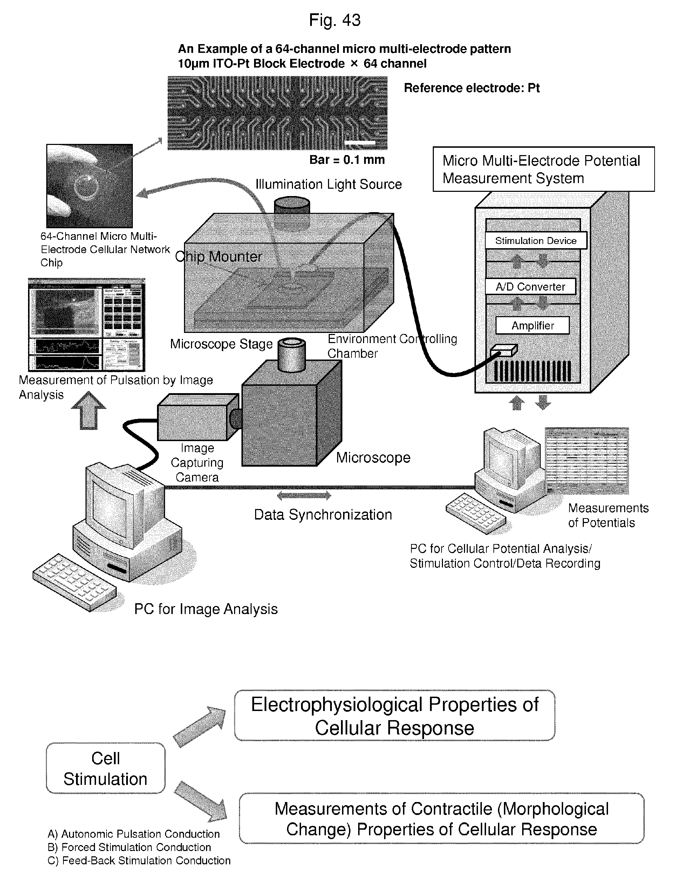

Figure 43 is a schematic diagram showing an example of a system configuration of the present invention to simultaneously measure the mechanical properties and electrical properties of cardiomyocytes. The present system includes (1) a cellular network chip that can be used for culturing a cell population, includes a plurality of micro-electrodes disposed on a substrate and can be used for acquiring cellular potential data of a small area of the population; (2) a chip mounter for mounting the chip and joining electrically with cell-stimulating and/or cellular potential measuring system; (3) an environmental control vessel which can be used to control the environment such as temperature, humidity, oxygen concentration and carbon dioxide concentration of the cell population being cultured; (4) a micro multi-electrode potential measurement system that can give stimulation to a specific cell of a cardiomyocyte population, and allows simultaneous continuous measurements of cellular potentials of various small areas in the cell population; (5) one or more position-coordinate probe microparticles configured to measure changes in shape of myocardial cells and allow easy identification of cardiomyocytes using a plastic, polymer, glass or metal microparticles such as polystyrene microparticles, glass microparticles or gold microparticles of sizes from at minimum about 0.1, $0.2\mu m$, $0.3\mu m$, $0.4\mu m$, $0.5\mu m$, $0.6\mu m$, $0.7\mu m$, or preferably about $0.8\mu m$, more preferably about $0.9\mu m$, or most preferably about $1\mu m$, to at most about $500\mu m$, $400\mu m$, $300\mu m$, preferably about $200\mu m$, more preferably about $100\mu m$ or most preferably about $50\mu m$, wherein the particles are disposed in the cell population by mixing them on the surface of the cardiomyocyte population in the cell network chip or in the cell population and culturing them; (6) an optical image capturing system for optically measuring the microparticles, wherein the system includes a light source, an optical microscope and an image-capturing camera to capture the images of the microparticles; and (7) a computer system for image analysis, cellular potential analysis, stimulus control and/or integrated data acquisition, wherein the system is capable of measurements of cellular potential and analysis of waveforms of the potential, measurements of cell displacements by image analysis and feedback stimulation based on the analysis results. In the measurements using the device system, as for the stimulus to cause depolarization of the myocardial cells, the stimulus can be provided by selecting mainly from three means, i.e., (A) measurements using conduction of autonomous pulsation of the cardiomyocyte population, (B) measurements of conduction by providing a forced electrical stimulation from outside to a specific cell in the cardiomyocyte population and (C) measurements of conduction by providing a feed-back stimulus at a specific timing to meet the relationship of the delay time and the value of the cellular potential based on the cell voltage measured data to the specific cells in the cardiomyocyte population.

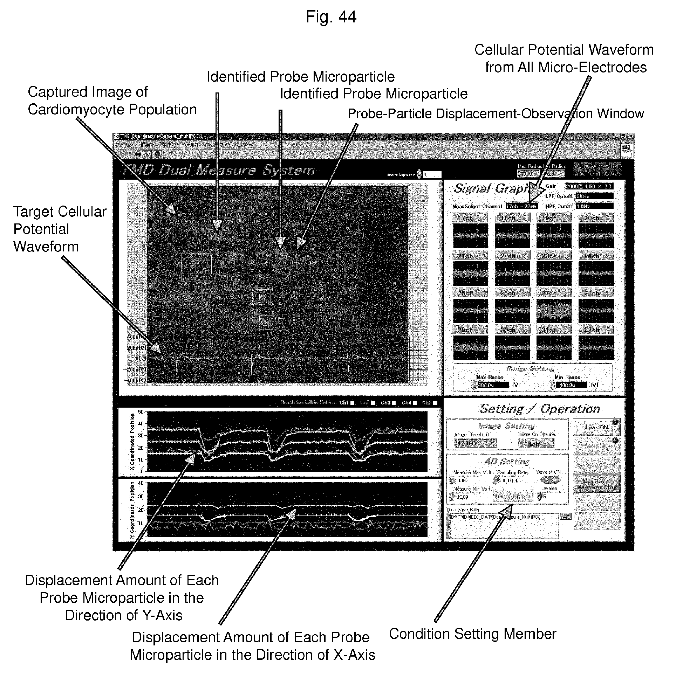

Figure 44 is an example of a data acquisition monitor screen showing an example of data obtained from an example of a system configuration of the present invention to simultaneously measure the mechanical properties and the electrical properties of cardiomyocytes. In a setting of this example, a large number of polystyrene microparticles are placed on the surface of the cardiomyocyte population, a probe-particle displacement-observation window for five of the polystyrene microparticles selected as probes is set, and the center of gravity of the window moves with the movement of the microparticles in the window, thereby displacement of the particular probe microparticles can be measured continuously as a change in vector time in the directions of the X-axis and Y-axis. Further, by measuring the cellular potential data of a particular target cardiomyocyte at a position being at the optical measurement, any change in conduction stimuli response of Na ion channels, Ca ion channels and K ion channels can be measured in correlation with changes in cell shape. In particular, the capability of simultaneous measurements of displacements of a plurality of probe microparticles and measurements of changes in the displacement direction in addition to the change in the displacement amount makes it possible to estimate quantitatively whether the changes in the contractile strength in response to addition of a drug, which occurs due to the variation of the response characteristics of the myocardial cells in the cell population, occur uniformly or non-uniformly.

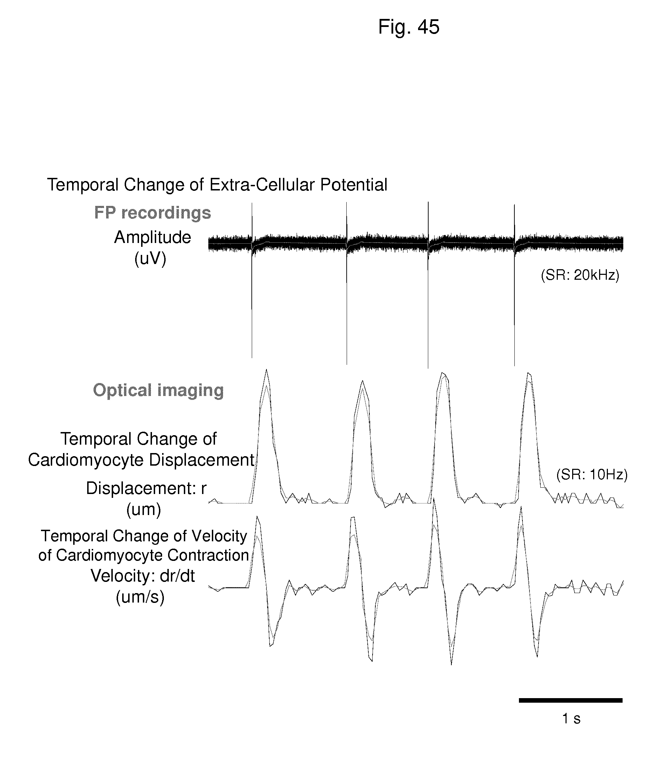

Figure 45 is an example of data obtained from an example of a system configuration of the present invention to simultaneously measure the mechanical properties and electrical properties of cardiomyocytes. As can be seen from the figure, in addition to the cellular potential data, at the same time, the amount of displacement of the cardiomyocytes and a displacement velocity data obtained by time differentiating the amount of displacement can be obtained.

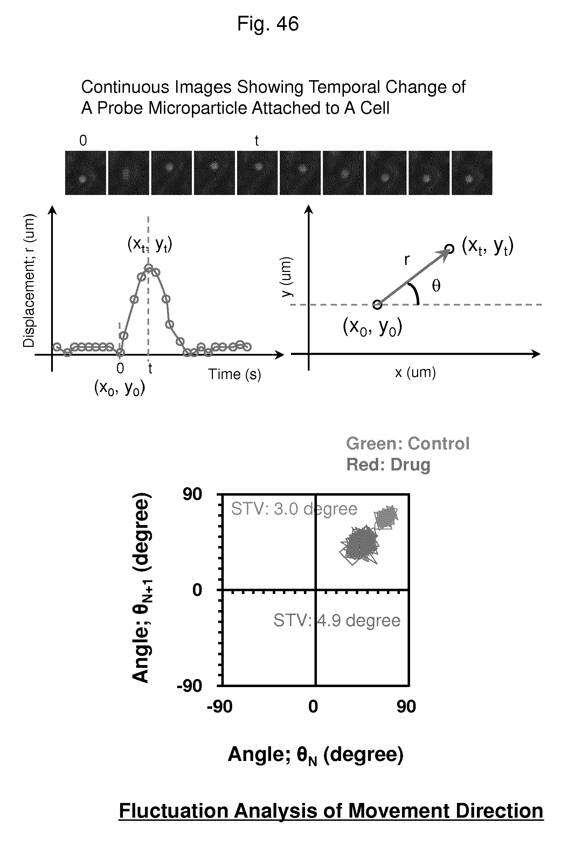

Figure 46 is a diagram illustrating an example of acquisition of data of directions of cell displacements obtained from an example of a system configuration of the present invention to simultaneously measure the mechanical properties and electrical properties of cardiomyocytes. In the upper part of the figure for continuous images which show time variation of probe microparticles attached to the cells, the displacement data is obtained as components of (X, Y); and by converting the components to a polar coordinate system $(r, \theta)$ comprised of displacement length r and angular change $\theta$, it is possible to estimate quantitatively the effect of a drug using two parameters of the displacement amount and the angular change. The lower part of the graph illustrates an example of a phenomenon observed when the fluctuations of the angle change of the displacement of the microparticles are in fact increased by the addition of the drug.

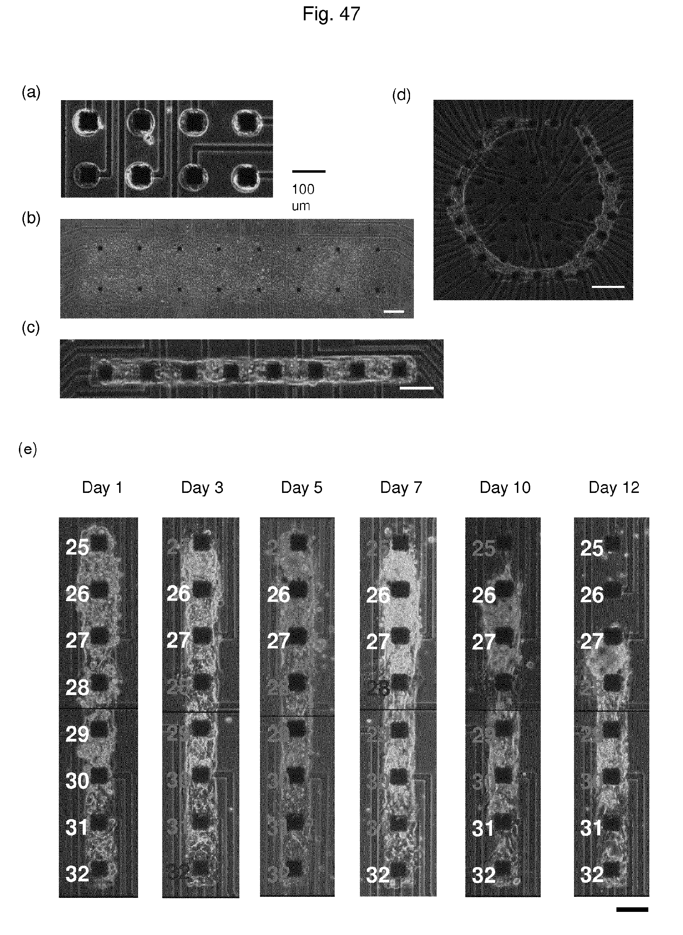

Figure 47 is a diagram illustrating an example of a spatial arrangement of myocardial cells in the network system of the present invention to simultaneously measure mechanical properties and electrical properties of the myocardial cells: (a) one micro-cluster of myocardial cells is arranged on one microelectrode; (b) cells are arranged in a myocardial cell-sheet spread two-dimensionally with respect to the two-dimensionally arranged microelectrode arrays; (c) the myocardial cells are arranged in a straight line on a one-dimensionally or linearly arranged microelectrode array, in which firing of myocardial cells at one end point is conducted to the other end; and (d) a myocardial-cell network is placed in a ring-like fashion on a ring-shaped microelectrode array, in which the ring-like arranged cardiomyocyte network can be arranged as a closed loop, or a part of the ring-like arranged cardiomyocyte network can be cut out to form an open loop. Here, when the cells are placed in a straight line as shown in (c) in particular, if adhesion of the cardiomyocytes to the substrate surface is not sufficient, then as shown in (e), the cells shrink gradually and become unable to maintain the spatial arrangement of cellular network to form a cell mass because the cell-to-cell contractile force of the cardiomyocytes is too strong as compared with their adhesion to the substrate. In order to avoid this result, it is effective to release contractile force by arranging the cells in a ring-shape fashion as shown in (d). Further, it is also effective to use a collagen vitrigel in place of conventional collagen for the collagen layer on the substrate surface.

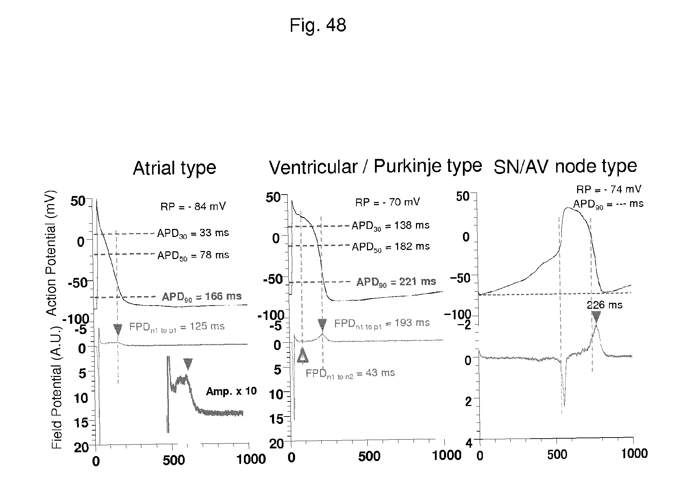

Figure 48 shows graphs of cellular potential (Action Potential) of human stem cell-derived cardiomyocytes (top three) and graphs of extracellular potential (Field Potential) obtained by temporal differentiation of the cellular potential (bottom three) as classified based on the cellular potential measurements of myocardial cells, which is the measurements well known in practice. Atrial muscle type cells are shown on the right, ventricular muscle type cells (Purkinje cells) are shown in the middle and atrioventricular node type cells are shown on the left of the graph. Therefore, when performing measurements of drug response of ventricular muscle or conduction response of the ventricular muscle, it is desirable that the measurements are performed using cells that have the feature as shown in the lower middle graph as measured in extracellular potential. This feature is characterized by generation of sharp inward current of Na ions within 20ms after the start of depolarization associated with a clear sudden depolarization, subsequent generation of gradual inward current of Ca ions within 100ms after the start of depolarization, and generation of prominent outward current of K ions observed on or after 100ms after the start of depolarization, in the absence of addition of a drug.

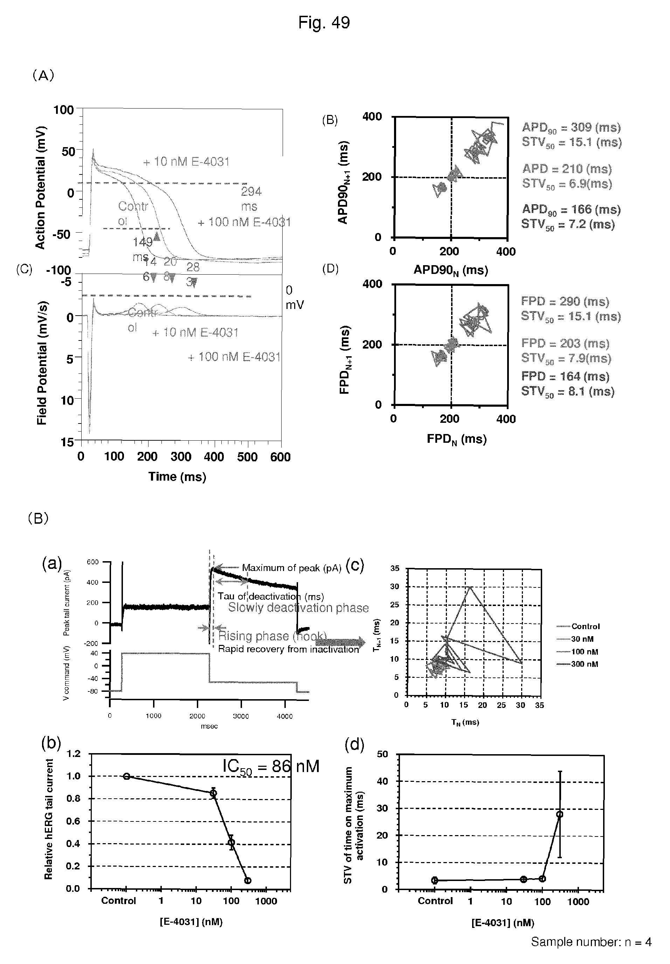

Figure 49 is a diagram showing cellular potential of cardiomyocytes, that is an example of the fluctuation changes in drug response of hERG ion channel. As shown in FIG 49(A), with an addition of an agent E4031 which specifically inhibits the hERG ion channel, a response of extracellular potential (Field Potential: FP), as already mentioned, generates a large fluctuation between adjacent pulsation cycles (the response stability is lost). In the same way, it is also found that cellular potential (Action Potential: AP) of human stem cell-derived cardiomyocytes also generates a fluctuation of a similar trend and scale to that of the FP. Therefore, even with a conventional electrophysiological cellular potential measuring method such as the patch clamp method, it is also possible to measure and estimate quantitatively the degree of fluctuation of the data obtained, rather than taking the average value of the response(s) observed. Further, in order to adjust the stability of the responses of the cells, even when measuring by a patch clamp method, for example, rather than performing the measurement with an isolated single cardiomyocyte, it is desirable to perform the measurement with a single cell which is within a cardiomyocyte population. Figure 49(B) also illustrates an example of the results of measured changes in cellular potential of whole cells in response to the inhibition by E4031 for CHO cells forcibly expressing only hERG ion channel. As can be seen from this result, in a conventional tail current measurement, an average of measured data is obtained, but the measurement becomes difficult as the current itself is reduced with a progress of blocking of ion channels. However, when a fluctuation measurement of cellular responses is performed, an increase in the fluctuation increases abruptly when the blocking probability of the hERG ion channel increases. Therefore, it is effective to use a combination of the two measurement techniques, in which tail current measurements are performed when the current amount is large, and measurements of fluctuation are performed when the current-based measurements are difficult due to a large amount of blocking.

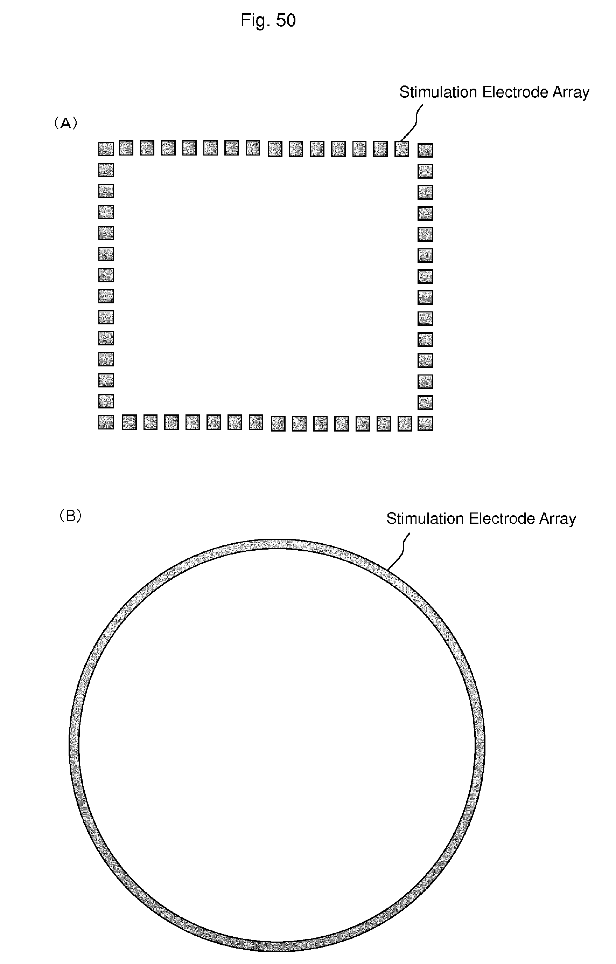

Figure 50 is a diagram illustrating the principle of cell stimulation at any position by superposition of stimulation potentials from a stimulating electrode array. As shown in FIG 50(A), a large number of stimulation electrode arrays, in which the electrode potential and the phase between the adjacent electrodes can be controlled, are arranged two-dimensionally on a substrate. From each electrode, a weak potential change, which does not cause to depolarize cells with the single electrode, is generated and superimposed. In this way, the superimposed potential sufficient to provide a cellular stimulation is specifically generated at a certain position in a two-dimensional surface. For this purpose, the field strength and a phase pattern of an electrode that need to be generated by each electrode are calculated based on the rules of Fourier synthesis, and thereby it is possible to stimulate only a specific location. As an example, as shown in Figure 50(B), for example, the electrode array is arranged in a circular ring shape, and is controlled by the method of the above, and stimulation by a focused electric field may be provided at the center of the ring. When this electrode array is arranged as if it is floating in space in the Z-axis direction from the (R-axis direction) XY plane in a cell culture layer, the arrangement of the stimulation electrode array for electric field focusing in a plane on which an electrode array is arranged (R-axis direction) and in a plane of the electric field irradiation direction (Z-axis direction) is specifically as follows. First, when an electric field is focused at a point $Z_f$ on the Z axis, the distance to $Z_f$ from $R_0$ is defined as $m\lambda$ where the point at which a perpendicular line to the R-axis plane from the focal point $Z_f$ is $R_0$. Here it is assumed that $\lambda$ is the wavelength of the stimulation signal wave based on the conduction velocity in the cell population to be generated from the stimulation electrodes, and m is a natural number. If the stimulating electrodes are placed at the position of $R_0$, the radius of the micro-electrodes arranged concentrically is expressed as follows:

$$R_n = \sqrt{2m \cdot n + n^2} \cdot \lambda$$

Here, $R_n$ is the radius of the location of the n-th phase from the center. The position $R_n$ represents the concentric location of the n-th stimulation electrode and the width of the radius is at most $\pm \lambda/4$ approximately in terms of conduction velocity and in terms of the distance from the position of the focal point for stimulation. Of course, it is possible to specifically stimulate the focal point of the concentric circle even if the stimulation electrode is not arranged as a reference at the position of $R_0$. Also, as for $Z_f$, a stimulation electrode array may be arranged on the same substrate as the substrate on which a cell network is located so as to have $Z_f = 0$. Further, when the focal position is displaced from the center of the circle, it is possible to provide stimulation to any particular position within stimulating ring electrodes by converting the phase of the stimulation signal of each stimulation electrode from the conduction velocity in accordance with the displacement. In the above description, although a cardiomyocyte network is described as an example, as long as cells have transmission capability of excitation conduction between cells, the same procedure is applicable.

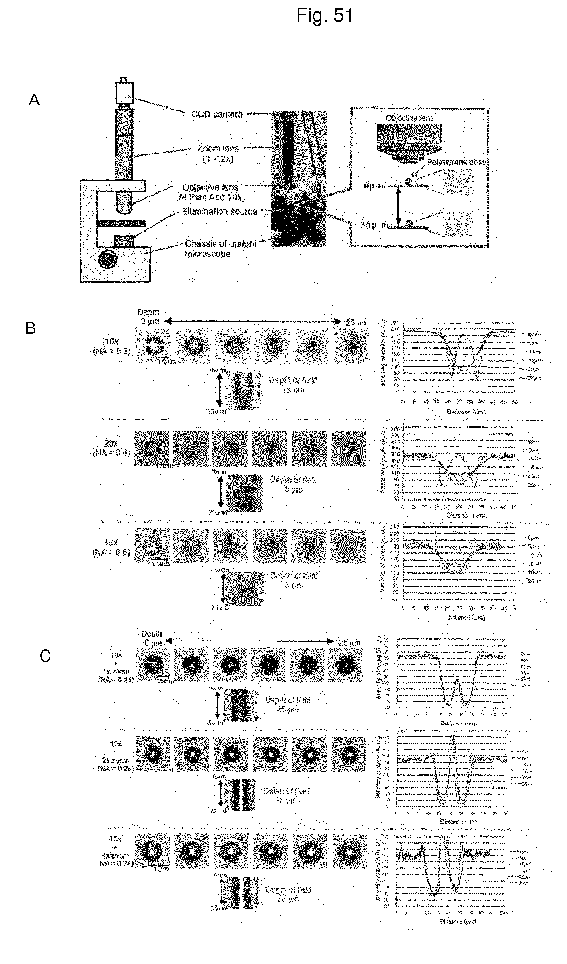

Figure 51 illustrates effects of a combination of a zoom lens system and an objective lens having a numerical aperture less than 0.3 for optical measurements of microparticles. In an ordinary optical system, an image from the objective lens is directly formed on an image-recording device such as a CCD camera. In such cases, the depth of focus corresponds to the number of aperture (NA) of the objective lens, and when the magnification is magnified, there is a problem that the depth of focus at which blur of the image does not occur becomes shallow. In order to solve this problem, the image obtained by the objective lens of low magnification (i.e., a low numerical aperture) may be magnified by a zoom lens system which is added downstream. The spatial resolution of the image is defined by the numerical aperture. In the present invention, because probe particles whose shape is already known are used, as long as the image of the microparticles does not blur at the expense of some spatial resolution, the exact space coordinates of the microparticles can be obtained, and thus there is no problem. Figure 51A illustrates an example of a configuration of an optical system of the present invention. A zoom optical system is arranged downstream of an objective lens, and a video camera is arranged downstream of the zoom optical system. Figure 51B is a result from direct observation of microparticles using objective lenses with different magnifications (numerical apertures) and observation of image blur in a depth direction. As can be seen from the result, an image of a focal depth of up to 15μm can be observed without blur with a x10 objective lens (numerical aperture 0.3). However, for either with a x20 (numerical aperture 0.4) or a x40 (numerical aperture 0.6), it is only possible to obtain an image without blur for up to the depth direction of about 5μm. Figure 51C is a result of observation of an image of an optical system in which a zoom system is arranged in practice in addition to a ×10 objective lens (numerical aperture 0.28). As can be seen from this result, even when the magnification is magnified to a level of magnification equal to those with a x20 objective lens or a x40 objective lens (positional coordinate resolution) using a zoom system, the depth of focus of about 25μm is maintained, making it possible to track the probe microparticle without losing its coordinates and with resolution of positional coordinates by using a similar image processing even for large displacement in the thickness direction, especially for a cardiomyocyte network which engages in contractile motion.

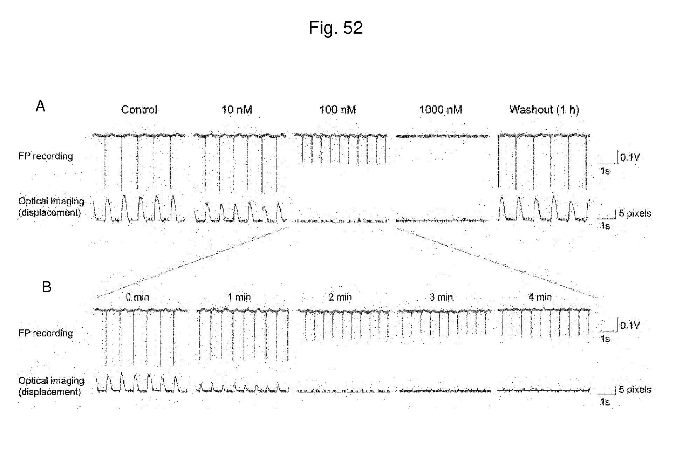

Figure 52 shows an example in which cellular potential measurement and mechanical measurement were simultaneously performed using the system described above. Figure 52A shows a result of simultaneous measurement of the extracellular potential (FP) and change in the developed tension (optical imaging) when verapamil is added as an example of an agent that disperses the contractile tension of the cardiomyocytes. The time variation of the loss of contractile force at a drug concentration of 100nM is shown in Figure 52B. As can be seen from Figure 52B, the electrical firing of excitatory conduction of cells is maintained, contractile forces disappear rapidly, and although an electrophysiological excitation conduction continues eventually, it can be seen that the mechanical contraction force disappears. Further, as can be seen from the data for 1000nM in Figure 52A, in this case, it can be seen that electrophysiological excitation does not occur and mechanical contraction does not occur. As can be seen from this example, use of only electrophysiological measurement causes difficulty in accurately predicting at what point the mechanical contraction is actually lost while the electrical excitation is maintained. Further, with only the mechanical measurement, it is difficult to identify whether the contraction force is lost at the time the mechanical contraction is lost while the electrical excitation is maintained, or the electrical excitation itself is lost and the contraction is lost. However, as shown in FIG 52, it is possible to quantitatively evaluate how the contractile force is lost while there are still electrical excitation stimuli, if both the electrophysiological measurement and the mechanical measurement can be simultaneously measured.

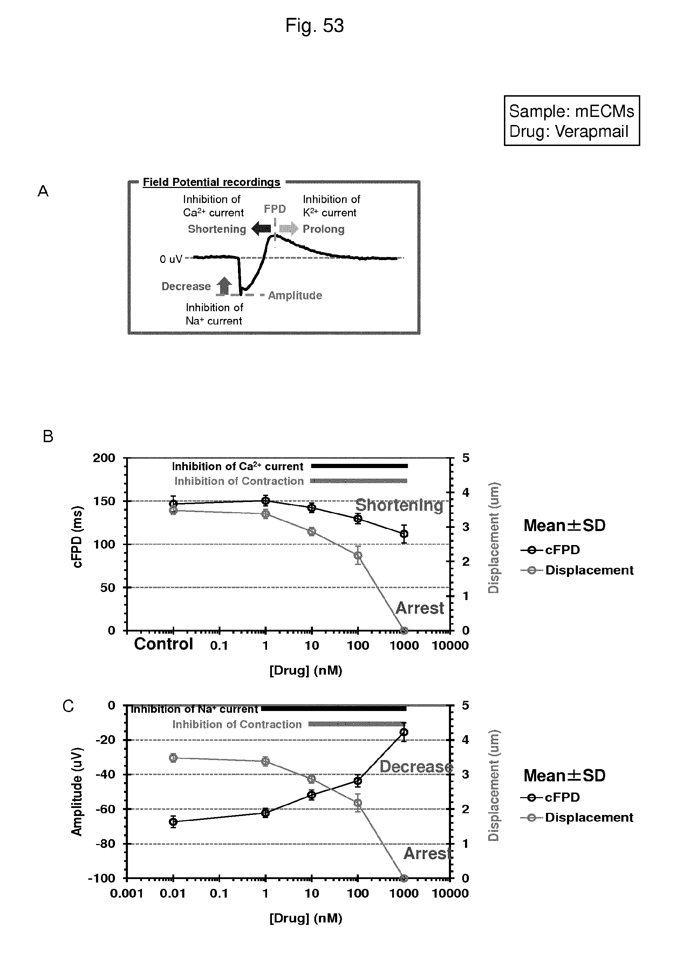

Figure 53 is graphs summarizing the results obtained in Figure 52. Figure 53A is an extracellular potential waveform actually obtained by electrophysiological extracellular potential. With administration of a drug, the following changes occur in extracellular potential. If the drug has a sodium ion channel blocking activity, a reduction of the first portion of the spike waveform occurs, and if the drug has a calcium ion channel blocking activity, the waveform changes in the direction to reduce FPD (time to the inward current peak position from the first spike of sodium), and if the drug has a potassium ion channel blocking activity, the waveform changes just opposite, i.e., in the direction to extend the FPD. Thus, if a drug generates inhibition of calcium ion channels, that causes contraction force and the drug causes potassium ion channel blocking at the same time, apparently reduced FPD cannot be simultaneously measured with respect to a decrease in the contractile force (because the extension effect of potassium counteracts the reducing effect of the FPD due to inhibition of calcium). Therefore, in practice, it is necessary to simultaneously measure the mechanical measurement in addition to electrical measurement.

Figure 53B is a graph summarizing the correlation of actual changes in the FPD and the changes in contractile force (displacement). It is clear that the loss of contractile force is present where the decrease in FPD is not so prominent.

Figure 53C is the analysis result of the loss of contractile force from another perspective. Specifically, as mentioned in the description of Figure 52, the relationship of the change in the intensity of the first spike of sodium and the change in contractile force is illustrated. As can be seen from the figure, the increased inhibition of sodium ion channels is dependent on the concentration of the drug, but in view of the results of Figure 53B, it is found that the first spike maintains sufficient strength to elicit an electrophysiological response of cells (responses of calcium ion channels and of potassium ion channels), and therein loss of tension occurs.

From the above comprehensive analysis, inhibition of sodium ion channels occurs by drug administration, however it is an inhibition at the level where there is still sufficient spare capacity for the generation of stimulation. Further, when the inhibition of calcium ion channels and the inhibition of potassium ion channels are in the same level, there is not a lot of movement in the position of the FPD, and therefore there is no problem about the position of the FPD. It is expected that the occurrence of QT prolongation risks associated with the FPD is not observed. However, in practice, it is analyzed that the loss of contractile force occurs because the inhibition of calcium ion channels occurs.

As the results indicate, it is possible to estimate the relationship between the inhibitory effects of a calcium ion channel and inhibitory effects of a potassium ion channel by combining the simultaneous measurements of electrophysiological extracellular potential waveform analysis and measurements of tension generation, which conventionally could not be estimated by only electrical measurement.

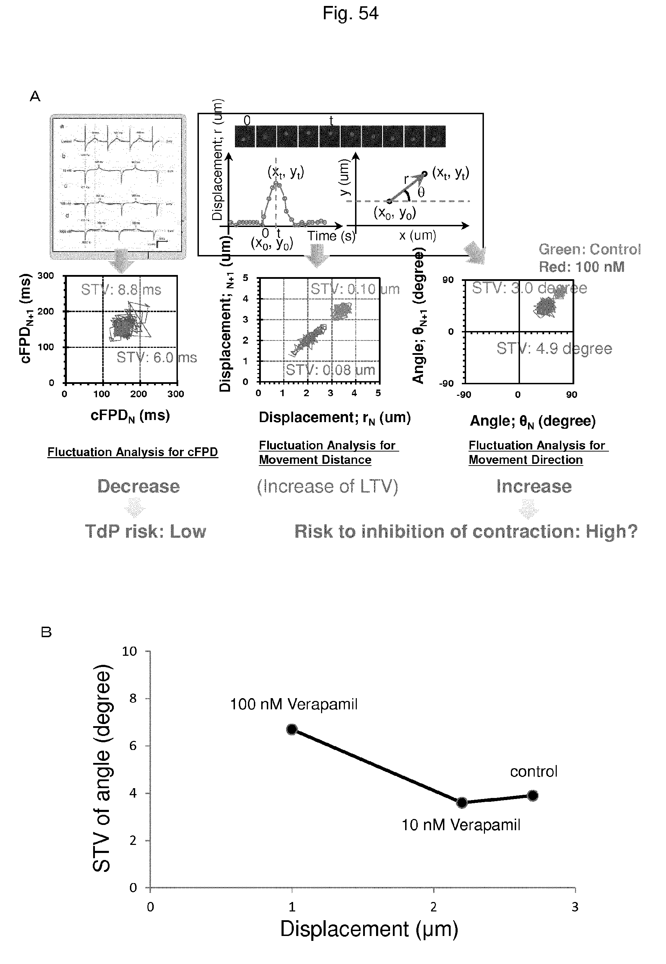

Figure 54A further summarizes points of view of pro-arrhythmic risk measurement by electrical/optical simultaneous measurement in addition to the above point of view. The measurements of time fluctuation of FPD obtained by electrophysiological measurements, fluctuation (variation) of movement distance (displacement amount) between contraction intervals for muscle contraction obtained by optical measurement and fluctuation (variation) of movement direction (angle) between contraction intervals can be performed simultaneously in the system of the present invention. In particular, in addition to the conventional pro-arrhythmic effect due to electrophysiological legacy reasons, it is also possible to analyze to what extent any loss of uniformity in the contractile forces of the cells is due to variation in the original quality of the cardiomyocyte population caused by drug administration as compared to the variation of the FPD by electrophysiological measurements. For example, as a viewpoint which could not be estimated by a conventional measurement methods, as also shown in the figure, it is observed that the angle fluctuation also increases, and the variation of the contractile force takes a maximum value with the administration of a drug, while it is observed that fluctuation increase of FPD is not seen. From this, it can be quantitatively estimated that the behavior of myocardial cell population becomes non-uniform in accordance with the decrease in contractile force, and a failure in pump function which requires cooperativity occurs.

In this way, the "fluctuation" analysis obtained for both the displacement direction and angular direction together becomes an indicator for the determination of drug properties which cannot be obtained with only electrophysiological measurements.

Figure 54B is a further graphical presentation of how the increase in fluctuation changes with respect to the reduction of the displacement amount. Upon administration of 10nM verapamil, angle fluctuation barely increases against a decrease in contractile force. However, it can be seen that upon administration of 100nM verapamil, a rapid fluctuation of contractile direction in the angular direction (i.e., randomization of contraction direction of the population) occurs.

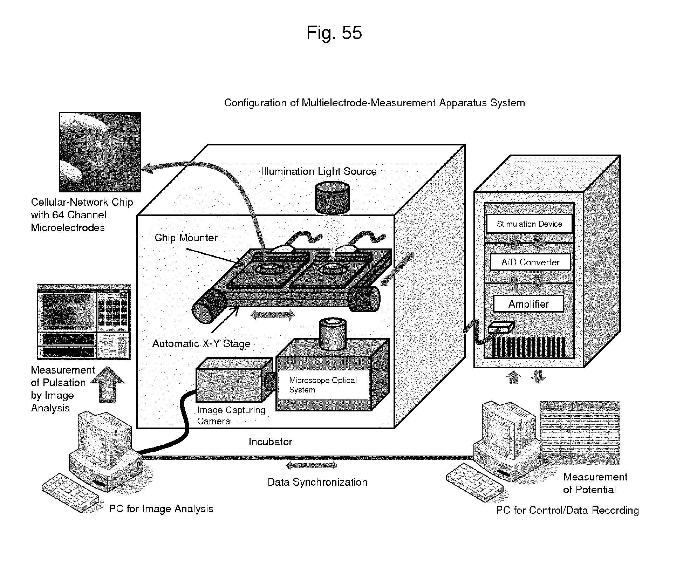

Figure 55 shows an example of a device configuration that combines measurements of extracellular potential and an optical system for measurements of changes in contractile function. Two or more cardiomyocyte-network chips incorporating an extracellular potential measurement capability are placed on a stage that can be moved in three dimensions, a chip mounter is combined with each of the chips and successive measurements of the potential can be performed continuously. As for the optical measurement, it is possible to perform the measurements of displacement (contraction distance) and direction of the displacement (contraction angle) of the myocardial cell in each chip by periodically moving the stage. Here, the measurement of fluctuation can be performed by obtaining pulsation data for n = 50 times for each chip.

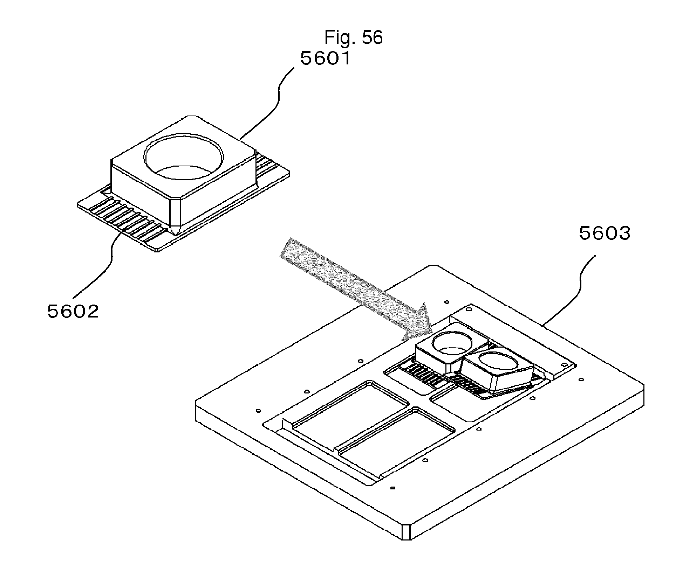

Figure 56 illustrates an example of a structure of a high-throughput cardiomyocyte-network array chip composed of a cell culture module array. In general, when measuring responses to drugs, use of multiwall-type cell culture plates is common in order to increase the measurement throughput. In fact, however, for cell culture plates with a multi-well structure, there is a possibility that all wells may not necessarily be utilized effectively because of problems such as that the state of the cell culture not being good in some well(s), or that cells may not adhere to the plate. In order to solve this problem, each well in the multi-well plate may be separated from each other in advance, and after starting culturing, only wells with good conditions are chosen to combine to make a multi-well plate, thereby making it possible to have all the plates in a good condition available. One example shown in Figure 56 simply illustrates this. Cell(s) are cultured in a well 5601 which is separated in advance to make the smallest unit. Wells which have cultured cells in good conditions are arranged in a plate 5603 to form a high-throughput cardiomyocyte network array chip. Here, since the electrode array 5602 for electrical measurements of extracellular potentials and cell stimulation is arranged in each well, contacts for connecting them are arranged in advance in the plate to place the well. Particularly, since wells can be replaced conveniently in unit of a single well (as one block), economical effects can be achieved by replacing wells of a cell network fatigued with drugs on the plate in a well-by-well manner rather than replacing the wells in a plate-by-plate manner.

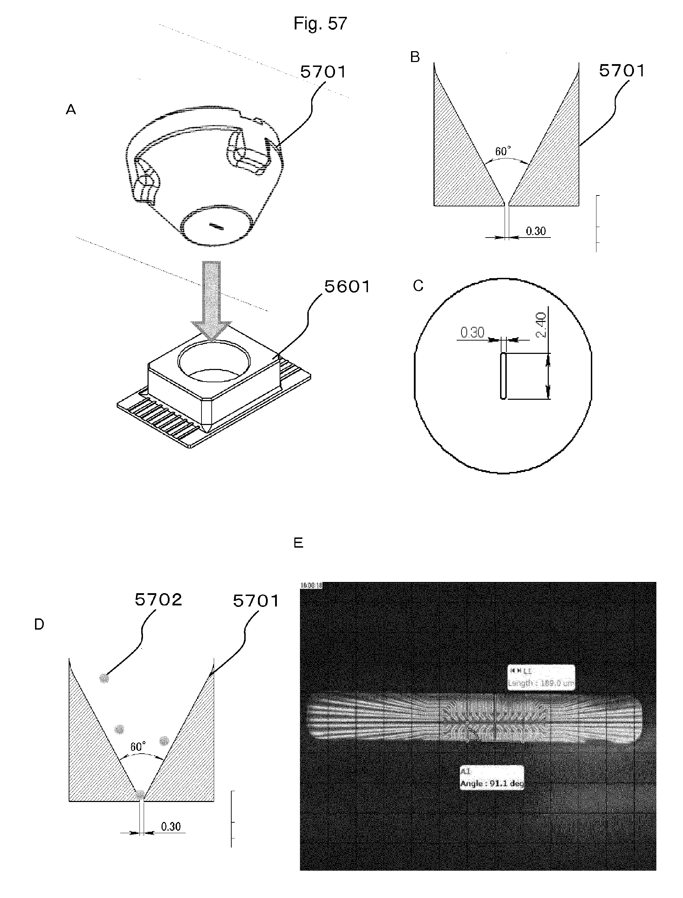

Figure 57 illustrates an example of a configuration of a cellular network deployment technique using a sample loader 5701 to place cardiomyocyte(s) effectively to each well 5601. As also shown in FIG 57A, the sample loader 5701 has a structure that can be inserted into the upper surface of the well 5601. Further, as also shown in Figure 57B and Figure 57C, the bottom surface of the sample loader has a slit with a width of $100\mu m$ to $300\mu m$ and a length of $500\mu m$ to about 3mm, and the inner surface of the sample loader is configured in a funnel-like shape. Thus, as shown in Figure 57D, by dropping a liquid 5702 containing cells onto the inner surface, cells are allowed to settle, and are arranged linearly in the same manner as the shape of the slit. While in the present example, it is configured that the cells precipitate effectively by a steep slope of 30 degrees from the vertical direction, it is possible to precipitate the cells effectively in the slit of the bottom surface by a slope of 40 degrees or less. Here, as long as the cell concentration in the liquid containing the cells is adjusted quantitatively in advance, it is possible to adjust the total number of cells to be arranged by only adjustment of the amount of the liquid, and a monolayer of a cardiomyocyte network can be constructed by minimizing the amount of cells, and a multilayer (e.g., two or three layers) of the cellular network can be constructed by increasing the amount of cells. Further, as shown in Figure 57E, if the structure of the sample loader is adjusted such that the orientation of the slit matches with the orientation of the electrodes which are arranged in a straight line as shown in the present example, cells can be arranged effectively to the electrode array by just inserting the sample loader in the wells. Further, as shown in this example, in order to perform effectively optical measurements and perform solution exchange effectively, the sample loader is effective to precipitate and dispose the cells effectively on the chip and it is preferable that the sample loader is removed when measurements of the cells are performed.

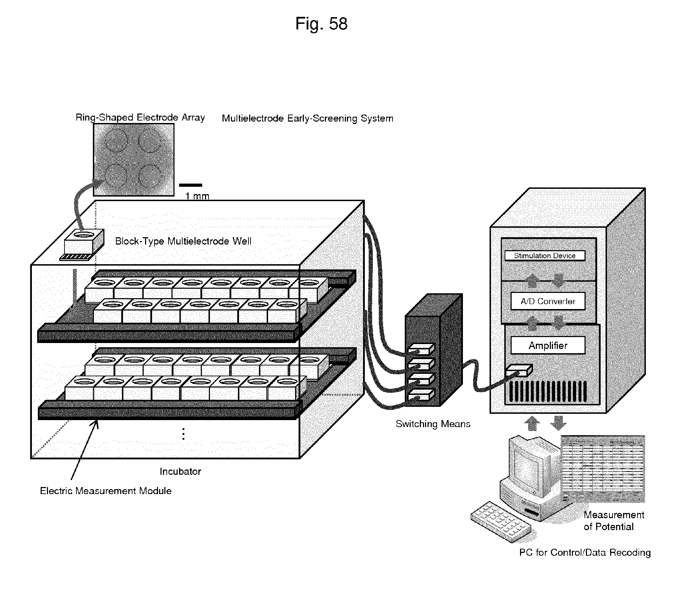

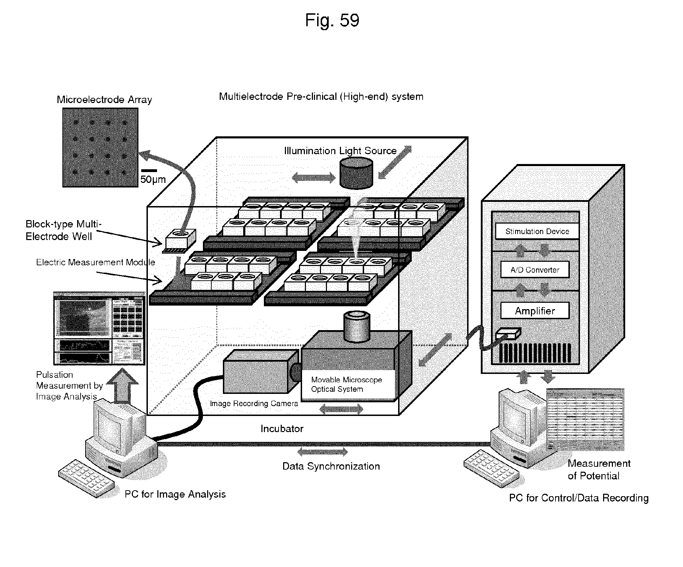

Figure 58 illustrates another example of a system for extracellular potential measurement by a multi-electrode system by arranging the actual block-type wells described above. In Figure 57 above, although the arrangement of the electrodes has discrete electrodes arranged in series, in this example, the electrodes in the well are arranged in a ring-like fashion as shown in Figure 12 above and the like. To arrange the cells in a ring-like fashion in the well, a sample loader with a ring-shaped slit on the bottom surface and not the linear slit on the bottom surface as shown in Figure 57 is used. Figure 59 illustrates a configuration in which an optical measurement module is further incorporated, and measurements of mechanical properties in each well can be performed by moving the optical system.

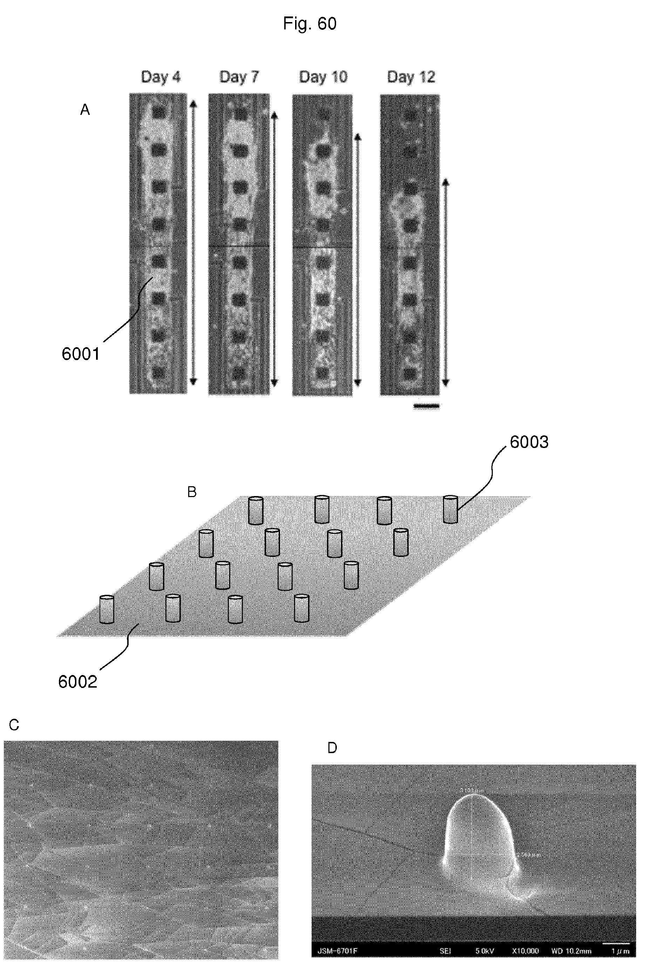

Figure 60 is a diagram illustrating the structure of a substrate on which microprojections are disposed regularly to prevent contraction of cardiomyocytes in a culture medium during measurements using a myocardia-cell network and a myocardial cell sheet. Figure 60A shows, as also shown in Figure 47, an example of experimental results showing a state in which a cardiomyocyte network 6001 gradually peeled off from the collagen layer on the bottom by its generation of contractile force, and gradually contracted to a mass. If the cardiac muscle cells are allowed to contract in clumps like this, they disappear from the microelectrode array that is arranged, making it difficult to measure the extracellular potential, excitatory stimulation conduction velocity from the network, and the time fluctuation thereof. In order to avoid this, as shown in Figure 60B, micro-protrusions (pillars) 6003 may be arranged regularly in the region of the substrate surface 6002 in which the myocardial cell sheet or the cardiomyocyte network is cultured. Figure 60D and Figure 60C are actual electron micrographs of an example of arranging pillars with $3\mu m$ in diameter, $5\mu m$ in height and with a pitch of $50\mu m$. Here, it is preferable that the diameter of the pillar is $5\mu m$ or less and the height is $3\mu m$ or more, and the pitch of the arrangement on the substrate of the pillars is $50\mu m$ or less. Although a cylindrical shape is shown in this example, a rectangular parallelepiped shape may also be used.

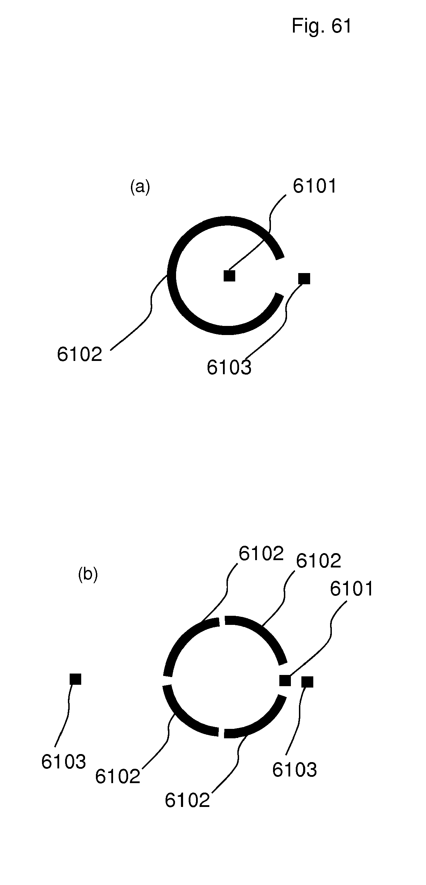

Figure 61 is a schematic diagram showing another example of the electrode arrangement of the multi-electrode substrate shown in FIG 42. First, in Figure 61(a), a measuring electrode 6102 is arranged in a circular ring form, and a stimulation electrode 6101 is disposed in the center of the circular ring. When cardiomyocytes are cultured in a two-dimensional sheet-like fashion on these electrodes, it is possible to measure by the measurement electrodes 6102 how the excitation conduction of myocardial cells ignited by forced stimulation from the center electrode 6101 propagates concentrically. Here, if fluctuation of the conduction occurs by an addition of a drug, waveform disturbance can be measured by the measurement electrode 6102 which is disposed on the circumference. Here, the distance from the center electrode 6101 of the annular measuring electrode 6102 is preferably $200\mu m$ or more. Figure 61 (b) is a schematic diagram showing the annular measurement electrode 6102 divided into four portions. Figure 61(a), a 2-dimensional cardiomyocyte sheet is used. In this example, cardiomyocytes are annularly arranged on the annular measurement electrodes 6102 and the stimulation electrode 6101. As for the propagation of the excitation conduction of the annular cardiomyocyte network, it is possible to estimate the rotation direction of the excitation conduction by measuring the time difference of excitation conduction between the four-divided measurement electrodes 6102.

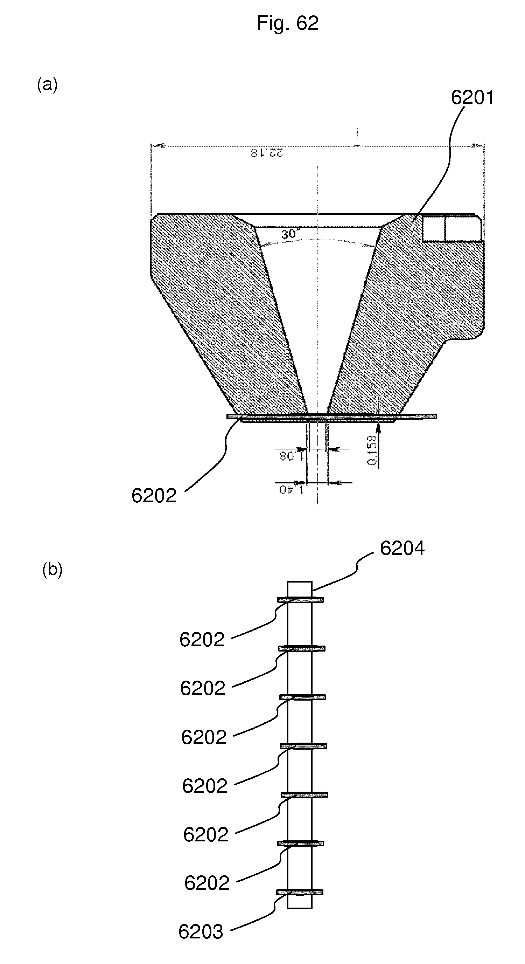

Figure 62 illustrates schematically an example of an embodiment in which metal micro wires are used as electrodes. In the example shown in FIG 62(a), small platinum electrodes 6202 of $10\mu m$ thickness is disposed on the bottom surface of container 6201 having the same shape as the sample loader shown in FIG 57, where the surface of the electrodes is modified with platinum black. If cardiomyocytes are dropped into the vessel 6201, platinum electrodes are incorporated into the cardiomyocyte network that has precipitated, and measurements of cardiomyocyte potential can be performed in the same way as when using the deposition electrode pattern placed on the substrate bottom surface. As shown in the top view of Figure 62(b), using the measurement micro-electrode wires 6202 which are regularly arranged, the extracellular potential of the myocardial cells which are arranged around the wires as well as the conduction between adjacent wires can also be measured. In particular, using one of the wires as a stimulation electrode wire 6203, measurements of the excitation conduction of the cells by forced stimulus are also possible. In this example, a platinum wire of $10\mu m$ in thickness is used. However, measurements with the same spatial resolution are possible as long as the thickness is $30\mu m$ or less. Further, the wire structure has also the effect of fixing the cells surrounding the wire so as to prevent the cardiomyocyte network from contraction as described above in relation to Figure 60.

INDUSTRIAL APPLICABILITY

According to the present invention, it is made possible to evaluate whether cardiomyocytes obtained through differentiation of stem cells, such as iPS cells, are healthy cardiomyocytes that can be used for agent screening or regenerative medicine for cardiomyocytes.

DESCRIPTION OF REFERENCE NUMERALS

1: transparent substrate, 2: microelectrode, 2c: reference electrode, 2': lead wire of microelectrode 2, $3_1$, $3_2$, $3_3$, $3_4$: a wall by agarose gel, $4_1$, $4_2$, $4_3$ and $4_4$: gap, 7: peripheral surrounding wall, $8_1$, $8_2$, $8_3$: pipe, PC: personal computer, Ms: operation signal of PC, $10,10_1,10_2,10_3$, ---, $10_n$: cardiomyocytes or fibroblasts, 15: transparent stage of an optical observation device, 16: X-Y drive unit, 18: Z drive unit, $CH_1$, $CH_2$, $CH_3$, $CH_n$: cell holding unit, CCC: cell communication channel, $10_G$:cell population, $11_a$: barrier, $11_b$: opening, 19, 191,192,193: dichroic mirror, 20, 201: a band-pass filter, 21, 211: camera, 22: light source, 221: fluorescent light source, 23,231: band-pass filter, 24,241: shutter, 25: condenser lens, 26: objective lens, 27: movable electrode, 28: ground electrode, 29, 291: switching circuit, 30, 301: electrical signal measuring circuit, 31,311: electrical stimulation circuit, 32: cardiomyocytes, 33: fibroblasts, 34: pipette for cell placement, 35: N-th round transmission pathway, 36: (N +1)th round transmission pathway, 37: (N +2)th round transmission pathway, 38: measuring electrode, 39: reference electrode, 40: liquid sending system, 41: cell population arranged in a ring shape, 42: 96-well plate, 43: photo-sensitive element of a camera, 44: cell, 45: cell stimulation electrode, 100: cardiotoxicity testing apparatus, 4201: multi-electrode substrate, 4202: cell culture vessel, 4203: flow of a solution, 4204: liquid level, 4301: glass substrate, 4302: transparent electrode, 4303: metal layer, 4304: insulating film, 4401: stimulating electrode, 4402: measurement electrodes, 4403: reference electrode, 4404: cell culturing area, 5601: single well, 5602: electrode array, 5603: plate, 5701: sample loader, 5702: liquid containing cells, 6001: myocardial cell network, 6002: surface of the substrate, 6003: micro protrusions (pillar), 6101: stimulation electrode, 6102: measurement electrode, 6103: reference electrode, 6201: container, 6202: micro- electrode wire for measurement, 6203: stimulation electrode wire, 6204: groove.

**Claims**

1. A cardiotoxicity evaluation apparatus, comprising:

   a substrate;
   a plurality of stably pulsating subject cardiomyocytes or a cell population comprising the subject cardiomyocytes and non-cardiomyocytes including fibroblasts placed on the substrate;
   a wall formed on the substrate to surround the periphery of the cell population and to fill a cell culture medium;
   at least one microelectrode on which a single cell of the cell population or a local portion of the cell population is placed;
   a reference electrode provided in the area which is to be filled with the cell culture medium and is surrounded by the wall;
   a potential-measuring means for measuring a cellular potential of the cell that is placed on the microelectrode using lead wires which are respectively connected to each of the microelectrodes and a lead wire which is connected to the reference electrode;
   a control/recording means for controlling an electrical stimulation delivered to the microelectrode and for recording data of the potential measured by the potential measuring means,
   microparticles of a diameter of from about 1 $\mu$m to about 50 $\mu$m having different optical properties to the cell population comprising the cardiomyocytes, wherein said microparticles are disposed in the cell population or in one or more places of the cell population;
   an optical measurement system comprising an irradiation light source, an optical microscope and an image-capturing camera for optically measuring the microparticles, wherein said optical measurement system continuously measures a position and a change in the positions of the microparticles as displacement data which includes temporal displacement data and data of change in angle of the orientation of the displacement; and
   a recording means for recording the data of the potential and the displacement data which are correlated with each other.

2. A cardiotoxicity evaluation apparatus, comprising:

   a substrate;
   a plurality of stably pulsating subject cardiomyocytes or a cell population comprising the subject cardiomyocytes and non-cardiomyocytes including fibroblasts placed on the substrate;
   a wall formed on the substrate to surround the periphery of the cell population and to fill a cell culture medium;
   at least one microelectrode on which a single cell of the cell population or a local portion of the cell population is placed;
   a reference electrode provided in the area which is to be filled with the cell culture medium and is surrounded by the wall;
   a potential-measuring means for measuring a cellular potential of the cell that is placed on the microelectrode using lead wires which are respectively connected to each of the microelectrodes and a lead wire which is connected to the reference electrode;
   a control/recording means for controlling an electrical stimulation delivered to the microelectrode and for recording data of the potential measured by the potential measuring means;
   microparticles of a diameter of from about 1 $\mu$m to about 50 $\mu$m having different optical properties than the cell population comprising the cardiomyocytes, wherein said microparticles are disposed in the cell population or in one or more places of the cell population;
   an optical measurement system comprising an object lens having a numerical aperture of about 3 $\mu$m or less and a zoom lens system downstream of the object lens, wherein said optical measurement system continuously measures a position and a change in the positions of the microparticles as displacement data which includes temporal displacement data and data of change in angle of the orientation of the displacement; and
   a recording means for recording data of the potential and the displacement data which are correlated with each other.

3. The cardiotoxicity evaluation apparatus according to claim 1 or 2, further comprising a culture medium supply/discharge channel for supplying the cell culture medium to and/or discharging the cell culture medium from the region surrounded by the wall.

4. The cardiotoxicity evaluation apparatus according to any one of claims 1 to 3, wherein said microelectrode comprises a stimulation electrode for stimulating a cell and a measurement electrode for measuring a cellular potential of the cell.

5. A cardiotoxicity evaluation method, comprising:

   selecting a cardiomyocyte which is **characterized by** a sharp generation of inward current of Na ions within about 20ms or less after a start of depolarization with clear rapid depolarization start, a subsequent slow generation of inward current of Ca ions within about 100ms after the start of the depolarization, and a prominent generation of outward current of K ions observed in about 100ms or after the start of the depolarization with no addition of a drug as a cardiomyocyte for measurements, and
   measuring an extracellular potential of the cardiomyocyte using the cardiotoxicity evaluation apparatus according to claim 1.

6. A cardiotoxicity evaluation method, comprising:

   selecting a cardiomyocyte which is **characterized by** a sharp generation of inward current of Na ions within about 20ms or less after a start of depolarization with clear rapid depolarization start, a subsequent slow generation of inward current of Ca ions within about 100ms after the start of the depolarization, and a prominent generation of outward current of K ions observed in about 100ms or after the start of the depolarization with no addition of a drug as a cardiomyocyte for measurements, and
   measuring an extracellular potential of the cardiomyocyte using the cardiotoxicity evaluation apparatus according to claim 2.

7. The cardiotoxicity evaluation method according to claim 5 or 6, wherein said cardiotoxicity evaluation apparatus further comprises a culture medium supply/discharge channel for supplying the cell culture medium to and/or discharging the cell culture medium from the region surrounded by the wall.

8. The cardiotoxicity evaluation method according to any one of claims 5 to 7, wherein the microelectrodes comprise a stimulation electrode for stimulating the cells and a potential measurement electrode for measuring a cellular potential of the cells.

9. A cardiotoxicity evaluation apparatus, comprising:

   a substrate;
   a plurality of stably pulsating subject cardiomyocytes or a cell population comprising the subject cardiomyocytes and non-cardiomyocytes including fibroblasts placed on the substrate;
   a wall formed on the substrate to surround the periphery of the cell population and to fill a cell culture medium;
at least one microelectrode on which a single cell of the cell population or a local portion of the cell population is placed;
an array of stimulation electrodes for stimulating the cells comprising a plurality of microelectrodes disposed two-dimensionally on the substrate, wherein a signal strength and a phase of the microelectrodes are mutually controllable;
a reference electrode disposed in the area which is to be filled with the cell culture medium and is surrounded by the wall;
a potential-measuring means for measuring a cellular potential of the cell that is placed on the microelectrode using lead wires which are respectively connected to each of the microelectrodes and a lead wire which is connected to the reference electrode; and
a control/recording means for controlling an electrical stimulation delivered to the microelectrodes for stimulating the cells, and for recording data of the potential measured by the potential-measuring means.

10. The cardiotoxicity evaluation apparatus of claim 9, further comprising a culture medium supply/discharge channel for supplying the cell culture medium to and/or discharging the cell culture medium from the region surrounded by the wall.

11. A cardiotoxicity evaluation method, comprising:

    selecting a cardiomyocyte which is **characterized by** a sharp generation of inward current of Na ions within about 20ms or less after a start of depolarization with clear rapid depolarization start, a subsequent slow generation of inward current of Ca ions within about 100ms after the start of the depolarization, and a prominent generation of outward current of K ions observed in about 100ms or after the start of the depolarization with no addition of a drug as a cardiomyocyte for measurements, and
    measuring an extracellular potential of the cardiomyocyte using a cardiotoxicity evaluation apparatus which comprises:

       a substrate;
       a plurality of stably pulsating subject cardiomyocytes or a cell population comprising the subject cardiomyocytes and non-cardiomyocytes such as fibroblasts placed on the substrate;
       a wall formed on the substrate to surround the periphery of the cell population and to fill a cell culture medium;
       at least one microelectrode on which a single cell of the cell population or a local por-

an array of stimulation electrodes for stimulating the cells comprising a plurality of microelectrodes disposed two-dimensionally on the substrate, wherein a signal strength and a phase of the microelectrodes are mutually controllable;
a reference electrode disposed in the area which is to be filled with the cell culture medium and is surrounded by the wall;
a potential-measuring means for measuring cellular potential of the cell that is placed on the microelectrode using lead wires which are respectively connected to each of the microelectrodes and a lead wire which is connected to the reference electrode; and
a control/recording means for controlling an electrical stimulation delivered to the microelectrodes for stimulating the cells, and for recording data of the potential measured by the potential-measuring means.

12. The cardiotoxicity evaluation method according to claim 11, wherein the cardiotoxicity evaluation apparatus further comprises a culture medium supply/discharge channel for supplying the cell culture medium to and/or discharging the cell culture medium from the region surrounded by the wall.

13. A cardiotoxicity evaluation method, comprising:

comparatively analyzing fluctuation (dispersion) of movement distance (displacement) and movement direction (angle) of myocardial cells between contraction intervals in association with contraction of cardiac muscle and fluctuation (dispersion) of FPD (time to maximum inward current position from the first spike of sodium) by electrophysiological measurement using an apparatus system capable of performing measurements and/or analysis of optical measurements of cardiomyocytes in synchronization with measurements of membrane potential of the cardiomyocytes,
wherein said movement distance and movement direction are obtained by optical measurements of the myocardial cells when they brought into contact with a target drug.

14. A replaceable well system, comprising:

one or more unit wells each comprising a single well, wherein said well comprises an electrode array including a cellular potential measuring electrode, a cell stimulation electrode and a reference electrode for myocardial cells on a bottom surface of the well; and
a well plate comprising one or more compartments to which the one or more unit wells can be mounted interchangeably;
wherein the wells are replaceable in a one-by-one basis and usable for forming a cardiomyocyte-network array, and wherein each of the compartments of the well plate comprises a contact connected correspondingly to one of the lead wires of the electrode array, and each of the unit wells is arranged interchangeably with respect to the compartment.

15. A cardiomyocyte measuring apparatus system, comprising:

two or more cardiomyocyte-network chips each comprising a well for accommodating a cardiomyocyte, and an electrode array including a cell stimulation electrode, a cellular potential measuring electrode and a reference electrode on a bottom surface of the well;
a stage for mounting the chips;
a potential measuring system comprising a power supply for providing electric stimulation to the cardiomyocyte through the electrode array and measuring an extracellular potential of the cardiomyocyte;
an optical observation system for optically observing the cardiomyocytes comprising an optical microscope, a camera for recording images and an illumination light source; and
a control and/or analysis device for recording and/or analyzing the potential measuring data and the optical observation data in a synchronous manner.

16. The cardiomyocyte measuring apparatus system according to claim 15 which is used for evaluation of cardiotoxicity.

17. A multi-electrode substrate for the potential measuring of cardiomyocyte network arranged in a circle, comprising:

either

(i) a ring-shaped potential measuring electrode corresponding to the cardiomyocyte network, wherein a portion of the electrode is cut out;

a stimulation electrode for applying a local force stimuli located in the cut-out site of the potential measuring electrode; and
a reference electrode for eliminating noise, which is disposed near the outside of the ring, or

(ii) a plurality of potential measuring electrodes in which the ring-shaped potential

30

measuring electrode as describe in (i) above is further cut out into two or more parts;

a stimulation electrode for applying a local forced stimuli, which is located in the cut-out site of the potential measuring electrodes; and a reference electrode for eliminating noise, which is disposed near the outside of the ring.

18. A multi-electrode substrate for measuring the potential of cardiomyocyte network using a two-dimensional cardiomyocytes sheet, comprising:

a ring-shaped potential measuring electrode, wherein a portion of the electrode is cut out; a stimulation electrode for applying a local force stimuli, which is located in the center of the ring; and a reference electrode for eliminating noise, which is disposed near the outside of the cut-out site of the potential measuring electrode.

19. A sample loader for placing a cardiomyocyte sample in a block-type unit well comprised of one well comprising an electrode array including a cellular potential measuring electrode, a stimulating electrode and a reference electrode arranged on the bottom surface of the well, wherein the sample loader has an external shape corresponding to the shape of the unit well such that the sample loader can be inserted into a top surface of the unit well; a funnel-shaped inner structure; and a slit (opening) corresponding to the shape of the electrode of the unit well on a bottom surface, and wherein the cardiomyocytes can be placed on the electrode by dropping an appropriate amount of the cardiomyocyte sample onto the inner surface of the sample loader.

20. A cardiomyocyte culture measurement substrate on which microprojections are disposed regularly to prevent contraction of the cardiomyocyte during culturing and measuring using a cardiomyocyte network or a myocardial cell sheet.

21. A cardiotoxicity evaluation apparatus **characterized in that** a microelectrode wire is used as an electrode.

Fig. 1

Fig. 2

CH

Fig. 3

Fig. 4

(a) Pulse of cell population

(b) Pulse of target cell (normal state)

(c) Pulse of target cell (on-drug state)

Fig. 5

(a) Changes in volume of cell due to pulsation of cell population

(b) Changes in volume of cell due to pulsation of target cell (normal state)

(c) Changes in volume of cell due to pulsation of target cell (on-drug state)

Fig. 6

(a) Changes in extracellular potentials of target cell (normal state)

(b) Changes in extracellular potentials of target cell (on-drug state)

Fig. 7

Fig. 8

Fig. 9

(a)

Convert FPD data
into Poincare plots

(b)

Fig. 10

(a)

**Number and arrangement of cells under control**

Generation of re-entry

(b)

100 μm

Fig. 11

(a)

**Cell population having a certain width that allows selection of conduction pathways**

(b)

Chamber
    Length: 450 μm
    Width: about 50 μm

Total: 50 cells
    Myocyte: 32 cells
    Fibroblast: 18 cells

(c)

Blue: Nuclear
Green: Mitochondria (Cardiomyocyte)

50 μm

6 Myocytes & 2
Fibroblasts in
yellow box

100 μm

Fig. 12

(a)

40

41

38

39

φ1-3 mm
ring shape

42

(b)

Normal
conduction

N          N          N

Abnormal
conduction

N  E  U        N  E    R

Sampling rate: ~ 1 kHz

500 ms

Fig. 13

(a)

2

Φ10, 20, 50 μm

Nuclear stain image. Bar: 20 μm

(b)

20 μm

ISI = 1.1 sec, SD = 0.7 sec, CV = 66 %

(c)

Cell number: 8

Cell to be
measured

Cells adjacent to
the cell to be measured

ISI = 1.1 sec, SD = 0.08 sec, CV = 7.2 %

Fig. 14

Local electrical stimulation
system (tungsten electrode)

Tip diameter: 5 μm

CCD neuro-chip

Transfer data
every 1/10,000 seconds

Cell 44

45

Cell 44

43

CCD Element    CCD Element

5μm

Transfer data every 1/10,000 seconds
Real-time calculation by FPGA for image processing

Image processing technique can be employed for data processing!

Feedback to stimulation electrode

EP 2 772 531 A1

Fig. 15

45

Fig. 16

EP 2 772 531 A1

*In vivo*
(Individual level)

*In vitro*
(Cell network)

$Na^+, Ca^{2+}$
IKr, IKs

$Na^+, Ca^{2+}$Inhibitions ⟨Corresponds to⟩ Potential measurements
IKr, IKs

Potential measurements
(Analysis of waveforms as
changes in cellular potential)

Bar: 100 μm

$Na^+$Inhibition ⟨Corresponds to⟩ Potential measurement
(Conduction velocity
between adjacent cells)

Cardiac waveform
(abnormal cardiac rhythm)
Output of blood

⟨Corresponds to⟩ Optical measurement
(Change in shape of
myocardial cell)

On-stage incubator

MEA

Automated X-Y stage    1480nm Laser

TdP (Torsades de Pointes)
Atrial fibrillation/Ventricular
fibrillation model

⟨Corresponds to⟩ Myocardial cell network model
(Re-entry circuit)

On-chip myocardial cell network
Optical/Potential measurement system

Cardiac hypertrophy model ⟨Corresponds to⟩ Myocardial cell network model
(Structural cell arrangement:
myocardial cells + fibroblast cells)

Fig. 17

Fig. 18

Fig. 19

(a)

(b)

$$STV = \frac{\displaystyle\sum_{n=1}^{k} |\,(FPD_{n+1} - FPD_{mean}) - (FPD_n - FPD_{mean})\,|}{k \times \sqrt{2}}$$

$$= \frac{\displaystyle\sum_{n=1}^{k} |\,FPD_{n+1} - FPD_n\,|}{k \times \sqrt{2}} \qquad \cdot\cdot\cdot (\text{Equation 1})$$

## Fig. 20

(a)

(b)

$$LTV = \frac{\displaystyle\sum_{n=1}^{k} \left| (FPD_{n+1} - FPD_{mean}) + (FPD_n - FPD_{mean}) \right|}{k \times \sqrt{2}}$$

$$= \frac{\displaystyle\sum_{n=1}^{k} \left| FPD_{n+1} + FPD_n - 2FPD_{mean} \right|}{k \times \sqrt{2}} \qquad \cdots \text{(Equation 2)}$$

Fig. 21

(a)

(b)

Fig. 22

Fig. 23

Fig. 24

(a)

(b)

|  | Before Addition of an Agent | Low Volume Addition | Medium Volume Addition |

A — Fibrillation/Stopped

B — Fibrillation/Stopped

C

D

(c)

$$STV = \frac{\sum_{n=1}^{k} |V_{n+1} - V_n|}{k \times \sqrt{2}} \qquad \cdots (\text{Equation 3})$$

Fig. 25

Conventional in vivo measurement (ECG)

Surface ECG

QT prolongation

Long QT syndrome

QT interval

Field Potential in accordance with the present invention (Multi-electrode method)

(Measurement of potential of a cell network)

500 ms

(Measurement of potential of a single cell)

$T_{duration}$ = QT interval ?

$FP_{pre}$   $FP_{Max}$

$K^+$

$Ca^{2+}$

$FP_{Min}$   $Na^+$

N-C:  52 ms
N-K: 155 ms

Conventional in vitro measurement (Patch clamp method)

Action Potential

Cardiac Action Potential

0 mV

AP prolongation

QT interval

Change in potential of an organ

Change in potential of a cell network

Change in potential of a cell

Potential of a cell

Comparison of QT area Poincare plotting

Superposition of changes of potentials of cells in the network

Integral

Differential

Signal of an organ

Signal of a cell network

Signal of a single cell

EP 2 772 531 A1

Fig. 26

Data of
Comparison
with ECG

Super-
posing
Circuit

Accumulation/
Analysis Unit for
Waveform Data

Differe
ntial
Circuit

Data of
Comparison
with
Cell
Potential

Mechanism
for Direct
Prediction of
an
Occurrence
of
Extrasystole

FP Waveform Data
from Each of
the Electrodes

Estimation
of State
of Ion
Channels
from the
Prediction
of Cell
Potential

EP 2 772 531 A1

Fig. 27

(A)

L8  R8
L7  R7
L6  R6
L5  R5
L4  R4
L3  R3
L2  R2
L1  R1

(B)

L8
L7
L6
L5
L4
L3
L2
L1
R1 (PM)
R2
R3
R4
R5
R6
R7
R8

S

200 ms

82 msec (S.D. = 1.2 ms, spike number = 33, CV = 1.4 %)

(C)

1 s

1 mV

Fig. 28

Fig. 29

$$y = 489.43x^{-0.424}$$
$$R^2 = 0.5158$$

Fig. 30

Fig. 31

(a)

Measurement
Electrode →

Stimulation
Electrode →

200 μm

(b)

Measurement
Electrode

Stimulation
Electrode →

500 um

Fig. 32

10

2

Feedback System
for Electrical
Current Recording

Power
Source

Feedback
Mechanism

Stimulation
Mechanism

Fig. 33

## Fig. 34

(a)

(b)

FPDc (^1/3) Fredericia
FPDc (^1/2.5)
FPDc (^1/2) Bazett

Fig. 35

Table. 1  Heart rate correction of beating frequency, field potential duration

| | Beating Frequency | | | | FPD | | | FPDc | | |
|---|---|---|---|---|---|---|---|---|---|---|
| | Average (Hz) | S.D. (Hz) | C.V. (%) | Spike No. | Average (ms) | S.D. (ms) | C.V. (%) | Average (ms) | S.D. (ms) | C.V. (%) |
| 0.4 Hz (ctrl) | 0.37 | 0.01 | 1.5 | 110 | 795.3 | 13.9 | 1.7 | 536.7 | 9.7 | 1.8 |
| 0.5 Hz | 0.50 | 0.00 | 0.0 | 94 | 675.6 | 6.6 | 1.0 | 513.7 | 5.0 | 1.0 |
| 0.6 Hz | 0.60 | 0.00 | 0.0 | 142 | 638.5 | 7.5 | 1.2 | 521.8 | 6.1 | 1.2 |
| 0.8 Hz | 0.75 | 0.00 | 0.0 | 189 | 613.7 | 8.6 | 1.4 | 547.8 | 7.7 | 1.4 |
| 1.3 Hz | 1.28 | 0.00 | 0.0 | 253 | 489.4 | 11.5 | 2.4 | 540.2 | 12.7 | 2.4 |
| 1.8 Hz | 1.78 | 0.00 | 0.0 | 306 | 431.2 | 9.1 | 2.1 | 542.8 | 11.4 | 2.1 |

Fig. 36(a)

(a)

2c        10   2

**Difference Circuit**

# Fig. 36(b)

## Fig. 36(c)

Fig. 37

(a)

FP waveforms of cardiomyocytes
obtained from microelectrodes

X Axis: (Conventional) Average Values of FPD

E-4031
(n=27)

Y Axis: Fluctuation of FPD between adjacent
waveforms (Phasic Evaluation)

(b)

Fig. 38

Fig. 39

1st layer
2nd layer
3rd layer
4th layer

20 mm
Laminated by Heat Seal
under Pressure

8-well plate with 3D injection/ exhort path made of integral acryl plate.

Plate size : □125 mm      Electrode size: φ10 μm

100 μm

8-well (8 electrodes / 1-well) ITO electrodes

8-well Multi-Electrode
Culture Plate with Liquid
Reflux Function

8-Well Multi-Electrode
Culture Plate with Liquid
Reflux Function
Integrated in a Culture
Measurement System

Fig. 40

Fig. 41

Cell Observation Area

4304

4302

4304

4303

4301

Fig. 42

(a)

4402    4402    4404

4401
4403
4403

(b)

4404

4401
4403
4402
4403

(c)

4402    4404

4401
4403
4402    4402
4403

(d)    4401

4402
4403

(e)    4402    4401

4402
4402    4403

## Fig. 43

**An Example of a 64-channel micro multi-electrode pattern
10μm ITO-Pt Block Electrode × 64 channel**

**Reference electrode: Pt**

**Bar = 0.1 mm**

64-Channel Micro Multi-
Electrode Cellular Network
Chip

Illumination Light Source

Chip Mounter

Micro Multi-Electrode Potential
Measurement System

Stimulation Device

A/D Converter

Amplifier

Measurement of Pulsation by Image
Analysis

Microscope Stage

Environment Controlling
Chamber

Image
Capturing
Camera

Microscope

Data Synchronization

Measurements
of Potentials

PC for Cellular Potential Analysis/
Stimulation Control/Deta Recording

PC for Image Analysis

Electrophysiological Properties of
Cellular Response

Cell
Stimulation

Measurements of Contractile (Morphological
Change) Properties of Cellular Response

A) Autonomic Pulsation Conduction
B) Forced Stimulation Conduction
C) Feed-Back Stimulation Conduction

EP 2 772 531 A1

Fig. 44

Captured Image of Cardiomyocyte Population

Identified Probe Microparticle
Identified Probe Microparticle

Cellular Potential Waveform from All Micro-Electrodes

Probe-Particle Displacement-Observation Window

Target Cellular Potential Waveform

Displacement Amount of Each Probe Microparticle in the Direction of Y-Axis

Displacement Amount of Each Probe Microparticle in the Direction of X-Axis

Condition Setting Member

Fig. 45

Temporal Change of Extra-Cellular Potential

FP recordings

Amplitude
(uV)

(SR: 20kHz)

Optical imaging

Temporal Change of
Cardiomyocyte Displacement

Displacement: r
(um)

(SR: 10Hz)

Temporal Change of Velocity
of Cardiomyocyte Contraction
Velocity: dr/dt
(um/s)

1 s

Fig. 46

Continuous Images Showing Temporal Change of
A Probe Microparticle Attached to A Cell

Fluctuation Analysis of Movement Direction

Fig. 47

(a)

100
um

(b)

(c)

(d)

(e)

|          | Day 1 | Day 3 | Day 5 | Day 7 | Day 10 | Day 12 |
|----------|-------|-------|-------|-------|--------|--------|

Fig. 48

Fig. 49

(A)

(B)

Sample number: n = 4

## Fig. 50

(A)

Stimulation Electrode Array

(B)

Stimulation Electrode Array

Fig. 51

Fig. 52

Fig. 53

Sample: mECMs
Drug: Verapmail

A

**Field Potential recordings**

Inhibition of
Ca²⁺ current    FPD    Inhibition of
                       K²⁺ current

Shortening              Prolong

0 uV

Decrease

Amplitude

Inhibition of
Na⁺ current

B

Inhibition of Ca²⁺ current
Inhibition of Contraction

Shortening

Arrest

Control    [Drug] (nM)

Mean±SD
cFPD
Displacement

C

Inhibition of Na⁺ current
Inhibition of Contraction

Decrease

Arrest

[Drug] (nM)

Mean±SD
cFPD
Displacement

Fig. 54

EP 2 772 531 A1

Fig. 55

Configuration of Multielectrode-Measurement Apparatus System

Fig. 56

5601

5602

5603

Fig. 57

Fig. 58

Ring-Shaped Electrode Array    Multielectrode Early-Screening System

1 mm

Block-Type Multielectrode Well

Stimulation Device

A/D Converter

Amplifier

Switching Means

Incubator

Electric Measurement Module

Measurement
of Potential

PC for Control/Data Recoding

Fig. 59

Multielectrode Pre-clinical (High-end) system

Fig. 60

Fig. 61

(a)

6101

6102

6103

(b)

6102 6102

6101

6103

6102

6103

6102

Fig. 62

(a)

(b)

## INTERNATIONAL SEARCH REPORT

| International application No. |
| --- |
| PCT/JP2012/076860 |

**A. CLASSIFICATION OF SUBJECT MATTER**
*C12M1/34*(2006.01)i, *C12M3/00*(2006.01)i, *C12Q1/02*(2006.01)i, *G01N27/30* (2006.01)i, *G01N27/416*(2006.01)i

According to International Patent Classification (IPC) or to both national classification and IPC

**B. FIELDS SEARCHED**

Minimum documentation searched (classification system followed by classification symbols)
C12M1/34, C12M3/00, C12Q1/02, G01N27/30, G01N27/416

Documentation searched other than minimum documentation to the extent that such documents are included in the fields searched
| Jitsuyo Shinan Koho | 1922–1996 | Jitsuyo Shinan Toroku Koho | 1996–2012 |
| Kokai Jitsuyo Shinan Koho | 1971–2012 | Toroku Jitsuyo Shinan Koho | 1994–2012 |

Electronic data base consulted during the international search (name of data base and, where practicable, search terms used)
JSTPlus/JMEDPlus/JST7580(JDreamII), WPI, PubMed

**C. DOCUMENTS CONSIDERED TO BE RELEVANT**

| Category* | Citation of document, with indication, where appropriate, of the relevant passages | Relevant to claim No. |
| --- | --- | --- |
| Y | WO 2010/064700 A1 (Tokyo Medical and Dental University), 10 June 2010 (10.06.2010), entire text; particularly, claims 1 to 26 & EP 2371941 A1 | 1–8 |
| Y | Kazuhiro NONAKA et al., "Analysis of Cell Population Behavior on a Culture Surface Using a Particle Image Velocimetry", Life Support, 2010, vol.22, no.3, pages 125 to 131, entire text | 1–8 |

☒ Further documents are listed in the continuation of Box C.　　☐ See patent family annex.

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 20 December, 2012 (20.12.12) | 08 January, 2013 (08.01.13) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2012/076860 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | WO 2008/152983 A1  (Tokyo Medical and Dental University, Mitsubishi Chemical Safety Institute Ltd.), 18 December 2008 (18.12.2008), entire text & US 2010/0178692 A1    & EP 2157166 A1 | 1-8 |
| A | WO 2008/149976 A1  (Tokyo Medical and Dental University, Mitsubishi Chemical Safety Institute Ltd.), 11 December 2008 (11.12.2008), entire text & US 2010/0173351 A1    & EP 2157165 A1 | 1-8 |
| A | JP 2008-263858 A  (Tokyo Medical and Dental University), 06 November 2008 (06.11.2008), entire text (Family: none) | 1-8 |
| A | JP 2006-515420 A  (E.I. Du Pont de Nemours & Co.), 25 May 2006 (25.05.2006), entire text & US 2005/0019842 A1    & EP 1558750 A | 1-8 |
| A | FUJITA, K., et al., Time-resolved observation of surface-enhanced Raman scattering from gold nanoparticles during transport through a living cell., J. Biomed. Opt., 2009, Vol.14, No.2, 024038-1 - 024038-7, entire text | 1-8 |
| P,Y | WO 2012/043820 A1  (Tokyo Medical and Dental University, Mitsubishi Chemical Medience Corp.), 05 April 2012 (05.04.2012), entire text; particularly, claims 1 to 20; fig. 1 to 46 (Family: none) | 1-8 |

Form PCT/ISA/210 (continuation of second sheet) (July 2009)

# EP 2 772 531 A1

<table>
<tr><td>**INTERNATIONAL SEARCH REPORT**</td><td>International application No.<br>PCT/JP2012/076860</td></tr>
</table>

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
See extra sheet.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.:
1-8

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

97

**INTERNATIONAL SEARCH REPORT** | International application No.

PCT/JP2012/076860

Continuation of Box No.III of continuation of first sheet(2)

The following nine inventions are involved in claims 1-21.
Invention 1: claims 1-8
The inventions relating to a device for evaluating cardiotoxicity, said device comprising: a substrate; a cell mass containing cardiomyocytes; a wall for filling a cell culture liquid; at least one microelectrode; a reference electrode; a potential-measuring means for measuring cell potential; a controlling/recording means for recording potential data; microparticles; an optical measurement system for measuring the position and displacement of the microparticles; and a recording means for recording the potential data and displacement data using a correlation therebetween, and a method for evaluating cardiotoxicity with the use of said device.
Invention 2: claims 9-12
The inventions relating to a device for evaluating cardiotoxicity, said device comprising: a substrate; a cell mass containing cardiomyocytes; a wall for filling a cell culture liquid; at least one microelectrode; a stimulus electrode array comprising microelectrodes; a reference electrode; a potential-measuring means for measuring cell potential; and a controlling/recording means for recording potential data, and a method for evaluating cardiotoxicity with the use of said device.
Invention 3: claim 13
The invention relating to a method for evaluating the cardiotoxicity of a drug, characterized by comprising a step for comparing and analyzing the fluctuation (dispersion) in the motion distance (displacement magnitude) and motion direction (angle) of cardiomyocytes between the individual contraction intervals, which relate to muscular contraction and are obtained by optical measurement upon the contact of the cardiomyocytes with the drug, and the fluctuation (dispersion) in FPD (the time from the first sodium spike to the introrse current maximum value point) which is obtained by electrophysiological measurement.
Invention 4: claim 14
The invention relating to a well system wherein wells are replaceable in a single well unit constituting a cardiomyocyte network array, said system being characterized by comprising a unit well which is provided, on the bottom surface of the well, with an electrode array comprising an electrode for measuring the cell potential of a cardiomyocyte, an electrode for stimulating a cell and a reference electrode, and a well plate provided with one or more divisions on which one or more said unit wells can be loaded in a replaceable manner.
Invention 5: claims 15 and 16

(Continued to next extra sheet)

**INTERNATIONAL SEARCH REPORT**

International application No.

PCT/JP2012/076860

The inventions relating to a cardiomyocyte measurement device system comprising: two or more cardiomyocyte network chips provided with a well for housing a cardiomyocyte therein and an electrode array comprising an electrode for stimulating a cell, an electrode for measuring the cell potential of a cardiomyocyte and a reference electrode; a stage for loading the chips thereon; a potential-measuring system comprising a power source for measuring extracellular potential; an optical observation system comprising an optical microscope, an image-capturing camera and a power source for illumination; and a controlling/analyzing device for synchronously recording and analyzing potential measurement data and optical observation data.

Invention 6: claims 17 and 18

The inventions relating to a multielectrode substrate for measuring the potential of a cardiomyocyte network, said multielectrode substrate comprising a toric potential-measuring electrode with a partial defect in the electrode, a stimulus electrode and a reference electrode or a plurality of potential-measuring electrodes, a stimulus electrode and a reference electrode, or a multielectrode substrate for measuring the potential of a cardiomyocyte network using a two-dimensional cardiomyocyte sheet, said multielectrode substrate comprising a toric potential-measuring electrode with a partial defect in the electrode, a stimulus electrode and a reference electrode.

Invention 7: Claim 19

The invention relating a sample loader for loading a cardiomyocyte sample on a block type unit well which comprises a well provided, on the bottom surface thereof, with an electrode array comprising an electrode for measuring cell potential, an electrode for stimulating a cell and a reference electrode.

Invention 8: claim 20

The inventions relating to a substrate for measuring cardiomyocyte culture wherein microprojections for preventing contraction of cardiomyocytes in the course of culture and measurement with the use of a cardiomyocyte network or a cardiomyocyte sheet are provided at intervals.

Invention 9: claim 21

The invention relating to a device for evaluating cardiotoxicity characterized in that a microelectrode wire is used as an electrode.

Document 1 discloses a device for examining myocardial toxicity, characterized by comprising: a substrate; a cell mass containing cardiomyocytes; a cell connecting channel consisting of cardiomyocytes and fibroblasts; a wall for filling a cell culture liquid; a means for supplying/discharging the cell culture liquid; a means for adding a drug to the cell culture liquid; a plurality of microelectrodes; a reference electrode; a means for measuring and recording cell potential (i.e., a potential-measuring means for measuring cell potential and a means for recording potential data); and a means for optically measuring cell state, and a method for evaluating cardiotoxicity with the use of said device (entire text, in particular, claims 1-26 and fig. 1-16).

(Continued to next extra sheet)

Form PCT/ISA/210 (extra sheet) (July 2009)

99

**INTERNATIONAL SEARCH REPORT**

International application No.

PCT/JP2012/076860

Although it appears that the technical feature common to Invention 1 and Invention 2 is to use a device for evaluating cardiotoxicity which comprises: a substrate; a cell mass containing cardiomyocytes; a wall for filling a cell culture liquid; at least one microelectrode; a reference electrode; a potential-measuring means for measuring cell potential; and a controlling/recording means for recording potential data, such a device for evaluating cardiotoxicity is disclosed in Document 1. Therefore, this technical feature cannot be considered as a special technical feature.

Although it appears that the technical feature common to Invention 1 and Invention 3 is to evaluate cardiotoxicity with the use of cardiomyocytes, such a method for evaluating cardiotoxicity is disclosed in Document 1. Therefore, this technical feature cannot be considered as a special technical feature.

Although it appears that the technical feature common to Invention 1 and Invention 4 is to relate to an instrument whereby an electrode for measuring cell potential is used for cardiomyocytes, such an instrument is disclosed in Document 1. Therefore, this technical feature cannot be considered as a special technical feature.

Although it appears that the technical feature common to Invention 1 and Invention 5 is to use a device which comprises cardiomyocytes, electrodes, a potential-measuring means for measuring cell potential and an optical measurement system, such a device for evaluating cardiotoxicity is disclosed in Document 1. Therefore, this technical feature cannot be considered as a special technical feature.

Although it appears that the technical feature common to Invention 1, Invention 6 and Invention 7 is to relate to an instrument whereby the potential of a cardiomyocyte is measured using a plurality of electrodes, such an instrument is disclosed in Document 1. Therefore, this technical feature cannot be considered as a special technical feature.

Although it appears that the only one technical feature common to Invention 1 and Invention 8 is to use cardiomyocytes, use of cardiomyocytes is disclosed in Document 1. Therefore, this technical feature cannot be considered as a special technical feature.

Although it appears that the only one technical feature common to Invention 1 and Invention 9 is to relate a device for evaluating cardiotoxicity using electrodes, such a device is disclosed in Document 1. Therefore, this technical feature cannot be considered as a special technical feature.

In conclusion, nine inventions, which do not form a single general inventive concept, are grasped from claims 1-21.

Document 1: WO 2010/064700 A1

Form PCT/ISA/210 (extra sheet) (July 2009)

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- JP 2006094703 A

## Non-patent literature cited in the description

- **BRENNAN M ; PALANISWAMI M ; KAMEN P.** Do existing measures of Poincare plot geometry reflect non-linear features of heart rate variability? Biomedical Engineering. *IEEE Transactions on, Proc. IEEE Transactions on Biomedical Engineering,* 2001, vol. 48, 1342-1347
- **KANTERS JK ; HOLSTEIN-RATHLOU NH ; AGNER E.** Lack of evidence for low-dimensional chaos in heart rate variability. *Journal of Cardiovascular Electrophysiology,* 1994, vol. 5 (7), 591-601
- **STORELLA RJ ; WOOD HW ; MILLS KM et al.** Approximate entropy and point correlation dimension of heart rate variability in healthy subjects. *Integrative Physiological & Behavioral Science,* 1994, vol. 33 (4), 315-20
- **PATRICK DAVEY.** How to correct the QT interval for the effects of heart rate in clinical studies. *Journal of Pharmacological and Toxicological Methods,* 2002, vol. 48, 3-9